# EUROPEAN PATENT SPECIFICATION

(11) **EP 3 840 832 B1**
(45) Date of publication and mention of the grant of the patent: **11.10.2023**
(21) Application number: 19759716.4
(22) Date of filing: 23.08.2019
(51) Int. Cl.: A61P 17/00, C07D 487/04, A61K 31/5025

(54) **IMIDAZO[1,2-B]PYRIDAZINES AS TRK INHIBITORS**
IMIDAZO[1,2-B]PYRIDAZINE ALS TRK-HEMMER
IMIDAZO[1,2-B]PYRIDAZINES UTILISÉES EN TANT QU'INHIBITEURS DE TRK

(30) Priority: 23.08.2018 GB 201813791
(43) Date of publication of application: 30.06.2021
(73) Proprietor: BenevolentAI Bio Limited, London W1T 5HD (GB)
(72) Inventor: BROWN, Alan, London W1T 5HD (GB); GLEN, Angela, London W1T 5HD (GB)
(74) Representative: Gill Jennings & Every LLP
(86) International application number: PCT/GB2019/052378
(87) International publication number: WO 2020/039209

(56) References cited:
- WO-A1-2012/034091
- WO-A1-2020/016594
- VADIM BERNARD-GAUTHIER ET AL: "Development of subnanomolar radiofluorinated (2-pyrrolidin-1-yl)imidazo[1,2-b]pyridazin e pan-Trk inhibitors as candidate PET imaging probes", MEDCHEMCOMM, vol. 6, no. 12, 1 January 2015 (2015-01-01), pages 2184-2193, XP055626148, United Kingdom ISSN: 2040-2503, DOI: 10.1039/C5MD00388A

## Description

The present invention relates to certain imidazo[1,2-b]pyridazine compounds and the pharmaceutically acceptable salts of such compounds. The invention also relates to compositions containing the compounds, and such compounds and salts in treating diseases or conditions associated with tropomyosin-related kinase (Trk), activity. More specifically the invention relates to the compounds and their salts useful as inhibitors of Trk.

Tropomyosin-related kinases (Trks) are a family of receptor tyrosine kinases activated by neurotrophins, a group of soluble growth factors including Nerve Growth Factor (NGF), Brain-Derived Neurotrophic Factor (BDNF) and Neurotrophin-3 (NT-3) and Neurotrophin-4/5 (NT-4/5). The Trk receptors include three family members TrkA, TrkB and TrkC that bind to and mediate the signal transduction derived from the Neurotrophins. NGF activates TrkA, BDNF and NT-4/5 activate TrkB and NT3 activates TrkC.

Tropomyosin-related kinases have been implicated in the following diseases: atopic dermatitis, psoriasis, eczema and prurigo nodularis, acute and chronic itch, pruritus, inflammation, cancer, restenosis, atherosclerosis, thrombosis, pruritus, lower urinary tract disorder, inflammatory lung diseases such as asthma, allergic rhinitis, lung cancer, psoriatic arthritis, rheumatoid arthritis, inflammatory bowel diseases such as ulcerative colitis, Crohn's disease, fibrosis, neurodegenerative disease, diseases disorders and conditions related to dysmyelination or demyelination, certain infectious diseases such as *Trypanosoma cruzi* infection, (Chagas disease), cancer related pain, chronic pain, neuroblastoma, ovarian cancer, colorectal cancer, melanoma, head and neck cancer, gastric carcimoma, lung carcinoma, breast cancer, glioblastoma, medulloblastoma, secratory breast cancer, salivary gland cancer, papillary thyroid carcinoma, adult myeloid leukaemia, tumour growth and metastasis and interstitial cystitis. (C. Potenzieri and B. J. Undem, Clinical & Experimental Allergy, 2012 (42) 8-19; Yamaguchi J, Aihara M, Kobayashi Y, Kambara T, Ikezawa Z, J Dermatol Sci. 2009;53:48-54; Dou YC, Hagstromer L, Emtestam L, Johansson O., Arch Dermatol Res. 2006;298:31-37; Johansson O, Liang Y, Emtestam L., Arch Dermatol Res. 2002;293:614-619; Grewe M, Vogelsang K, Ruzicka T, Stege H, Krutmann J., J Invest Dermatol. 2000;114:1108-1112; Urashima R, Mihara M .Virchows Arch. 1998;432:363-370; Kinkelin I, Motzing S, Koltenzenburg M, Brocker EB., Cell Tissue Res. 2000;302:31-37; Tong Liu & Ru-Rong Ji, Pflugers Arch - Eur J Physiol, DOI 10.1007/s00424-013-1284-2, published online 1 May 2013.); International Patent Application publication numbers WO2012/158413, WO2013/088256, WO2013/088257 and WO2013/161919, (Brodeur, G. M., Nat. Rev. Cancer 2003, 3, 203-216), (Davidson. B. , et al. , Clin. Cancer Res. 2003, 9, 2248-2259), (Bardelli, A , Science 2003, 300, 949), (Truzzi, F. , et al. , Dermato-Endocrinology 2008, 3 (I), pp. 32-36), Yilmaz,T. , et al. , Cancer Biology and Therapy 2010, 10 (6), pp. 644-653), (Du, J. et al. ,World Journal of Gastroenterology 2003, 9 (7), pp. 1431-1434), (Ricci A, et al., American Journal of Respiratory Cell and Molecular Biology 25 (4), pp. 439-446), (Jin, W. , et al. , Carcinogenesis 2010, 31 (11), pp. 1939-1947), (Wadhwa, S. , et al., Journal of Biosciences 2003, 28 (2), pp. 181-188), (Gruber-Olipitz, M. , et al. , Journal of Proteome Research 2008, 7 (5), pp. 1932-1944), (Euthus, D. M. et al. , Cancer Cell 2002, 2 (5), pp. 347-348),(Li, Y. -G. , et al., Chinese Journal of Cancer Prevention and Treatment 2009, 16 (6), pp. 428-430), (Greco, A , et al. , Molecular and Cellular Endocrinology 2010, 321 (I), pp. 44-49), (Eguchi, M., et al., Blood 1999, 93 (4), pp. 1355-1363), (Nakagawara, A (2001) Cancer Letters 169: 107-114; Meyer, J. et al. (2007) Leukemia,1-10; Pierottia, M. A and Greco A, (2006) Cancer Letters 232:90- 98; Eric Adriaenssens, E., et al. Cancer Res (2008) 68:(2) 346-351 ), (FreundMichel, V; Frossard, N. , Pharmacology ck Therapeutics (2008) 117(1), 52-76), (Hu Vivian Y; et. al. The Journal of Urology (2005), 173(3), 1016-21), (Di Mola, F. F, et. al. Gut (2000) 46(5), 670-678) (Dou, Y. -C. ,et. al. Archives of Dermatological Research (2006) 298(1), 31-37), (Raychaudhuri, S. P. , et al. , J. Investigative Dermatology (2004) 122(3), 812-819) and (de Melo-Jorge, M. et al. , Cell Host ck Microbe (2007) 1 (4), 251-261).

WO2012/034091 discloses imidazo[1,2-b]pyridazines which are tropomyosin kinase inhibitors.

The scope of the invention is as defined in the attached claims.

In one aspect, the invention provides a compound of Formula (I): 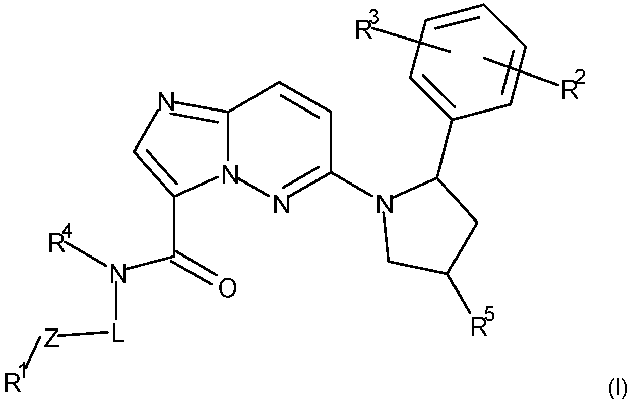 or a pharmaceutically acceptable salt or solvate thereof; wherein:
L is (CR⁶R⁷)ᵣ;
Z is absent or selected from:
   i) 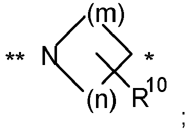 and
   ii) 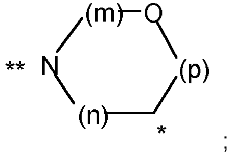
wherein * denotes the point of attachment to L and ** denotes the point of attachment to R1;
m is 1 or 2;
n is 1 or 2;
p is 0 or 1;
provided that the sum of m, n and p is in the range of 2 to 4;
r is 0 or 1;
R¹ is -XR⁹;
X is selected from -CH₂-, -C(O)-, and -S(O₂)-;
R² is -SR⁸;
R³ is selected from H and halo;
R⁴ is selected from H and (C₁-C₃)alkyl;
R⁵ is selected from H, hydroxyl and halo;
R⁶ and R⁷ are each independently selected from H and (C₁-C₃)alkyl;
R⁸ is methyl;
R⁹ is phenyl substituted by a group selected from hydroxy, -OC(O)(C₁-C₆)alkyl, C(O)OH and -C(O)O(C₁-C₆)alkyl, wherein the phenyl ring is optionally further substituted by halo; R¹⁰ is selected from H and (C₁-C₃)alkoxy.

In this aspect of the invention, the compound according to Formula (I) is not 6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboxamide, shown below: 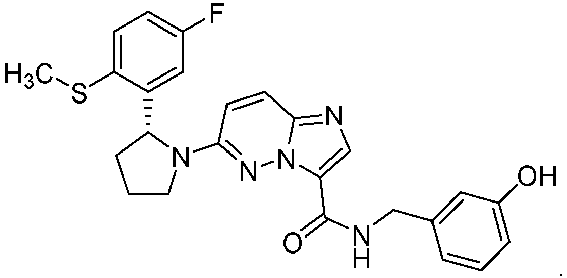 6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboxamide is included in the aspects of the invention that refer to a pharmaceutical composition, and aspects of the invention that refer to a compound for use as a pharmaceutical or for use in treating or preventing a condition or disorder which is mediated by Trk.

In one embodiment of the invention as defined anywhere above, R¹ is -CH₂R⁹.

In another embodiment of the invention as defined anywhere above, R³ is H or fluoro.

In another embodiment of the invention as defined anywhere above, R⁴ is H.

In another embodiment of the invention as defined anywhere above, R⁵ is H or fluoro.

In another embodiment of the invention as defined anywhere above, R⁵ is H.

In another embodiment of the invention as defined anywhere above, R⁶ is H.

In another embodiment of the invention as defined anywhere above, R⁹ is phenyl substituted by hydroxy wherein the hydroxyphenyl is optionally further substituted by fluoro.

In another embodiment of the invention as defined anywhere above, R¹⁰ is H.

In another embodiment of the invention as defined anywhere above, r is 0.

In another embodiment of the invention as defined anywhere above, Z is absent.

In another embodiment, the invention provides a compound of Formula I' 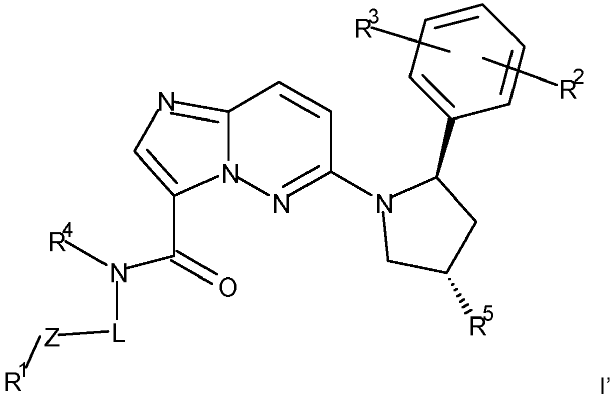 or a pharmaceutically acceptable salt or solvate thereof wherein R¹, R², R³, R⁴, R⁵, L and Z, are as defined anywhere hereinabove in respect of a compound of Formula I.

In another embodiment, the invention provides a compound of Formula Ia 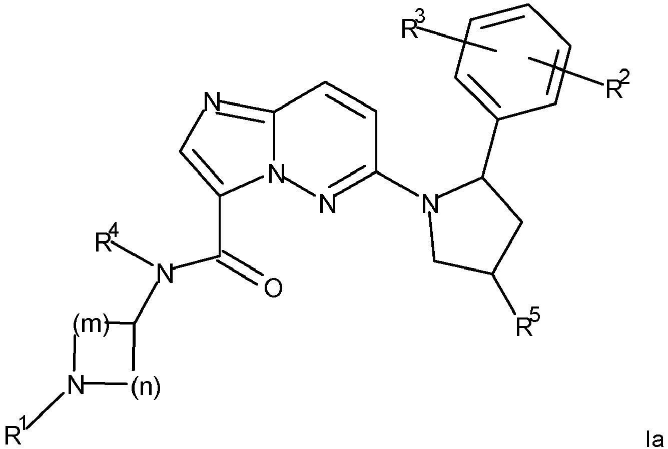 or a pharmaceutically acceptable salt or solvate thereof wherein R¹, R², R³, R⁴, R⁵, m and n, are as defined anywhere hereinabove in respect of a compound of Formula I.

In a further embodiment, the invention provides a compound of Formula la' 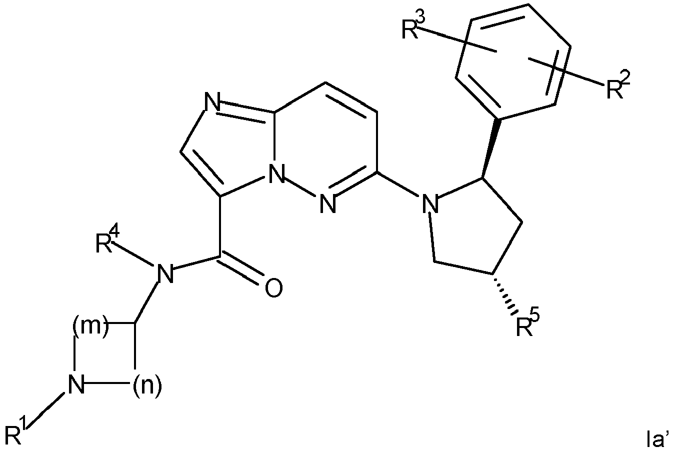 or a pharmaceutically acceptable salt or solvate thereof wherein R¹, R², R³, R⁴, R⁵, m and n, are as defined anywhere hereinabove in respect of a compound of Formula I.

In an alternative embodiment, the invention provides a compound of Formula Ib 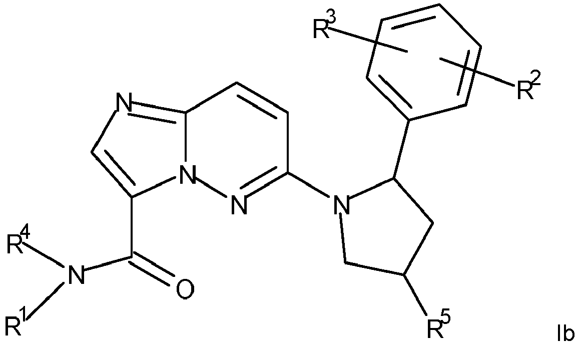 or a pharmaceutically acceptable salt or solvate thereof wherein R¹, R², R³, R⁴, R⁵, m and n, are as defined anywhere hereinabove in respect of a compound of Formula I.

In a further embodiment, the invention provides a compound of Formula Ib' 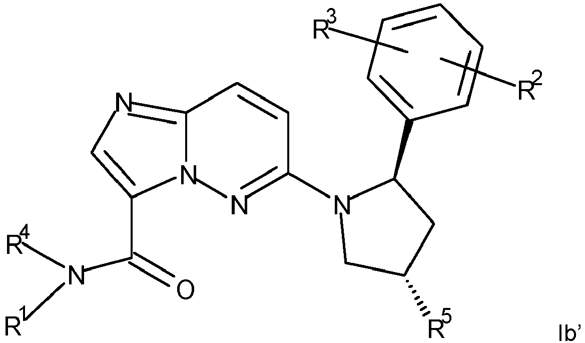 or a pharmaceutically acceptable salt or solvate thereof wherein R¹, R², R³, R⁴, R⁵, m and n, are as defined anywhere hereinabove in respect of a compound of Formula I.

In another embodiment, individual compounds according to the invention are those listed in the Examples section below.

In another embodiment of the invention, there is provided a compound according to the invention which is selected from Examples 1 to 31 or a pharmaceutically acceptable salt or solvate thereof.

In another embodiment of the invention, there is provided a compound according to the invention which is selected from:
6-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[2-[5-fluoro-2-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
6-[4-fluoro-2-[5-fluoro-2-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[4-fluoro-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[4-fluoro-2-[5-fluoro-2-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[4-fluoro-2-[5-fluoro-2-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[4-fluoro-2-[5-fluoro-2-(methylsulfanyl)phenyl]pyrrolidin-1-yl]-N-[(3-hydroxyphenyl)methyl]imidazo[1,2-b]pyridazine-3-carboxamide;
6-[4-fluoro-2-[5-fluoro-2-(methylsulfanyl)phenyl]pyrrolidin-1-yl]-N-[1-[(3-hydroxyphenyl)methyl]pyrrolidin-3-yl]imidazo[1,2-b]pyridazine-3-carboxamide;
3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}phenyl pentanoate;
Methyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[4-fluoro-2-[5-fluoro-2-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
Butyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
Ethyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[4-fluoro-2-[5-fluoro-2-(methylsulfanyl)phenyl]pyrrolidin-1-yl]-N-[(4-fluoro-3-hydroxyphenyl)methyl]imidazo[1,2-b]pyridazine-3-carboxamide;
N-[1-[(4-fluoro-3-hydroxyphenyl)methyl]pyrrolidin-3-yl]-6-[2-[3-fluoro-5-(methylsulfanyl)phenyl]pyrrolidin-1-yl]imidazo[1,2-b]pyridazine-3-carboxamide;
Methyl 3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
6-[4-fluoro-2-[3-fluoro-5-(methylsulfanyl)phenyl]pyrrolidin-1-yl]-N-[1-[(3-hydroxyphenyl)methyl]pyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-{1-[(4-fluoro-3-hydroxyphenyl) methyl]azetidin-3-yl}imidazo[1,2-b]pyridazine-3-carboxamide;
6-[4-fluoro-2-[3-fluoro-5-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[4-fluoro-2-[3-fluoro-5-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[4-fluoro-2-[3-fluoro-5-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[4-fluoro-2-[3-fluoro-5-(methylsulfanyl)phenyl]pyrrolidin-1-yl]-N-[1-[(4-fluoro-3-hydroxyphenyl)methyl]-4-methoxypyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-[4-[(4-fluoro-3-hydroxyphenyl)methyl]-1,4-oxazepan-6-yl]imidazo[1,2-b]pyridazine-3-carboxamide;
6-[4-fluoro-2-[3-fluoro-5-(methylsulfanyl)phenyl]pyrrolidin-1-yl]-N-[(4-fluoro-3-hydroxyphenyl)methyl]imidazo[1,2-b]pyridazine-3-carboxamide;
6-[4-fluoro-2-[3-fluoro-5-(methylsulfanyl)phenyl]pyrrolidin-1-yl]-N-[1-(3-hydroxybenzoyl)pyrrolidin-3-yl]imidazo[1,2-b]pyridazine-3-carboxamide;
3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}phenyl acetate;
3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid;
Butyl 3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
5- [4-fluoro-2-[3-fluoro-5-(methylsulfanyl)phenyl]pyrrolidin-1-yl]-N-{1-[(3-hydroxyphenyl)methyl]piperidin-4-yl}pyrazolo[1,5-a]pyrimidine-3-carboxamide;
or a pharmaceutically acceptable salt or solvate thereof.

In another embodiment of the invention, there is provided a compound according to the invention which is selected from:
6-[(2 R)-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[(2R)-2-[5-fluoro-2-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-[(3-hydroxyphenyl)methyl]imidazo[1,2-b]pyridazine-3-carboxamide;
6-[(2R,4S)-4-fluoro-2-[5-fluoro-2-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide;
3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}phenyl pentanoate;
Methyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[5-fluoro-2-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide; Butyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
Ethyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[5-fluoro-2-(methylsulfanyl)phenyl]pyrrolidin-1-yl]-N-[(4-fluoro-3-hydroxyphenyl)methyl]imidazo[1,2-b]pyridazine-3-carboxamide;
N-[(3S)-1-[(4-fluoro-3-hydroxyphenyl)methyl]pyrrolidin-3-yl]-6-[(2R)-2-[3-fluoro-5-(methylsulfanyl)phenyl]pyrrolidin-1-yl]imidazo[1,2-b]pyridazine-3-carboxamide;
Methyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[3-fluoro-5-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(4-fluoro-3-hydroxyphenyl) methyl]azetidin-3-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S,4S)-1-[(4-fluoro-3-hydroxyphenyl)methyl]-4-methoxypyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(6S)-4-[(4-fluoro-3-hydroxyphenyl)methyl]-1,4-oxazepan-6-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(6R)-4-[(4-fluoro-3-hydroxyphenyl)methyl]-1,4-oxazepan-6-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(4-fluoro-3-hydroxyphenyl)methyl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S)-1-(3-hydroxybenzoyl)pyrrolidin-3-yl]imidazo[1,2-b]pyridazine-3-carboxamide;
3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}phenyl acetate;
3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid;
Butyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
5-[(2R,4S)-4-fluoro-2-[3-fluoro-5-(methylsulfanyl)phenyl]pyrrolidin-1-yl]-N-{1-[(3-hydroxyphenyl)methyl]piperidin-4-yl}pyrazolo[1,5-a]pyrimidine-3-carboxamide; or a pharmaceutically acceptable salt or solvate thereof.

In the embodiments mentioned herein, where only certain variables are defined, it is intended that the remainder of the variables are as defined in any embodiment herein. Thus, the invention provides for the combination of limited or optional definitions of variables.

The following terms as used herein are intended to have the following meanings:
"Optionally substituted" as used herein means the group referred to can be unsubstituted, or substituted at one or two or three positions by any one or any combination of the substituents listed thereafter.

As used herein, the term "halogen" or "halo" refers to fluoro, chloro, bromo, and iodo.

As used herein, the term "alkyl" refers to a fully saturated branched or unbranched hydrocarbon moiety having up to 20 carbon atoms. Unless otherwise provided, alkyl refers to hydrocarbon moieties having 1 to 16 carbon atoms, 1 to 10 carbon atoms, 1 to 7 carbon atoms, or 1 to 4 carbon atoms. Representative examples of alkyl include, but are not limited to, methyl, ethyl, n-propyl, iso-propyl, n-butyl, sec-butyl, iso-butyl, *tert-butyl, n-*pentyl, isopentyl, neopentyl, n-hexyl, 3-methylhexyl, 2,2- dimethylpentyl, 2,3-dimethylpentyl, n-heptyl, n-octyl, n-nonyl, n-decyl and the like.

"C₁-C₃ alkyl", "C₁-C₆ alkyl", "C₁-C₈ alkyl" and the like, as used herein, denotes an alkyl group that contains one to three, six or eight (or the relevant number) carbon atoms.

As used herein, the term "cycloalkyl" refers to saturated or unsaturated non-aromatic monocyclic, bicyclic or tricyclic hydrocarbon groups of 3-12 carbon atoms. Unless otherwise provided, cycloalkyl refers to cyclic hydrocarbon groups having between 3 and 9 ring carbon atoms or between 3 and 7 ring carbon atoms. Exemplary monocyclic hydrocarbon groups include, but are not limited to, cyclopropyl, cyclobutyl, cyclopentyl, cyclopentenyl, cyclohexyl and cyclohexenyl and the like. Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl and the like.

"C₃-C₈-cycloalkyl" denotes a cycloalkyl group having 3 to 8 ring carbon atoms, for example a monocyclic group such as a cyclopropyl, cyclobutyl, cyclopentyl or cyclohexyl, cycloheptyl, cyclooctyl, cyclononyl or cyclodecyl, or a bicyclic group such as bicycloheptyl or bicyclooctyl. Different numbers of carbon atoms may be specified, with the definition being amended accordingly.

As used herein, the term "alkoxy" refers to alkyl-O-, wherein alkyl is defined herein above. Representative examples of alkoxy include, but are not limited to, methoxy, ethoxy, propoxy, 2-propoxy, butoxy, tert-butoxy, pentyloxy, hexyloxy, cyclopropyloxy-, cyclohexyloxy- and the like. Typically, alkoxy groups have about 1-7, more preferably about 1-4 carbons.

As used herein, the term "heterocycloalkyl" refers to a saturated or unsaturated non-aromatic ring or ring system, e.g., which is a 4-, 5-, 6-, or 7-membered monocyclic, 7-, 8-, 9-, 10-, 11-, or 12-membered bicyclic or 10-, 11-, 12-, 13-, 14- or 15-membered tricyclic ring system and contains at least one heteroatom selected from O, S and N, where the N and S can also optionally be oxidized to various oxidation states. The heterocyclic group can be attached at a heteroatom or a carbon atom. A C-linked heterocyclic group can be attached at a carbon atom. Examples of heterocycles include tetrahydrofuran (THF), dihydrofuran, 1, 4-dioxane, morpholine, 1,4-dithiane, piperazine, piperidine, 1,3-dioxolane, imidazolidine, imidazoline, pyrroline, pyrrolidine, tetrahydropyran, dihydropyran, oxathiolane, dithiolane, 1,3-dioxane, 1,3-dithiane, oxathiane, thiomorpholine, homomorpholine, and the like.

Throughout this specification and in the claims that follow, unless the context requires otherwise, the word "comprise", or variations such as "comprises" or "comprising", should be understood to imply the inclusion of a stated integer or step or group of integers or steps but not the exclusion of any other integer or step or group of integers or steps.

The compounds of the invention include compounds of formula (I), and salts thereof as hereinafter defined, polymorphs, isomers and solvates thereof (including optical, geometric and tautomeric isomers) as hereinafter defined and isotopically-labelled compounds of formula (I).

The invention includes also pharmaceutically acceptable salts of a compound of Formula (I). A "pharmaceutically acceptable salt" is intended to mean a salt of a free acid or base of a compound represented by Formula (I),that is non-toxic, biologically tolerable, or otherwise biologically suitable for administration to a subject. See, generally, G.S. Paulekuhn, et al., "Trends in Active Pharmaceutical Ingredient Salt Selection based on Analysis of the Orange Book Database", J. Med. Chem., 2007, 50:6665-72, S.M. Berge, et al., "Pharmaceutical Salts", J Pharm Sci., 1977, 66:1 -19, and Handbook of Pharmaceutical Salts, Properties, Selection, and Use, Stahl and Wermuth, Eds., Wiley-VCH and VHCA, Zurich, 2002.

Examples of pharmaceutically acceptable salts are those that are pharmacologically effective and suitable for contact with the tissues of subjects without undue toxicity, irritation, or allergic response. A compound of Formula (I), may possess a sufficiently acidic group, a sufficiently basic group, or both types of functional groups, and accordingly react with a number of inorganic or organic bases, and inorganic and organic acids, to form a pharmaceutically acceptable salt.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate , trifluoroacetate and trifluoromethylsulfonate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, trifluoromethylsulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

Examples of pharmaceutically acceptable salts particularly include sulfates, pyrosulfates, bisulfates, sulfites, bisulfites, phosphates, monohydrogen- phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1 ,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1 -sulfonates, naphthalene-2-sulfonates, and mandelates.

Additionally, any formula given herein is intended to refer also to hydrates, solvates, and polymorphs of such compounds, and mixtures thereof, even if such forms are not listed explicitly. A compound of Formula (I), or pharmaceutically acceptable salt of a compound of Formula (I) may be obtained as a solvate. Solvates include those formed from the interaction or complexation of compounds of the invention with one or more solvents, either in solution or as a solid or crystalline form. In some embodiments, the solvent is water and then the solvates are hydrates. In addition, certain crystalline forms of a compound of Formula (I), or a pharmaceutically acceptable salt of a compound of Formula (I), may be obtained as co-crystals. In certain embodiments of the invention, a compound of Formula (I), or a pharmaceutically acceptable salt of a compound of Formula (I), may be obtained in a crystalline form. In other embodiments, a compound of Formula (I), may be obtained in one of several polymorphic forms, as a mixture of crystalline forms, as a polymorphic form, or as an amorphous form. In other embodiments, a compound of Formula (I), may convert in solution between one or more crystalline forms and/or polymorphic forms.

Compounds of the invention that contain groups capable of acting as donors and/or acceptors for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the invention further provides co-crystals comprising a compound of formula (I).

Any formula given herein is intended to represent compounds having structures depicted by the structural formula as well as certain variations or forms. In particular, compounds of any formula given herein may have asymmetric centres and therefore exist in different enantiomeric forms. All optical isomers and stereoisomers of the compounds of the general formula, and mixtures thereof, are considered within the scope of the formula. Thus, any formula given herein is intended to represent a racemate, one or more enantiomeric forms, one or more diastereomeric forms, one or more atropisomeric forms, and mixtures thereof. Furthermore, certain structures may exist as geometric isomers (i.e., *cis* and *trans* isomers), as tautomers, or as atropisomers.

Included within the scope of the claimed compounds of the present invention are all stereoisomers, geometric isomers and tautomeric forms of the compounds of Formula (I), including compounds exhibiting more than one type of isomerism, and mixtures of one or more thereof. Also included are acid addition or base addition salts wherein the counter ion is optically active, for example, D-lactate or L-lysine, or racemic, for example, DL-tartrate or DL-arginine.

Where a compound of Formula (I) contains for example, a keto or guanidine group or an aromatic moiety, tautomeric isomerism ('tautomerism') can occur. It follows that a single compound may exhibit more than one type of isomerism. Examples of types of potential tautomerisms shown by the compounds of the invention include; amide ⇔ hydroxyl-imine and keto ⇔ enol tautomersims:

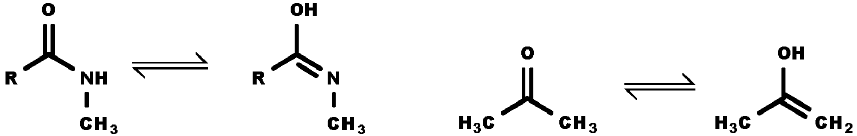

*Cis*/*trans* isomers may be separated by conventional techniques well known to those skilled in the art, for example, by chromatography and fractional crystallisation.

Conventional techniques for the preparation/isolation of individual enantiomers include chiral synthesis from a suitable optically pure precursor or resolution of the racemate (or the racemate of a salt or other derivative) using, for example, chiral high pressure liquid chromatography (HPLC).

Chiral compounds of the invention (and chiral precursors thereof) may be obtained in enantiomerically-enriched form using chromatography, typically HPLC, on a resin with an asymmetric stationary phase and with a mobile phase consisting of a hydrocarbon, typically heptane or hexane, containing from 0 to 50% ethanol, typically from 2 to 20%. Concentration of the eluate affords the enriched mixture.

Mixtures of stereoisomers may be separated by conventional techniques known to those skilled in the art (see, for example, *"*Stereochemistry of Organic Compounds" by E L Eliel (Wiley, New York, 1994)).

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non- superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included. Tautomers are one of two or more structural isomers that exist in equilibrium and are readily converted from one isomeric form to another.

Examples of tautomers include but are not limited to those compounds defined in the claims.

Any asymmetric atom (e.g., carbon or the like) of the compound(s) of the present invention can be present in racemic or enantiomerically enriched, for example the (R)-, (S)- or (R,S)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (R)- or (S)- configuration. Substituents at atoms with unsaturated bonds may, if possible, be present in *cis- (Z)-* or *trans- (E)-* form.

Accordingly, as used herein a compound of the present invention can be in the form of one of the possible isomers, rotamers, atropisomers, tautomers or mixtures thereof, for example, as substantially pure geometric *(cis* or *trans)* isomers, diastereomers, optical isomers (antipodes), racemates or mixtures thereof.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of final products or intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-O,O'-p-toluoyl tartaric acid, mandelic acid, malic acid or camphor-10-sulfonic acid. Racemic products can also be resolved by chiral chromatography, e.g., high pressure liquid chromatography (HPLC) using a chiral adsorbent.

Since the compounds of the invention are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1 %, more suitably at least 5% and preferably from 10 to 59% of a compound of the invention.

When both a basic group and an acid group are present in the same molecule, the compounds of the present invention may also form internal salts, e.g., zwitterionic molecules.

Any formula given herein is also intended to represent unlabelled forms as well as isotopically labelled forms of the compounds. Isotopically labelled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, and fluorine, such as ²H, ³H, ¹¹C, ¹³C, ¹⁴C, ¹³N, ¹⁵N, ¹⁵O, ¹⁷0, ¹⁸0, ¹⁸F, respectively. Such isotopically labelled compounds are useful in metabolic studies (preferably with ¹⁴C), reaction kinetic studies (with, for example ²H or ³H), detection or imaging techniques (such as positron emission tomography (PET) or single-photon emission computed tomography (SPECT)) including drug or substrate tissue distribution assays, or in radioactive treatment of subjects. Substitution with positron emitting isotopes, such as ¹¹C, ¹⁸F, ¹⁵O and ¹³N, can be useful in PET studies for examining substrate receptor occupancy. In particular, an ¹⁸F or ¹¹C labelled compound may be particularly preferred for PET studies. Further, substitution with heavier isotopes such as deuterium (i.e., ²H) may afford certain therapeutic advantages resulting from greater metabolic stability, for example increased *in vivo* half-life or reduced dosage requirements. Certain isotopically-labelled compounds of formula (I) for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Isotopically labelled compounds of this invention can generally be prepared by carrying out the procedures disclosed in the schemes or in the examples and preparations described below by substituting a readily available isotopically labelled reagent for a non-isotopically labelled reagent.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Pharmaceutically acceptable solvates in accordance with the invention include those wherein the solvent of crystallization may be isotopically substituted, e.g. D₂O, d₆-acetone, d₆-DMSO.

Exemplary compounds of the invention, and exemplary compounds for use according to the invention will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

All of the derivatives of Formula (I) can be prepared by the procedures described in the general methods presented below or by routine modifications thereof.

The routes below, including those mentioned in the Examples and Preparations, illustrate methods of synthesising the compound of Formula (I). The skilled person will appreciate that the compound of the invention, and intermediates thereto, could be made by methods other than those specifically described herein, for example by adaptation of the methods described herein, for example by methods known in the art. Suitable guides to synthesis, functional group interconversions, use of protecting groups, etc., are for example: "Comprehensive Organic Transformations" by RC Larock, VCH Publishers Inc. (1989); "Advanced Organic Chemistry" by J. March, Wiley Interscience (1985); *"*Designing Organic Synthesis" by S Warren, Wiley Interscience (1978); *"*Organic Synthesis - The Disconnection Approach" by S Warren, Wiley Interscience (1982); *"*Guidebook to Organic Synthesis" by RK Mackie and DM Smith, Longman (1982); *"*Protective Groups in Organic Synthesis" by TW Greene and PGM Wuts, Fifth Ed, John Wiley and Sons, Inc. (2014); and *"*Protecting Groups" by PJ, Kocienski, Georg Thieme Verlag (1994); and any updated versions of these standard works.

In addition, the skilled person will appreciate that it may be necessary or desirable at any stage in the synthesis of compounds of the invention to protect one or more sensitive groups, so as to prevent undesirable side reactions. In particular, it may be necessary or desirable to protect phenol or carboxylic acid groups. The protecting groups used in the preparation of the compounds of the invention may be used in a conventional manner. See, for example, those described in *'*Greene's Protective Groups in Organic Synthesis' by Theodora W Greene and Peter G M Wuts, fifth edition, (John Wiley and Sons, 2014), in particular Chapter 3 ("Protection for Phenols*")* Chapter 5 ("Protection for the Carboxyl group*"),* and Chapter 7 ("Protection for the Amino Group"), incorporated herein by reference, which also describes methods for the removal of such groups.

In the general synthetic methods below, unless otherwise specified, the substituents are as defined above with reference to the compound of Formula (I), above.

Where ratios of solvents are given, the ratios are by volume.

The skilled person will appreciate that the experimental conditions set forth in the schemes that follow are illustrative of suitable conditions for effecting the transformations shown, and that it may be necessary or desirable to vary the precise conditions employed for the preparation of the compound of Formula (**I**). It will be further appreciated that it may be necessary or desirable to carry out the transformations in a different order from that described in the schemes, or to modify one or more of the transformations, to provide the desired compound of the invention.

Compounds prepared according to the schemes described above may be obtained as single enantiomers, diastereomers, or regioisomers, by enantio- , diastero-, or regiospecific synthesis, or by resolution. Compounds prepared according to the schemes above may alternately be obtained as racemic (1:1) or non-racemic (not 1:1) mixtures or as mixtures of diastereomers or regioisomers. Where racemic and non-racemic mixtures of enantiomers are obtained, single enantiomers may be isolated using conventional separation methods known to one skilled in the art, such as chiral chromatography, recrystallization, diastereomeric salt formation, derivatization into diastereomeric adducts, biotransformation, or enzymatic transformation. Where regioisomeric or diastereomeric mixtures are obtained, single isomers may be separated using conventional methods such as chromatography or crystallization.

The compounds of the invention may be prepared by any method known in the art for the preparation of compounds of analogous structure. In particular, the compound of the invention can be prepared by the procedures described by reference to the Schemes that follow, or by the specific methods described in the Examples, or by similar processes to either.

The skilled person will appreciate that the experimental conditions set forth in the schemes that follow are illustrative of suitable conditions for effecting the transformations shown, and that it may be necessary or desirable to vary the precise conditions employed for the preparation of the compound of Formula (**I**). It will be further appreciated that it may be necessary or desirable to carry out the transformations in a different order from that described in the schemes, or to modify one or more of the transformations, to provide the desired compound of the invention
A compound of Formula (I) may be prepared from the compounds of Formulae (II), (III), (IV), (V), and (VI) as illustrated by **Scheme 1.** 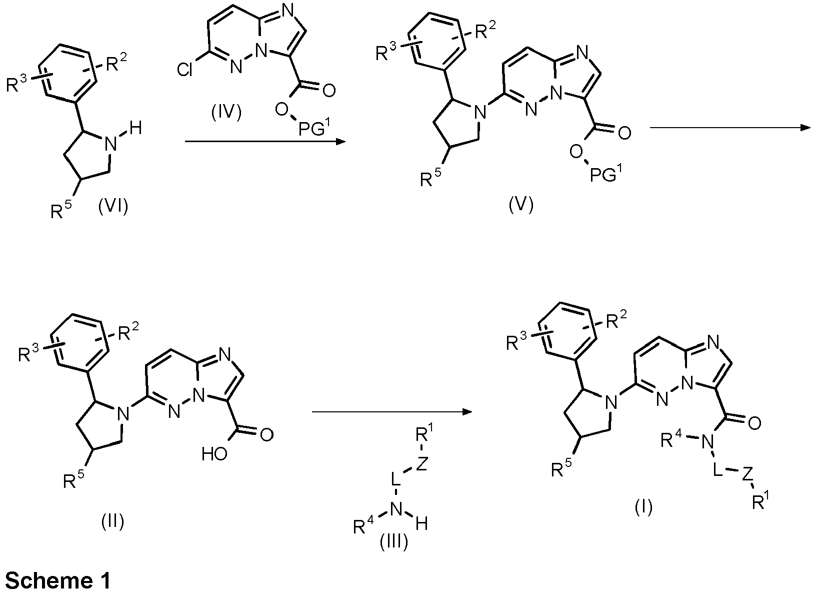 PG¹ is C1-C4 alkyl, preferably Me or Et

The amine of Formula (III) is commercially available or may be prepared by analogy to methods known in the literature or as illustrated in **Scheme 6.**

The chloride of Formula (IV) is commercially available or may be prepared by analogy to methods known in the literature.

Compounds of Formula (VI) are commercially available or may be prepared in chiral form by analogy with the methods described by Brinner et. al. (Org. Biomol. Chem., 2005,3, 2109-2113) or Fan et.al. (WO2012 034091). Alternatively, compounds of Formula (VI) may be prepared by analogy with the methods described by Huihui et. al. (J.A.C.S., 2016, 138, 5016-5019).

The compound of Formula (V) may be prepared by treatment of the amine of Formula (VI) with the chloride of Formula (IV), in the presence of an inorganic base in a polar aprotic solvent at elevated temperature. Preferred conditions, comprise treatment of the compound of Formula (IV) with the amine of Formula (VI) in the presence of KF in a solvent such as DMSO at elevated temperature, typically 130°C.

The compound of Formula (II) may be prepared by the hydrolysis of the compound of Formula (V) under suitable acidic or basic conditions in a suitable aqueous solvent.

Preferred conditions comprise the treatment of the ester of Formula (V) with KOH in aqueous EtOH at room temperature.

The compound of Formula (I) may be prepared by an amide bond formation of the acid of Formula (II) and the amine of Formula (III) in the presence of a suitable coupling agent and organic base in a suitable polar aprotic solvent. Preferred conditions, comprise the reaction of the acid of Formula (II) with the amine of Formula (III) in the presence of HATU or TPTU, in the presence of a suitable organic base, typically DIPEA in a suitable solvent, such as DMF at room temperature.

When R⁹ is a hydroxyl substituted phenyl group an appropriate phenol protecting group strategy, as selected by a person skilled in the art, may be employed, such as for example a silyl protecting group.

Alternatively, a compound of Formula (I) may be prepared from the compounds of Formulae (II), (III) and (VII) as illustrated by **Scheme 2.**

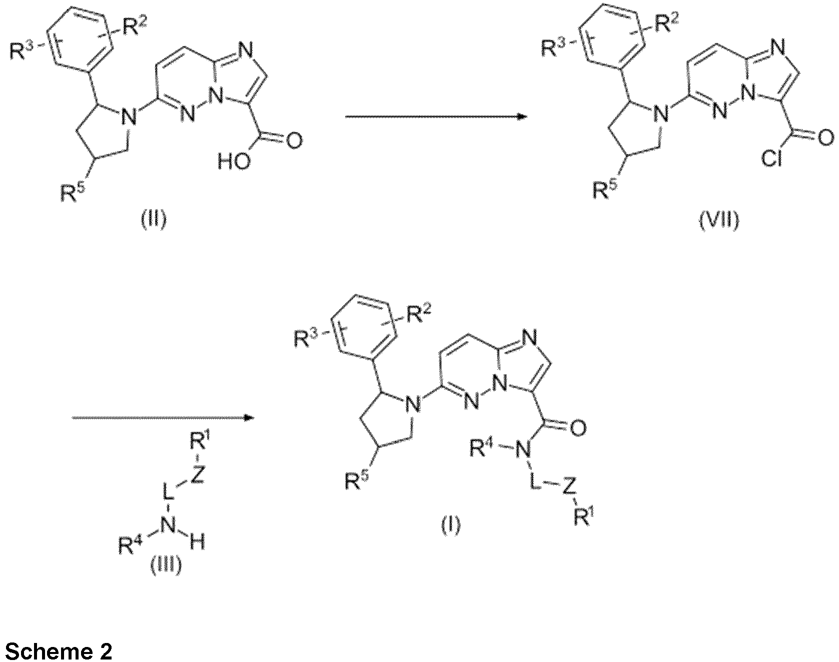

The amine of Formula (I) may be prepared by formation of the acid chloride of Formula (VII) from the acid of Formula (II), typically using oxalyl chloride and DMF in DCM at room temperature and the subsequent amide bond formation of the acid chloride of Formula (VII) and the amine of Formula (III) in the presence of a suitable organic base, typically triethylamine at 0°C.

Alternatively, compounds of Formula (I), wherein Z is present may be prepared from compounds of Formulae (VIII), (IX), (X) and (XI) using either a reductive amination (a), amidation (b) or sulphonamide formation reaction as illustrated in **Scheme 3.**

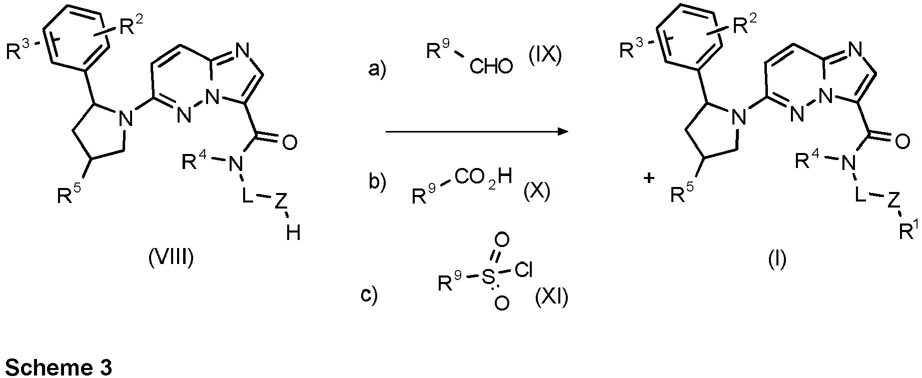

When Z is present and X is -CH₂-, the compound of Formula (I) may be prepared by the reductive amination (alternatively known as reductive alkylation) of an amine of Formula (VIII) with an aldehyde of Formula (IX) using a suitable reducing agent such as sodium triacetoxyborohydride in a suitable solvent such as DCM at an appropriate temperature such as room temperature.

When Z is present and X is -C(O)-, the amide of Formula (I) may be prepared by an amide bond formation of the acid of Formula (X) and the amine of Formula (VIII) in the presence of a suitable coupling agent and organic base, as previously described in **Scheme 1.** Preferred conditions comprise reaction of the acid of Formula (X) with the amine of Formula (X) in the presence of HATU, in the presence of a suitable organic base, typically DIPEA in DMF at room temperature.

When Z is present and X is -S(O)₂-, the sulfonamide of Formula (I) may be prepared by reaction of the amine of Formula (VIII) with a sulfonyl chloride of Formula (XI) in the presence of an organic base, such as Et₃N or DIPEA, in a suitable solvent such as DCM at room temperature.

Where R⁹ is a hydroxy substituted phenyl group an appropriate phenol protecting group strategy, as selected by a skilled person, may be employed.

Where R⁹ is a carboxyl substituted phenyl group an appropriate acid protecting group strategy, as selected by a skilled person, may be employed. Preferably, the protecting group is an alkyl ester, such as methyl.

Compounds of Formula (VIII), may be prepared from compounds of Formulae (II), (XII), and (XIII), as illustrated in **Scheme 4.**

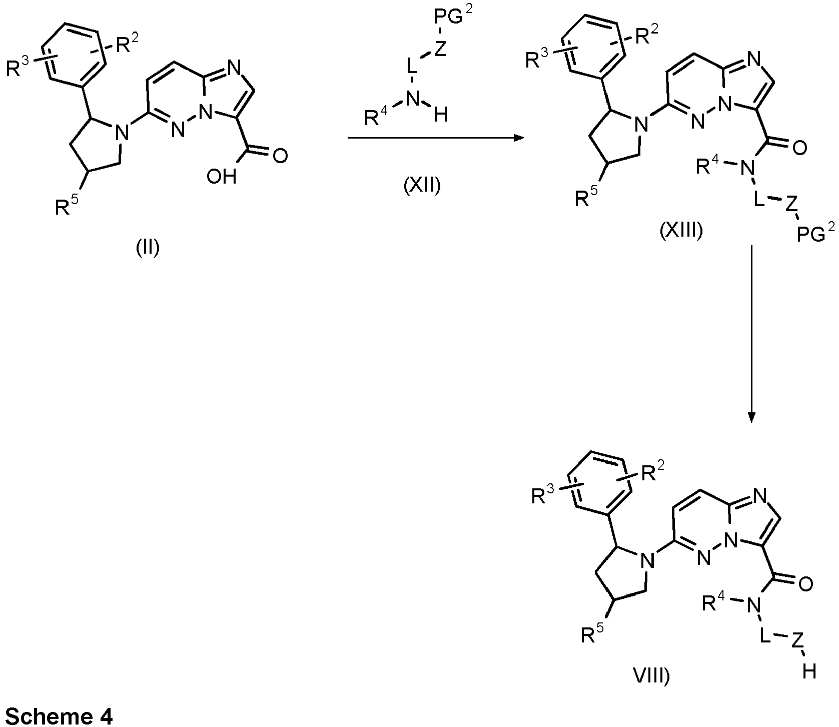

PG² is a N protecting group, typically a carbamate and preferably Boc.

The amine of Formula (XII) is commercially available or may be prepared by analogy to methods known in the literature or as illustrated in Scheme 6.

The amide of Formula (XIII) may be prepared by an amide bond formation of the acid of Formula (II) and the amine of Formula (XII) in the presence of a suitable coupling agent and organic base, as previously described in **Scheme 1.** Preferred conditions comprise reaction of the acid of Formula (II) with the amine of Formula (XII) in the presence of HATU, in the presence of a suitable organic base, typically DIPEA in DMF at room temperature. The amine of Formula (VIII) may be prepared by a suitable deprotection reaction typically involving treatment of the compound of Formula (XIII) with an acid such as HCl or TFA in a suitable aprotic solvent such as DCM or dioxane at an appropriate temperature such as 0°C to reflux temperature, preferably at room temperature.

Compounds of Formula (III), may be prepared from compounds of Formulae (XIV), (XV), (XVI) and (XVII) as illustrated in **Scheme 5.**

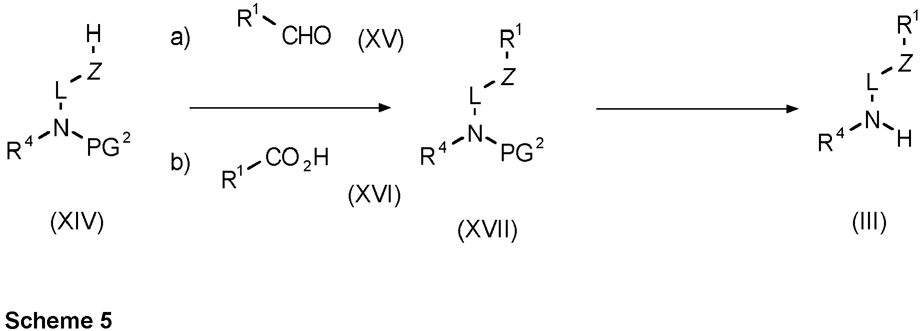

PG² is a suitable amine protecting group, typically a carbamate and preferably Boc.

The compounds of Formulae (XIV), (XV) and (XVI) are commercially available or may be prepared by analogy to methods known in the literature.

The amine of Formula (XVII) may be prepared using either a reductive amination (a) or amidation (b) procedure of compounds of Formula (XV) and (XVI) as previously described in **Scheme 3.**

The amine of Formula (III) may be prepared by a suitable amine deprotection reaction as previously described in **Scheme 4.**

In a further embodiment, compounds of Formula (I) may be converted to alternative compounds of Formula (I) using standard chemical transformations as illustrated in **Scheme 6** and **Scheme 7.**

Compounds of Formula (IB), wherein R⁹ is phenyl substituted by -OC-(O)(C₁-C₆)alkyl, may be prepared from compounds of Formula (IA), wherein R⁹ is phenyl substituted by OH, by treatment with a suitable (C₁-C₆)COCl or anhydride in the presence of an organic base, such as pyridine at room temperature.

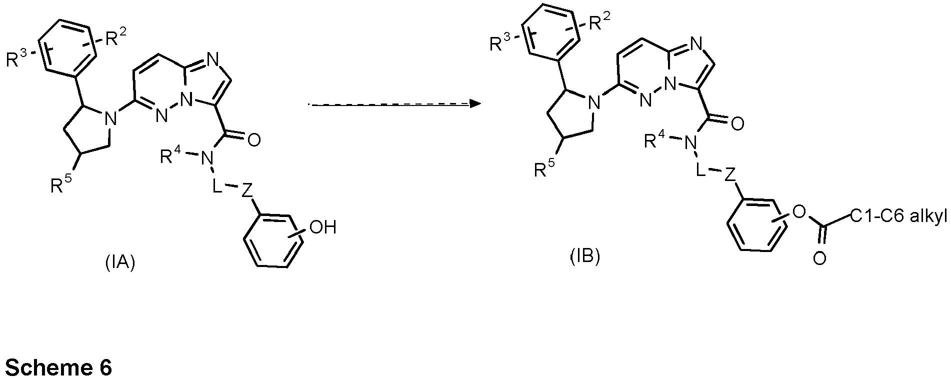

Compounds of Formula (ID), wherein R⁹ is phenyl substituted by -C-(O)O(C₁-C₆)alkyl, may be prepared from compounds of Formula (IC), wherein R⁹ is phenyl substituted by-C(O)₂H, by treatment with a suitable (C₁-C₆)OH in the presence of a suitable coupling agent such as DMAP and EDC.HCl at room temperature as illustrated in Scheme 7.

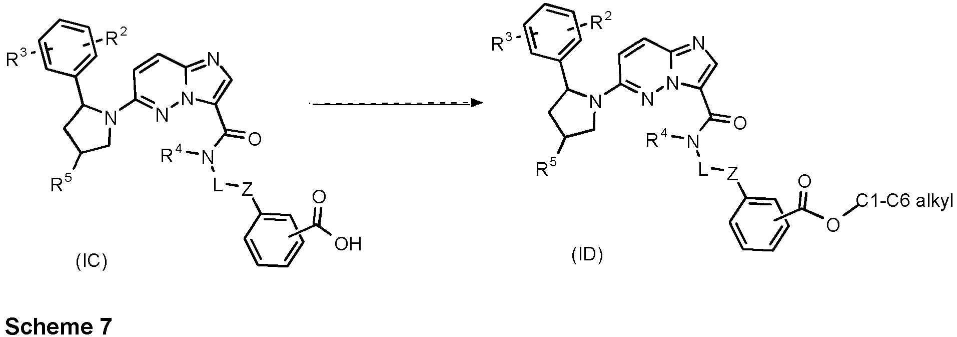

The above general schemes may be used to prepare compounds of the present invention.The desired specific compounds can be prepared by selecting the appropriate starting materials, reactants and reaction conditions.

The starting materials and reagents in the above scheme are all either available commercially or can be prepared following literature precedents.

Within the scope of this text, only a readily removable group that is not a constituent of the particular desired end product of the compounds of the present invention is designated a "protecting group", unless the context indicates otherwise. The protection of functional groups by such protecting groups, the protecting groups themselves, and their cleavage reactions are described for example in standard reference works, such as *'*Greene's Protective Groups in Organic Synthesis' by Theodora W Greene and Peter G M Wuts, fifth edition, (John Wiley and Sons, 2014), in particular Chapter 3 ("*Protection for Phenols*") and Chapter 5 ("*Protection for the Carboxyl group")*, incorporated herein by reference, which also describes methods for the removal of such groups , in J. F. W. McOmie, "Protective Groups in Organic Chemistry", Plenum Press, London and New York 1973, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, in H.-D. Jakubke and H. Jeschkeit, "Aminosäuren, Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974. A characteristic of protecting groups is that they can be removed readily (i.e. without the occurrence of undesired secondary reactions) for example by solvolysis, reduction, photolysis or alternatively under physiological conditions (e.g. by enzymatic cleavage).

Salts of compounds of the present invention having at least one salt-forming group may be prepared in a manner known to those skilled in the art. For example, salts of compounds of the present invention having acid groups may be formed, for example, by treating the compounds with metal compounds, such as alkali metal salts of suitable organic carboxylic acids, e.g. the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent preferably being used. Acid addition salts of compounds of the present invention are obtained in customary manner, e.g. by treating the compounds with an acid or a suitable anion exchange reagent. Internal salts of compounds of the present invention containing acid and basic salt-forming groups, e.g. a free carboxy group and a free amino group, may be formed, e.g. by the neutralisation of salts, such as acid addition salts, to the isoelectric point, e.g. with weak bases, or by treatment with ion exchangers. Salts can be converted into the free compounds in accordance with methods known to those skilled in the art. Metal and ammonium salts can be converted, for example, by treatment with suitable acids, and acid addition salts, for example, by treatment with a suitable basic agent.

Mixtures of isomers obtainable according to the invention can be separated in a manner known to those skilled in the art into the individual isomers; diastereoisomers can be separated, for example, by partitioning between polyphasic solvent mixtures, recrystallisation and/or chromatographic separation, for example over silica gel or by *e.g.* medium pressure liquid chromatography over a reversed phase column, and racemates can be separated, for example, by the formation of salts with optically pure salt-forming reagents and separation of the mixture of diastereoisomers so obtainable, for example by means of fractional crystallisation, or by chromatography over optically active column materials.

Intermediates and final products can be worked up and/or purified according to standard methods, e.g. using chromatographic methods, distribution methods, (re-) crystallization, and the like.

The following applies in general to all processes mentioned herein before and hereinafter.

All the above-mentioned process steps can be carried out under reaction conditions that are known to those skilled in the art, including those mentioned specifically, in the absence or, customarily, in the presence of solvents or diluents, including, for example, solvents or diluents that are inert towards the reagents used and dissolve them, in the absence or presence of catalysts, condensation or neutralizing agents, for example ion exchangers, such as cation exchangers, e.g. in the H+ form, depending on the nature of the reaction and/or of the reactants at reduced, normal or elevated temperature, for example in a temperature range of from about -100 °C to about 190 °C, including, for example, from approximately -80 °C to approximately 150 °C, for example at from -80 to -60 °C, at room temperature, at from -20 to 40 °C or at reflux temperature, under atmospheric pressure or in a closed vessel, where appropriate under pressure, and/or in an inert atmosphere, for example under an argon or nitrogen atmosphere.

At all stages of the reactions, mixtures of isomers that are formed can be separated into the individual isomers, for example diastereoisomers or enantiomers, or into any desired mixtures of isomers, for example racemates or mixtures of diastereoisomers, for example analogously to the methods described under "Additional process steps".

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofuran or dioxane, liquid aromatic hydrocarbons, such as benzene or toluene, alcohols, such as methanol, ethanol or 1- or 2-propanol, nitriles, such as acetonitrile, halogenated hydrocarbons, such as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopentane, methycyclohexane, or mixtures of those solvents, for example aqueous solutions, unless otherwise indicated in the description of the processes. Such solvent mixtures may also be used in working up, for example by chromatography or partitioning.

The compounds, including their salts, may also be obtained in the form of hydrates, or their crystals may, for example, include the solvent used for crystallization. Different crystalline forms may be present.

Also described herein are forms of the process in which a compound obtainable as an intermediate at any stage of the process is used as starting material and the remaining process steps are carried out, or in which a starting material is formed under the reaction conditions or is used in the form of a derivative, for example in a protected form or in the form of a salt, or a compound obtainable by the process according to the invention is produced under the process conditions and processed further in situ.

All starting materials, building blocks, reagents, acids, bases, dehydrating agents, solvents and catalysts utilized to synthesize the compounds of the present invention are either commercially available or can be produced by organic synthesis methods known to one of ordinary skill in the art (Houben-Weyl 4th Ed. 1952, Methods of Organic Synthesis, Thieme, Volume 21).

Also provided herein is a process for the preparation of compounds of formula I or a pharmaceutically acceptable salt or solvate thereof.

Also provided herein is a process of preparing a compound of the present invention or a pharmaceutically acceptable salt or solvate thereof comprising the step of:
amide bond formation via acid-amine coupling of the acid of Formula (II) 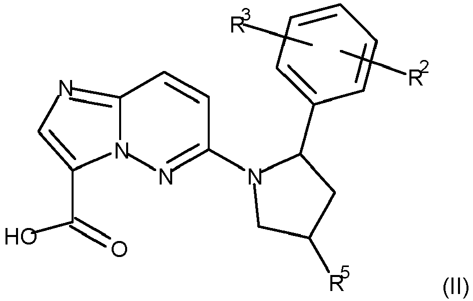
and the amine of Formula (III)

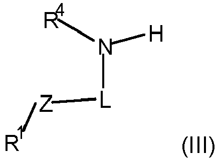

in the presence of a suitable coupling agent and organic base in a suitable polar aprotic solvent., wherein R¹, R², R³, R⁴and R⁵, L and Z are as defined anywhere hereinabove in respect of a compound of Formula I.

Also provided herein is a process of preparing a compound of the present invention or a pharmaceutically acceptable salt or solvate thereof comprising the step of:
amide bond formation of the acid chloride of Formula (VII) 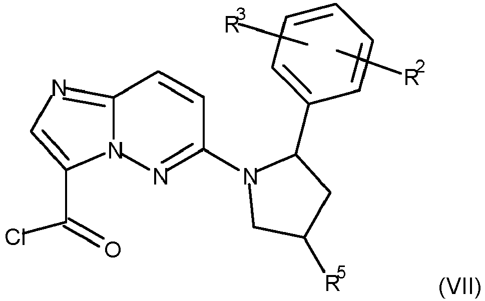
and the amine of Formula (III)

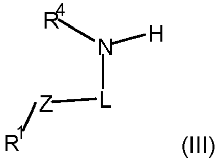

in the presence of a suitable coupling agent and organic base in a suitable polar aprotic solvent., wherein R¹, R², R³, R⁴and R⁵, L and Z are as defined anywhere hereinabove in respect of a compound of Formula I.

Also provided herein is a process of preparing a compound of the present invention or a pharmaceutically acceptable salt or solvate thereof, wherein Z is present and X is -CH₂-, comprising the step of:
reductive amination of an amine of Formula (VIII) 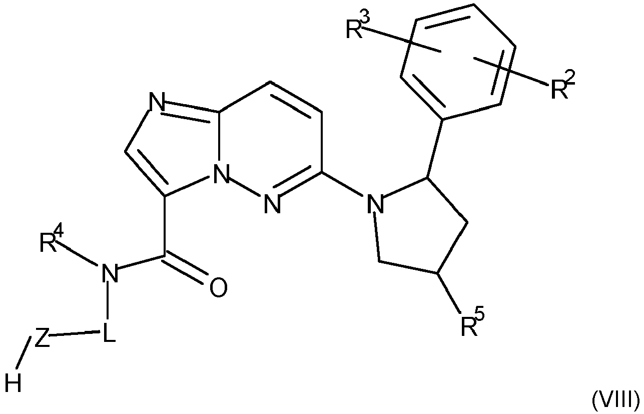
with an aldehyde of Formula (IX)

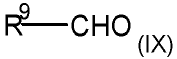

in the presence of a suitable reducing agent in a suitable solvent such as DCM at an appropriate temperature such as room temperature, wherein R¹, R², R³, R⁴, R⁵ and R⁹, L and Z are as defined anywhere hereinabove in respect of a compound of Formula I.

Also provided herein is a process of preparing a compound of the present invention or a pharmaceutically acceptable salt or solvate thereof, wherein Z is present and X is -C(O)-, comprising the step of:
amide bond formation via acid-amine coupling of the amine of Formula (VIII) 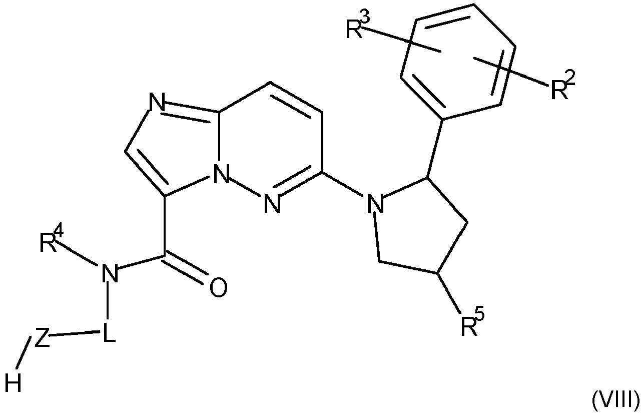
and the acid of Formula (X)

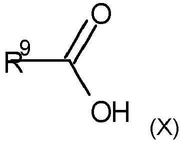

in the presence of a suitable coupling agent and organic base in a suitable polar aprotic solvent., wherein R¹, R², R³, R⁴, R⁵ and R⁹, L and Z are as defined anywhere hereinabove in respect of a compound of Formula I.

Also provided herein is a process of preparing a compound of the present invention or a pharmaceutically acceptable salt or solvate thereof, wherein Z is present and X is X is - S(O)₂-, comprising the step of:
sulphonamide bond formation of the amine of Formula (VIII) 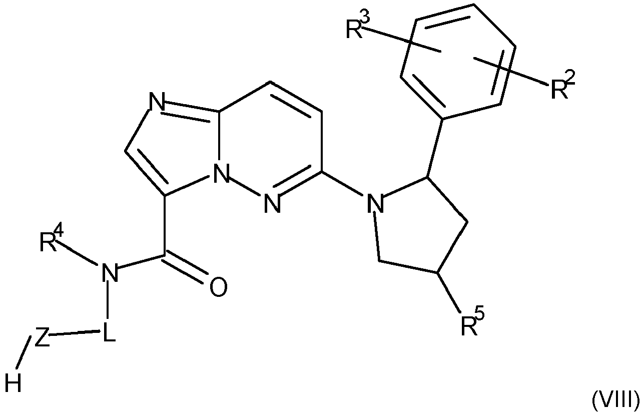
and the sulfonyl chloride of Formula (XI)

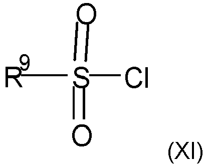

in the presence of a suitable coupling agent and organic base in a suitable polar aprotic solvent., wherein R¹, R², R³, R⁴, R⁵ and R⁹, L and Z are as defined anywhere hereinabove in respect of a compound of Formula I.

Also included herein are any variant of the present processes, in which an intermediate product obtainable at any stage thereof is used as starting material and the remaining steps are carried out, or in which the starting materials are formed *in situ* under the reaction conditions, or in which the reaction components are used in the form of their salts or optically pure antipodes.

Compounds of the invention and intermediates can also be converted into each other according to methods generally known to those skilled in the art.

Also provided herein are novel intermediate compounds.

The compounds of the invention exhibit valuable pharmacological properties, e.g. Trk modulating properties, e.g. as indicated in in vitro and in vivo tests as provided in the next sections and are therefore indicated for therapy.

Having regard to their ability to inhibit Trk activity, the compounds of the invention, hereinafter alternately referred to as "agents of the invention", are useful in the treatment or prevention of a condition or disorder which is mediated by Trk.

In particular, the compounds of the present invention are useful for the treatment of disorders or conditions mediated by the high affinity neurotrophin receptors TrkA, TrkB and TrkC, and the actions of their cognate neurotrophin ligands - NGF, BDNF/NT-4/5, NT-3 - on these receptor tyrosine kinases. Particularly the compounds are useful for treating or preventing conditions of skin (dermal) inflammation and itch (pruritus) that are mediated by the high affinity neurotrophin receptors TrkA, TrkB and TrkC, and associated with inflammation and nerve hypersensitivity, in particular atopic dermatitis.

Infiltration and activation of immune cells in the skin (including T-cell, mast cells, eosinophils) play a key role in inflammatory skin pathologies (Ilkovitch D. J Leukoc Biol. 2011, 89(1):41-9; Kim et al, Int J Mol Sci. 2016,17(8)). Trk A, B, and C and their cognate endogenous neurotrophin ligands have been demonstrated to play a role in the immunological and neurogenic mechanisms associated with skin pathologies (Botchkarev et al, J Invest Dermatol. 2006, 126(8):1719-27.; Truzzi et al, Dermatoendocrinol. 2011, 3(1):32-6; Minnone et al, Int J Mol Sci. 2017, 11;18(5)), and mediate inflammatory functions of skin resident immune cells, particularly those involved in atopic dermatitis pathology (Raap et al, Clin Immunol. 2005, (5):419-24), including T-cells (Sekimoto et al, Immunol Lett. 2003, 88(3):221-6; Matsumura et al, J Dermatol Sci. 2015,78(3):215-23), mast cells (Quarcoo et al, J Occup Med Toxicol. 2009, Apr 22;4:8.), and eosinophils (Raap et al, J Allergy Clin Immunol. 2005, 115:1268-75; Raap et al, Clin Exp Allergy.2008, 38(9):1493-8).

NGF, BDNF, NT-3 and NT-4/5 levels are higher in the lesional skin cells and plasma of atopic dermatitis patients compared to normal subjects and levels correlate with disease severity (Yamaguchi et al, J Dermatol Sci. 2009, 53(1):48-54; Toyoda et al, Br J Dermatol 2002, 147:71-79; Raap et al, J Allergy Clin Immunol. 2005, 115:1268-75; Raap et al, Allergy. 2006, 61(12):1416-8). Trk levels are also upregulated in atopic dermatitis lesional skin cells (Dou et al, Arch Dermatol Res. 2006, (1):31-7; Raap et al, Clin Exp Allergy. 2008, 38(9):1493-8). In addition, the high affinity neurotrophin receptors and their endogenous ligands, in particular Trk A/NGF have been shown to sensitize primary afferent nerves and mediate dermal hyperinnervation, thereby contributing to peripheral itch sensitization and pruritus in particular in atopic dermatitis (Tominaga et al, J Dermatol. 2014, 41(3):205-12; Roggenkamp D et al, J Invest Dermatol 2012, 132: 1892-1900; Grewe et al, J Invest Dermatol 2000, 114:1108-1112). In preclinical mouse models of atopic dermatitis, inhibition of Trk signalling with small molecule compounds that have Trk inhibitory activity, reduced dermatitis and scratching behaviour, with concomitant decreases in nerve fibres in the epidermis (Takano et al, Br J Dermatol. 2007, 156(2):241-6; Narayanan et al, PLoS One. 2013, 26;8(12)).

The compounds of the present invention may be used for the treatment or prevention of skin pathologies or conditions including diseases of dermatitis such as atopic dermatitis (eczema), contact dermatitis, allergic dermatitis; diseases of pruritus such as urticaria (Rössing et al, Clin Exp Allergy. 2011, 41(10):1392-9), Cutaneous T-cell lymphoma (CTCL) -associated pruritus including Sezary syndrome (Suga et al, Acta Derm Venereol. 2013, 93(2):144-9; Saulite et al, Biomed Res Int. 2016 doi: 10.1155/2016/9717530); Psoriasis (Raychaudhuri et al, Prog Brain Res. 2004, 146:433-7); diseases of skin pain and neuropathy (Hirose et al, Pain Pract. 2016, 16(2):175-82; Wang et al, J Neurosci. 2009, 29(17):5508-15).

In particular, conditions or disorders which are mediated by Trk, in particular Trk A, B, and C, include, but are not limited to: diseases of pruritus and itch; autoimmune diseases of the skin; diseases of skin pain and neuropathy; and diseases of dermatitis.

Diseases of pruritus and itch include, but are not limited to: skin diseases, eczematous ; dermatitis, atopic; eczema ;dermatitis, contact; dermatitis, allergic contact; dermatitis, irritant ; dermatitis, photoallergic ; dermatitis, phototoxic ; psoriasis ; pruritus; pruritus ani; pruritus, hereditary localized; Sjogrens syndrome associated pruritis; idiopathic pruritus; sclerosis multiplex pruritus; prurigo nodularis; brachioradial pruritus; acute itch; chronic itch; diabetes pruritus; iron deficiency anaemia pruritus; polycythemia vera pruritus; graft-versus-host-disease ; uraemic pruritus; cholestatic pruritus; pruritic urticarial papules and plaques of pregnancy; pemphigoid gestationis; senile pruritus; HIV associated pruritus; shingles; herpes zoster oticus; larva migrans; tinea corporis; tungiasis; exanthema; Fox-Fordyce disease; skin diseases, parasitic; skin diseases, bacterial; cutaneous T-cell; lymphoma-associated pruritus; Sezary syndrome; mycosis fungoides; colorectal cancer; melanoma; head and neck cancer; drug eruption pruritus (iatrogenic); drug reactions; urticarial; vibratory urticarial; physical urticarial; familial cold urticarial; allergic urticarial; dermatographia; dermatitis herpetiformis; Grover disease.

Autoimmune diseases of the skin include, but are not limited to: autoimmune disease of skin and connective tissue; autoimmune disease with skin involvement; autoimmune bullous skin disease; pemphigoid, bullous.

Diseases of skin pain and neuropathy include but are not limited to: diabetic neuropathies ; neuralgia; painful neuropathy ; nerve compression syndromes ; neuritis; sensory peripheral neuropathy ; alcoholic neuropathy ; radiculopathy ; complex regional pain syndromes ; polyneuropathy due to drug; plantar nerve lesion; polyradiculopathy; sciatic neuropathy;trigeminal neuralgia.

Diseases of dermatitis include, but are not limited to: skin diseases, eczematous ; dermatitis, atopic ; eczema ; dermatitis, contact; dermatitis, allergic contact; dermatitis, irritant ; dermatitis, photoallergic ; dermatitis, phototoxic; chronic irritative hand dermatitis; dermatitis, occupational; fiberglass dermatitis ; dermatitis, toxicodendron ; eczema, dyshidrotic; eczematous dermatitis of eyelid; allergic contact dermatitis of eyelid; hand and foot dermatitis; digital dermatitis; dermatitis, exfoliative; radiodermatitis; dermatitis herpetiformis ; juvenile dermatitis herpetiformis ; autoimmune progesterone dermatitis ; dermatitis, seborrheic; pityriasis lichenoides; blepharitis; nummular dermatitis ; Seborrhea-Like Dermatitis with Psoriasiform Elements; infective dermatitis associated with HTLV-1; psoriasis; generalized pustular psoriasis; skin diseases, papulosquamous; parapsoriasis; keratosis; hyperkeratosis, epidermolytic; skin sarcoidosis; skin atrophy; erythematosquamous dermatosis; poikiloderma with neutropenia; erythema multiforme; angiolymphoid hyperplasia with eosinophilia; keratosis palmoplantaris striata 3; acne vulgaris; lamellar ichthyosis; lichen disease; lichen planus; actinic lichen planus; lichen planus, oral ; lichen planus follicularis ; lichen sclerosus et atrophicus ; lichen nitidus; lichen sclerosus; lichen simplex chronicus; scleroderma, limited; keratosis linearis with ichthyosis congenita and sclerosing keratoderma; erythrokeratoderma, reticular; keratosis palmoplantaris papulose; skin diseases, genetic; autosomal recessive congenital ichthyosis; autosomal recessive congenital ichthyosis 1; autosomal recessive congenital ichthyosis 2; autosomal recessive congenital ichthyosis 3 ;autosomal recessive congenital ichthyosis 4A; autosomal recessive congenital ichthyosis 5; autosomal recessive congenital ichthyosis 6; autosomal recessive congenital ichthyosis 7; autosomal recessive congenital ichthyosis 8 autosomal recessive congenital ichthyosis 9; autosomal recessive congenital ichthyosis 10; autosomal recessive congenital ichthyosis 11.

More particularly, the condition or disorder which is mediated by Trk, in particular Trk A, B, and C, may be atopic dermatitis.

Treatment in accordance with the invention may be symptomatic or prophylactic,

Thus in a further aspect the invention includes an agent of the invention for use as a pharmaceutical.

Therefore according to a further aspect, the invention provides an agent of the invention for treating or preventing a condition or disorder which is mediated by Trk, in particular Trk A, B, and C.

In another aspect the invention provides an agent of the invention for preventing or treating a condition or disorder which is mediated by Trk, in particular Trk A, B, and C, more particularly atopic dermatitis.

As referred to herein a "disorder" or a "disease" refers to an underlying pathological disturbance in a symptomatic or asymptomatic organism relative to a normal organism, which may result, for example, from infection or an acquired or congenital genetic imperfection.

A "condition" refers to a state of the mind or body of an organism which has not occurred through disease, e.g. the presence of a moiety in the body such as a toxin, drug or pollutant.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

"Prevention" of a condition or disorder refers to delaying or preventing the onset of a condition or disorder or reducing its severity, as assessed by the appearance or extent of one or more symptoms of said condition or disorder.

As used herein, the term "subject" refers to an animal. Typically the animal is a mammal. A subject also refers to for example, primates (e.g., humans), cows, sheep, goats, horses, dogs, cats, rabbits, rats, mice, fish, birds and the like. In certain embodiments, the subject is a primate. In yet other embodiments, the subject is a human.

As used herein, a subject is "in need of" a treatment if such subject would benefit biologically, medically or in quality of life from such treatment.

The term "a therapeutically effective amount" of an agent of the invention refers to an amount of the agent of the invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the agent of the invention that, when administered to a subject, is effective to at least partially alleviating, inhibiting, preventing and/or ameliorating a condition or disorder which is mediated by TrK, in particular Trk A, B, and C. In another non-limiting embodiment, the term "a therapeutically effective amount" refers to the amount of the agent of the invention that, when administered to a cell, or a tissue, or a non-cellular biological material, or a medium, is effective to at least partially inhibiting Trk activity, in particular Trk A, B, and C.

In one embodiment of the present invention, the condition or disorder which is mediated by Trk, in particular Trk A, B, and C, is selected from diseases of pruritus and itch; autoimmune diseases of the skin; diseases of skin pain and neuropathy; and diseases of dermatitis.

In a particular embodiment, the condition or disorder which is mediated by Trk, in particular Trk A, B, and C, is atopic dermatitis.

As described above, the agents of the invention, which inhibit Trk, in particular Trk A, B, and C, have various clinical applications and thus a further aspect of the invention provides pharmaceutical compositions containing agents of the invention. The use of these agents as a medicament forms a further aspect of the invention.

The active agents of the invention are used, alone or in combination with one or more additional active ingredients, to formulate pharmaceutical compositions of the invention. A pharmaceutical composition of the invention comprises: (a) an effective amount of at least one active agent in accordance with the invention; and (b) a pharmaceutically acceptable excipient.

Thus, in a further aspect the present invention provides a pharmaceutical composition comprising an agent of the invention and a pharmaceutically acceptable excipient.

Pharmaceutical compositions as described herein for use as a medicament, in particular for use in treating or preventing disorders or conditions medited by Trk, in particular Trk A, B, and C, such as the conditions described herein, form further aspects of the invention.

"Pharmaceutically acceptable" as referred to herein refers to ingredients that are compatible with other ingredients of the compositions as well as physiologically acceptable to the recipient.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith. Examples of excipients include calcium carbonate, calcium phosphate, various sugars and types of starch, cellulose derivatives, gelatin, vegetable oils, and polyethylene glycols.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

Pharmaceutical compositions according to the invention may be formulated in conventional manner using readily available ingredients. Thus, the active ingredient may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

The pharmaceutical composition can be formulated for particular routes of administration such as oral administration, parenteral administration, and rectal administration, etc. In addition, the pharmaceutical compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with
a) diluents, e.g., lactose, polylactone, dextrose, sucrose, mannitol, sorbitol, cellulose and/or glycine;
b) lubricants, e.g., silica, talcum, stearic acid, its magnesium or calcium salt and/or polyethylene glycol; for tablets also
c) binders, e.g., magnesium aluminum silicate, starch paste, gelatin, tragacanth, methylcellulose, sodium carboxymethylcellulose and/or polyvinylpyrrolidone; if desired
d) disintegrants, e.g., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of an agent of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for topical application to the skin or mucosa (e.g., to the skin and eyes), that is dermally or transdermally, include aqueous solutions, suspensions, ointments, creams, gels, hydrogels, microemulsions, dusting powders, dressings, foams, films, skin patches, wafers, implants, fibres, bandages or sprayable formulations, e.g., for delivery by aerosol or the like. Such topical delivery systems will in particular be appropriate for dermal application, e.g., for the treatment of atopic dermatitis. They are thus particularly suited for use in topical, including cosmetic, formulations well-known in the art. Such may contain solubilizers, stabilizers, tonicity enhancing agents, buffers and preservatives. Typical carriers include alcohol, water, mineral oil, liquid petrolatum, white petrolatum, glycerin, polyethylene glycol and propylene glycol. Penetration enhancers may be incorporated [see, for example, Finnin and Morgan, J Pharm Sci, 88 (10), 955-958 (October 1999).]

Suitable compositions for transdermal application include an effective amount of an agent of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

Dosages of agents of the invention employed in practising the present invention will of course vary depending, for example, on the particular condition to be treated, the effect desired and the mode of administration. In general, suitable daily dosages for administration by inhalation are of the order of 0.0001 to 30 mg/kg, typically 0.01 to 10 mg per patient, while for oral administration suitable daily doses are of the order of 0.01 to 100 mg/kg.

The present invention further provides anhydrous pharmaceutical compositions and dosage forms comprising the agents of the invention as active ingredients, since water may facilitate the degradation of certain compounds.

Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

The agent of the invention may be administered either simultaneously with, or before or after, one or more other therapeutic agent. The agent of the invention may be administered separately, by the same or different route of administration, or together in the same pharmaceutical composition as the other agents.

In one embodiment, the invention provides a product comprising an agent of the invention and at least one other therapeutic agent as a combined preparation for simultaneous, separate or sequential use in therapy. In one embodiment, the therapy is the treatment of a condition or disorder which is mediated by Trk, in particular Trk A, B, and C. Products provided as a combined preparation include a composition comprising the agent of the invention and the other therapeutic agent(s) together in the same pharmaceutical composition, or the agent of the invention and the other therapeutic agent(s) in separate form, e.g. in the form of a kit.

In one embodiment, the invention provides a pharmaceutical composition comprising an agent of the invention and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains an agent of the invention. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and topical, for administering the separate compositions at different dosage intervals, or for titrating the separate compositions against one another. To assist compliance, the kit of the invention typically comprises directions for administration.

In the combination therapies of the invention, the agent of the invention and the other therapeutic agent may be manufactured and/or formulated by the same or different manufacturers. Moreover, the agent of the invention and the other therapeutic may be brought together into a combination therapy: (i) prior to release of the combination product to physicians (e.g. in the case of a kit comprising the agent of the invention and the other therapeutic agent); (ii) by the physician themselves (or under the guidance of the physician) shortly before administration; (iii) in the patient themselves, e.g. during sequential administration of the agent of the invention and the other therapeutic agent.

Accordingly, the invention provides the use of an agent of the invention for treating a condition or disorder which is mediated by Trk, in particular Trk A, B, and C, wherein the medicament is prepared for administration with another therapeutic agent. The invention also provides the use of another therapeutic agent for treating a condition or disorder which is mediated by Trk, in particular Trk A, B, and C, wherein the medicament is administered with an agent of the invention.

The combination may serve to increase efficacy (e.g., by including in the combination a compound potentiating the potency or effectiveness of an active agent according to the invention), decrease one or more side effects, or decrease the required dose of the active agent according to the invention.

The invention also provides an agent of the invention for use in a method of treating a condition or disorder which is mediated by Trk, in particular Trk A, B, and C, wherein the agent of the invention is prepared for administration with another therapeutic agent. The invention also provides another therapeutic agent for use in a method of treating a condition or disorder which is mediated by Trk, in particular Trk A, B, and C, wherein the other therapeutic agent is prepared for administration with an agent of the invention. The invention also provides an agent of the invention for use in a method of treating a condition or disorder which is mediated by Trk, in particular Trk A, B, and C, wherein agent of the invention is administered with another therapeutic agent. The invention also provides another therapeutic agent for use in a method of treating a condition or disorder which is mediated by Trk, in particular Trk A, B, and C, wherein the other therapeutic agent is administered with an agent of the invention.

The invention also provides the use of an agent of the invention for treating a condition or disorder which is mediated by Trk, in particular Trk A, B, and C, wherein the subject has previously (e.g. within 24 hours) been treated with another therapeutic agent. The invention also provides the use of another therapeutic agent for treating a condition or disorder which is mediated by Trk, in particular Trk A, B, and C, wherein the subject has previously (e.g. within 24 hours) been treated with an agent of the invention.

In one embodiment, a compound of the invention is administered alongside one or more other therapeutically active agents. For example, the compounds of the invention may therefore be used in combination with one or more further agents for the treatment of atopic dermatitis, such as: one or more topical and/or oral corticosteroids; one or more antihistamines; one or more antibiotics; one or more topical calcineurin inhibitors such as tacrolimus and/or pimecrolimus; one or more systemic immunosuppressants such as cyclosporin, methotrexate, interferon gamma-1 b, mycophenolate mofetil and/or azathioprine; one or more PDE4 inhibitors such as crisaborole; one or more monoclonal antibodies such as dupilumab.

A skilled person will appreciate that an agent of the invention may be administered to a subject, particularly a human subject, wherein the subject is being treated with phototherapy for a condition or disorder which is mediated by Trk, in particular Trk A, B, and C, such as atopic dermatitis. A compound of the invention may also be administered to a subject, particularly a human subject, wherein the subject has previously (e.g. within 24 hours) been treated with phototherapy for a condition or disorder in which is mediated by Trk, in particular Trk A, B, and C, such as atopic dermatitis. A subject, particularly a human subject may also be treated with phototherapy for a condition or disorder which is mediated by Trk, in particular Trk A, B, and C, such as atopic dermatitis wherein a compound of the invention has previously (e.g. within 24 hours) been administered to a subject.

Accordingly, the invention includes as a further aspect a combination of an agent of the invention with one or more further agents for the treatment of atopic dermatitis, such as: one or more topical and/or oral corticosteroids; one or more antihistamines; one or more antibiotics; one or more topical calcineurin inhibitors such as tacrolimus and/or pimecrolimus; one or more systemic immunosuppressants such as cyclosporin, methotrexate, interferon gamma-1b, mycophenolate mofetil and/or azathioprine; one or more PDE4 inhibitors such as crisaborole; one or more monoclonal antibodies such as dupilumab; and phototherapy.

### In vitro assays

A suitable assay for determining the Trk inhibition activity of a compound is detailed herein below.

To determine the IC₅₀ of small molecule compounds for the Human TRK receptors, HTRF^{®} KinEASE^{™} kinase kits from Cisbio were used. Assays were carried out in low volume, black 384-well plates.

Recombinant Human TRK enzymes (Invitrogen) were incubated in the presence or absence of the compound (11-point dose response with FAC as 10µM) for 30 minutes at 23°C. Kinase reaction was started by addition of ATP to a mixture containing the enzyme (NTRK1-4nM, NTRK2-1nM, NTRK3-10nM) and substrate (1µM). Kinase reaction was allowed to carry on for 10 to 45 minutes at 23°C after which it was stopped by addition of the detection mix (supplied by vendor) containing EDTA, TK-Ab- labelled with Eu³⁺-cryptate (1:200 dilutions) and Streptavidin-XL665 (250nM). Assay plates were incubated in this detection mix for 60 minutes at 23°C. The resulting TR-FRET signal, calculated as the fluorescence ratio at 665/620 nm, was read on an Envision and was proportional to the level of phosphorylation of the peptide in the presence or absence of the compound. The uniformity of the plates were assured with Z' value [1-{3*(SDHPE+SDZPE)/ (ZPE-HPE)}]. The percent (%) effect i.e. Inhibition of compound was calculated in comparison to the signal in the positive (HPE) and negative control (ZPE) wells within each assay plate. The endpoint value % Inhibition for the Standard compound was evaluated in each experiment as a quality control measure. IC₅₀ was determined by plotting compound inhibition at respective dose in Graphpad prism5 using four parameter logistic curve fit.

Using the assay described above, the compounds of the present invention all exhibit of Trk inhibition activity, expressed as an IC₅₀ value, of less than 1 µM). Preferred examples have IC₅₀ values of less than 200nM and particularly preferred examples have IC₅₀ values of less than 50nM. IC₅₀ values for the compounds of Examples 1 to 31 are given below in Table 1.

| **Example Number** | **TrkA Enz (nM)** | **TrkB Enz (nM)** | **TrkC Enz (nM)** |
|---|---|---|---|
| 1 | 1.77 | 2.06 | 2.81 |
| 2 | 1.78 | 3.33 | 3.36 |
| 3 | 0.92 | 0.79 | 1.19 |
| 4 | 1.75 | 1.05 | 1.93 |
| 5 | 1.90 | 1.19 | 2.11 |
| 6 | 1.47 | 0.81 | 1.56 |
| 7 | 0.90 | 0.49 | 1.25 |
| 8 | 0.79 | 1.51 | 1.64 |
| 9 | 3.81 | 8.76 | 8.43 |
| 10 | 1.18 | 2.15 | 2.12 |
| 11 | 1.57 | 2.27 | 2.46 |
| 12 | 3.82 | 10.3 | 10.9 |
| 13 | 1.70 | 2.53 | 2.01 |
| 14 | 0.95 | 0.57 | 1.47 |
| 15 | 4.86 | 9.15 | 8.30 |
| 16 | 1.73 | 4.54 | 3.28 |
| 17 | 2.41 | 5.04 | 4.62 |
| 18 | 2.87 | 7.82 | 5.19 |
| 19 | 2.46 | 3.04 | 2.99 |
| 20 | 2.69 | 3.76 | 3.72 |
| 21 | 1.48 | 1.50 | 1.97 |
| 22 | 1.40 | 2.01 | 2.13 |
| 23 | 2.37 | 10.3 | 6.85 |
| 24 | 6.90 | 23.6 | 17.3 |
| 25 | 6.10 | 11.4 | 8.72 |
| 26 | 1.87 | 1.85 | 2.97 |
| 27 | 1.34 | 1.01 | 1.87 |
| 28 | 4.63 | 5.67 | 4.88 |
| 29 | 5.84 | 5.34 | 3.98 |
| 30 | 22.4 | 14.9 | 22.4 |
| 31 | 1.38 | 0.86 | 1.38 |

### EXAMPLES

Referring to the examples that follow, compounds of the preferred embodiments are synthesized using the methods described herein, or other methods, which are known in the art.

It should be understood that the organic compounds according to the preferred embodiments may exhibit the phenomenon of tautomerism. As the chemical structures within this specification can only represent one of the possible tautomeric forms, it should be understood that the preferred embodiments encompasses any tautomeric form of the drawn structure.

It is understood that the invention is not limited to the embodiments set forth herein for illustration, but embraces all such forms thereof as come within the scope of the above disclosure.

### General Conditions:

The following examples are intended to illustrate the invention and are not to be construed as being limitations thereon. Temperatures are given in degrees centigrade. If not mentioned otherwise, all evaporations are performed under reduced pressure. The structure of final products, intermediates and starting materials is confirmed by standard analytical methods, *e.g.,* microanalysis and spectroscopic characteristics, *e.g.,* MS, IR, NMR. Abbreviations used are those conventional in the art. If not defined, the terms have their generally accepted meanings.

### Abbreviations and acronyms used herein include the following:

| **Abbreviation/acronym** | **Term** |
|---|---|
| AcOH | Acetic acid |
| AgOAc | Silver acetate |
| aq | aqueous |
| Bn | benzyl |
| br | broad |
| °C | degrees Celsius |
| CDCl₃ | deutero-chloroform |
| Cs₂CO₃ | Cesium carbonate |
| Cy | cyclohexane |
| δ | chemical shift |
| d | doublet |
| dd | double doublet |
| ddd | Doublet of doublets of doublets |
| DCM | dichloromethane |
| DIPEA | N-ethyldiisopropylamine or N,N-diisopropylethylamine |
| DMAP | 4-(dimethylamino)pyridine |
| DMF | N,N-dimethylformamide |
| DMSO | Dimethylsulfoxide |
| DMSO-d₆ | hexadeuterodimethyl sulfoxide |
| Et | ethyl |
| Et₃N | triethylamine |
| EtOH | ethanol |
| EtOAc | ethyl acetate |
| g | gram |
| HCl | hydrochloric acid |
| HATU | (1-[Bis(dimethylamino)methylene]-1H-1,2,3-triazolo[4,5-b]pyridinium 3-oxid hexafluorophosphate) |
| H₂O | water |
| HPLC | high pressure liquid chromatography |
| Hr | hour |
| IPA | Isopropyl alcohol |
| KF | Potassium fluoride |
| KOH | Potassium hydroxide |
| L | litre |
| Lawesson's Reagent | 2,4-Bis(4-methoxyphenyl)-2,4-dithioxo-1,3,2,4-dithiadiphosphetane |
| LCMS | liquid chromatography mass spectrometry |
| m | multiplet |
| M | molar |
| mBar | millibar |
| Me | methyl |
| MeCN | acetonitrile |
| MeOH | methanol |
| MeOD-d₄ | deutero-methanol |
| 2-MeTHF | 2-methyltetrahydrofuran |
| mg | milligram |
| MHz | mega Hertz |
| mins | minutes |
| mL | millilitres |
| mmol | millimole |
| MS m/z | mass spectrum peak |
| MsCl | methanesulfonyl chloride |
| MTBE | Methyl *tert*-butyl ether |
| M/V | Mass volume ratio |
| N₂ | nitrogen |
| NaBH₄ | sodium borohydride |
| NaHCO₃ | sodium bicarbonate |
| NaOH | sodium hydroxide |
| NH₃ | ammonia |
| NH₄Cl | ammonium chloride |
| Na₂SO₄ | sodium sulfate |
| PtO₂ | platinum (IV) oxide |
| q | quartet |
| rt | room temperature |
| RT | retention time |
| s | singlet |
| sat. | saturated |
| soln. | solution |
| t | triplet |
| TBDMS | tert-butyldimethylsilyl |
| TBDMSCI | tert-butyldimethylsilyl chloride |
| TEAF | tetraethylammonium fluoride |
| THF | tetrahydrofuran |
| TMS | trimethylsilyl |
| µL | micro litres |
| v/v | volume volume percent |
| w/w | Weight/weight percent |

Referring to the examples that follow, compounds of the preferred embodiments were synthesized using the methods described herein, or other methods, which are known in the art.

The various starting materials, intermediates, and compounds of the preferred embodiments may be isolated and purified, where appropriate, using conventional techniques such as precipitation, filtration, crystallization, evaporation, distillation, and chromatography. Unless otherwise stated, all starting materials are obtained from commercial suppliers and used without further purification. Salts may be prepared from compounds by known salt-forming procedures.

It should be understood that the organic compounds according to the preferred embodiments may exhibit the phenomenon of tautomerism. As the chemical structures within this specification can only represent one of the possible tautomeric forms, it should be understood that the preferred embodiments encompasses any tautomeric form of the drawn structure.

¹H nuclear magnetic resonance (NMR) spectra were in all cases consistent with the proposed structures. Characteristic chemical shifts (δ) are given in parts-per-million downfield from tetramethylsilane (for ¹H-NMR) using conventional abbreviations for designation of major peaks: e.g. s, singlet; d, doublet; t, triplet; q, quartet; m, multiplet; br, broad. The following abbreviations have been used for common solvents: CDCl₃, deuterochloroform; DMSO-d₆, hexadeuterodimethyl sulfoxide; and MeOD-d₄, deuteron-methanol. Where appropriate, tautomers may be recorded within the NMR data; and some exchangeable protons may not be visible.

Mass spectra, MS (m/z), were recorded using either electrospray ionisation (ESI) or atmospheric pressure chemical ionisation (APCI). Where relevant and unless otherwise stated the m/z data provided are for isotopes ¹⁹F, ³⁵Cl, ⁷⁹Br and ¹²⁷I.

Where preparative TLC or silica gel chromatography have been used, one skilled in the art may choose any combination of solvents to purify the desired compound.

Example compounds of the present invention include:

### Example 1

### 6-f (2 R)-2-f 5-fluoro-2-(methylsulfanyl)phenyllpyrrolidin-1-yll-N-f 1-f (4-fluoro-3-hydroxyphenyl)methyl]azetidin-3-yl}imidazo[1,2-b]pyridazine-3-carboxamide

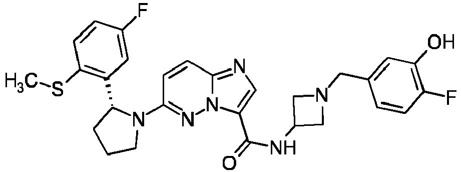

A suspension of N-(azetidin-3-yl)-6-[(2R)-2-[5-fluoro-2-(methylsulfanyl)phenyl]pyrrolidin-1-yl]imidazo[1,2-b]pyridazine-3-carboxamide (Preparation 17, 100 mg, 0.23 mmol), 4-fluoro-3-hydroxybenzaldehyde (36 mg, 0.26 mmol) and sodium triacetoxyborohydride (36 mg, 0.32 mmol) in DCM (4 ml) was stirred at rt overnight. The reaction was quenched using sat NaHCO₃ and extracted with DCM, the combined organic layers were dried (Na₂SO₄) and concentrated *in vacuo.* The crude material was purified by reverse phase chromatography 5-50% MeCN/H2O (0.1% NH3 modifier), followed by normal phase chromatography 0-10% DCM/MeOH. The solid was triturated 9:1 Et2O/EtOAc to afford the title compound as a colourless solid (22 mg, 17%).
LCMS *m*/*z* = 551.2 [M+H]⁺; 549.2 [M-H]⁺
¹HNMR (CDCl₃, 400 MHz): δ: 9.10 (br s, 1H), 8.19 (s, 1H), 7.70 (d, 1H), 7.29 (m, 1H), 7.0 (m, 3H), 6.76 (m, 2H), 6.42 (br s, 1H), 5.24 (d, 1H), 4.57 (br m, 2H), 3.84 (m, 8H), 2.62 (s, 3H), 2.53 (m, 1H), 2.06 ppm (m, 3H).

### Example 2

### 6-f (2 R)-2-f 5-fluoro-2-(methylsulfanyl)phenyllpyrrolidin-1-yll-N-f (3S)-1-f (4-fluoro-3-hydroxyphenyl)methyllpyrrolidin-3-yllimidazof 1,2-blpyridazine-3-carboxamide

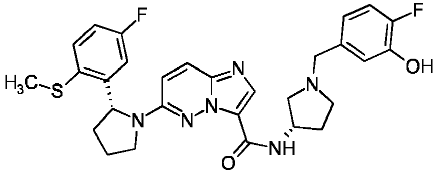

The following compound was prepared (30 mg, 23%) from 6-[(2R)-2-[5-fluoro-2-(methylsulfanyl)phenyl]pyrrolidin-1-yl]-N-[(3S)-pyrrolidin-3-yl]imidazo[1,2-b]pyridazine-3-carboxamide (Preparation 18) and 4-fluoro-3-hydroxybenzaldehyde according to the procedure described in Example 1.
LCMS *m*/*z* = 565.4 [M+H]⁺
¹H NMR (DMSO-d₆, 400 MHz): δ: 9.70 (s, 1H), 9.14 (br s, 1H), 7.92 (s, 1H), 7.87 (d, 1H), 7.39-7.38 (m, 1H), 7.14 (m, 1H), 7.00 (m, 1H), 6.95-6.84 (m, 1H), 6.73-6.65 (m, 1H), 6.35-6.08 (m, 1H), 5.20 (m 1H), 4.54-4.33 (m, 1H), 4.02-3.86 (m, 1H), 3.66-3.50 (m, 1H), 3.47-3.34 (m, 2H), 2.84-2.43 (m, 7H), 2.36-2.14 (m, 2H), 1.94-1.74 (m, 3H), 1.72-1.52 (m, 1H).

### Example 3

### 6-[(2R,4S)-4-fluoro-2-[5-fluoro-2-(methylsulfanyl)phenyl]pyrrolidin-1-yl]-N-{1-[(3-hydroxyphenyl)methyl]piperidin-4-yl}imidazo[1,2-b]pyridazine-3-carboxamide

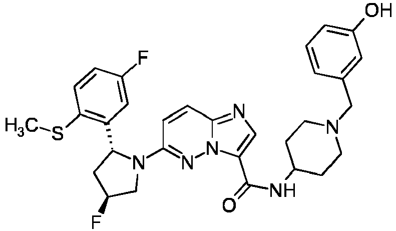

3-((4-Aminopiperidin-1-yl)methyl)phenol hydrochloride (Preparation 50, 2.86 g, 10.25 mmol) was added to a stirred solution of 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 28, 2.00 g, 5.12 mmol), TPTU (1.83 g, 6.15 mmol), and DIPEA (4.46 ml, 25.61 mmol) in dry DCM (36 ml) at rt under N₂. The solution was allowed to stir for 5 h, then diluted with DCM (50 ml) and the resulting solution was washed with distilled water (40 ml), saturated aqueous NaHCO₃ (3 × 40 ml), saturated aqueous NH₄Cl (3 × 40 ml) and distilled water (40 ml). The organic layer was dried (MgSO₄), filtered and concentrated *in vacuo* to afford an orange oil (3.5 g) which was loaded on a 120 g KP-Sil column and purified by Biotage^{®} with 1 CV EtOAc and then 0-15 % MeOH in EtOAc over 12 CVs. The relevant fractions were combined and then concentrated *in vacuo* to afford 1.95 of a white solid (1.95 g). The solid was kept under house vacuum at 35°C over for 65 hours to afford the title compound as white solid (1.70 g, 57%).
LCMS *m*/*z* = 579 [M+H]⁺
¹H NMR (MeOD-d₄; 396 MHz,): δ: 8.05 (s, 1H), 7.74 (m, 1H), 7.42 (m, 1H), 7.14-7.18 (m, 1H), 7.00-7.09 (m, 2H), 6.81-6.83 (m, 2H), 6.70-6.73 (m, 1H), 6.56 (m, 1H), 5.43-5.60 (m, 2H), 4.16-4.28 (m, 2H), 3.94-4.00 (m, 1H), 3.52 (s, 2H), 2.97-3.06 (m, 3H), 2.57 (s, 3H), 2.19-2.31 (m, 3H), 2.07 (s, 2H), 1.67-1.80 (m, 2H).

The following compounds were prepared from 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 28) and the appropriate amine according to the procedure described in Example 3.

| Example No | Structure and Name | Starting material, Yield and Data |
|---|---|---|
| 4 | 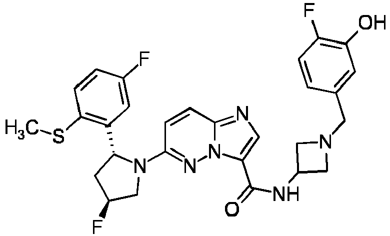 6-[(2R,4S)-4-fluoro-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide | Amine: Preparation 51 15 mg, 26%, colourless solid. |
| | | LCMS *m*/*z* = 569.1 [M+H]⁺ |
| | | ¹H NMR (MeOD-d₄, 396 MHz): δ: 8.06 (br s, 1H), 7.76 (d, 1H), 7.42 (m, 1H), 7.10-7.00 (m, 3H), 6.96-6.92 (m, 1H), 6.79-6.76 (m, 1H), 6.61 (d, 1H), 5.62-5.44 (m, 2H), 4.64-4.58 (m, 1H), 4.30-4.19 (m, 2H), 3.78-3.63 (m, 4H), 3.41-3.34 (m, 2H), 3.07-2.97 (m, 1H), 2.57 (s, 3H), 2.32-2.16 (m, 1H). |
| 5 | 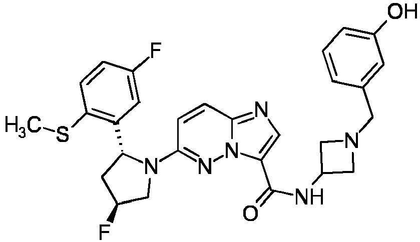 6-[(2R,4S)-4-fluoro-2-[5-fluoro-2-(methylsulfanyl)phenyl]pyrrolidin-1-yl]-N-{1-[(3-hydroxyphenyl)methyl]azetidin-3-yl}imidazo[1,2-b]pyridazine-3-carboxamide | Amine: Preparation 52 21.5 mg, 30%, colourless oil. |
| | | LCMS *m*/*z* = 551.1 [M+H]⁺ |
| | | ¹H NMR (MeOD-d₄, 396 MHz): δ: 8.03 (s, 1H), 7.74 (m, 1H), 7.39 (m, 1H), 7.14 (m, 1H), 7.03 (m, 2H), 6.79-6.76 (m, 2H), 6.71-6.68 (m, 1H), 6.59 (m, 1H), 5.59-5.41 (m, 2H), 4.62-4.56 (m, 1H), 4.27-4.16 (m, 2H), 3.77-3.65 (m, 4H), 3.40-3.34 (m, 2H), 3.04-2.94 (m, 1H), 2.54 (s, 3H), 2.29-2.12 (m, 1H). |
| 6 | 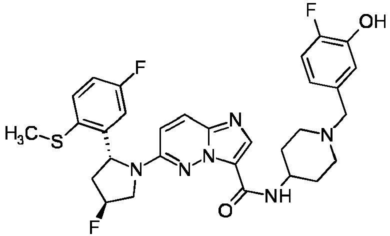 6-[(2R,4S)-4-fluoro-2-[5-fluoro-2-(methylsulfanyl)phenyl]pyrrolidin-1-yl]-N-{1-(4-fluoro-3-hydroxyphenyl)methyl]piperidin-4-yl}imidazo[1,2-b]pyridazine-3-carboxamide | Amine: Preparation 53 |
| | | 20 m, 26%, colourless solid. |
| | | LCMS *m*/*z* = 597.2 [M+H]⁺ |
| | | ¹H NMR (MeOD-d₄, 396 MHz): δ: 8.03 (s, 1H), 7.72 (m, 1H), 7.40 (m, 1H), 7.07-6.96 (m, 4H), 6.81 (m, 1H), 6.54 (m, 1H), 5.58-5.40 (m, 2H), 4.27-4.10 (m, 2H), 4.02-3.95 (m, 1H), 3.64 (s, 2H), 3.10-2.93 (m, 3H), 2.56 (s, 3H), 2.43-2.37 (m, 2H), 2.30-2.11 (m, 3H), 1.84-1.75 (m, 2H). |

### Example 7

### 6-[(2R,4S)-4-fluoro-2-[5-fluoro-2-(methylsulfanyl)phenyl]pyrrolidin-1-yl]-N-[(3-hydroxyphenyl)methyllimidazof 1,2-blpyridazine-3-carboxamide

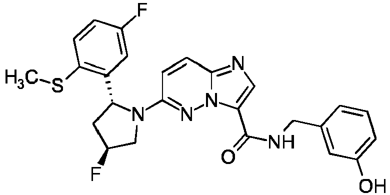

To a solution of 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 28, 40 mg, 0.102 mmol) in dry DCM (0.73 ml) at rt were added TPTU (365 mg, 0.122 mmol), 3-hydroxybenzylamine (138 mg, 0.122 mmol) and DIPEA (0.035 ml, 0.204 mmol) and the reaction mixture stirred at rt for 2 h. The reaction was diluted with DCM (15ml), washed with saturated NaHCOs solution (10 ml), saturated NH₄Cl solution (10 ml) and saturated brine solution (10 ml). The organic phase was dried over MgSO₄, concentrated *in vacuo* and purified by column chromatography (1-6 % MeOH in DCM) to afford the title compound as a colourless solid (281 mg, 55 %).
LCMS *m*/*z* = 496 [M+H]⁺
¹H NMR (MeOD-d₄, 396 MHz): δ: 8.95 (s, 1H), 8.73 (d, 1H), 8.06 (m, 2H), 7.82-7.77 (m, 3H), 7.63-7.56 (m, 3H), 6.38-6.17 (m, 2H), 5.47-5.37 (m, 2H), 4.95-4.82 (m, 2H), 3.94-3.85 (m, 1H), 3.31 (s, 3H), 3.09-2.89 (m, 1H).

### Example 8

### 6-{(2R,4S)-4-fluoro-2-[5-fluoro-2-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide

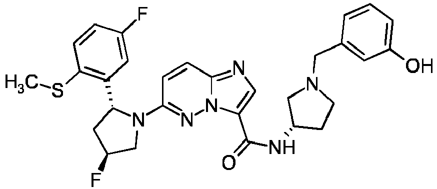

To a suspension of 3-{[(3S)-3-aminopyrrolidin-1-yl]methyl}phenol hydrochloride (Preparation 54, 1.02 g, 3.86 mmol) in DCM (40 ml) was added triethylamine ( 1.79 ml, 12.87 mmol) and the mixture was cooled to 3°C in ice/water bath. 6-[(2S,4S)-4-fluoro-2-[5-fluoro-2-(methylsulfanyl)phenyl]cyclopentyl]imidazo[1,2-b]pyridazine-3-carbonyl chloride (Preparation 43, 1.05 g, 2.57 mmol) in DCM (40 ml) was added dropwise over 10 min. The reaction was left to stir in ice/water bath for 30 min and then left to stir for 3 h at rt. The reaction was quenched by addition of saturated NaHCO₃ solution (50 ml). The aqueous layer was separated and the organic layer washed with NH₄Cl (50 ml) and brine (2 × 50 ml). The organic layer was filtered through a phase separator and reduced to dryness to give the title compound as a pale yellow foam (1.37 g, 95%).
LCMS *m*/*z* = 565.3 [M+H]⁺ and 563.2 [M-H]⁻
¹H NMR (MeOD-d₄, 396 MHz): δ: 8.05 (m, 1H), 7.72 (m, 1H), 7.42 (m, 1H), 6.98-7.15 (m, 3H), 6.82-6.85 (m, 2H), 6.67-6.69 (m, 1H), 6.51 (m, 1H), 5.57 (m, 1H), 5.29-5.42 (m, 1H), 4.63 (s, 1H), 4.07-4.19 (m, 2H), 3.52-3.71 (m, 2H), 2.75-3.03 (m, 4H), 2.58 (m, 3H), 2.42-2.51 (m, 2H), 2.12-2.29 (m, 1H), 1.84-1.90 (m, 1H).

### Example 9

### 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}phenyl pentanoate

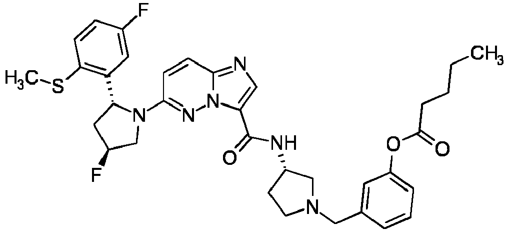

To a solution of 6-[(2R,4S)-4-fluoro-2-[5-fluoro-2-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide (Example 8, 65 mg, 0.117 mmol) in pyridine (3.0 ml) was added valeroyl chloride (30 mg, 0.246 mmol) at 0°C and the reaction was stirred at for 2h. A further portion of valeroyl chloride (21 mg, 0.175 mmol) was added to the reaction and stirred at 0°C for 2 h. The reaction was diluted with EtOAc (30 ml) and washed with saturated NaHCOs solution (3 × 50 ml) and brine (30 ml), dried (Mg₂SO₄) and concentrated *in vacuo.* The crude material was purified by normal phase chromatography 1-3% DCM/MeOH followed by reverse phase purification 40-100% acetonitrile/water. The material was re-purified by reverse phase chromatography (5-60%) to afford the title compound as a white solid (5.0 mg, 6.6%).
LCMS *m*/*z* = 649 [M+H]⁺
¹H NMR (MeOD-d₄, 396 MHz): δ: 8.05 (s, 1H), 7.35 (m, 1H), 7.43 (m, 1H), 7.35 (m, 1H), 7.25 (m, 1H), 7.19-7.16 (m, 1H), 7.07 (m, 1H), 7.02-6.95 (m, 2H), 6.51 (m, 1H), 5.56 (m, 1H), 5.33 (m, 1H), 4.69-4.57 (m, 1H), 4.25-4.05 (m, 2H), 3.78 (m, 1H), 3.72 (m, 1H), 3.10-2.86 (m, 3H), 2.72 (m, 1H), 2.56 (s, 3H), 2.53-2.40 (m, 4H), 2.28-2.09 (m, 1H), 1.94-1.83 (m, 1H), 1.61-1.52 (m, 2H), 1.41-1.27 (m, 2H), 0.89 (m, 3H).

### Example 10

### Methyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate

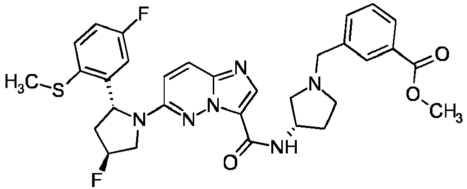

6-[(2R,4S)-4-fluoro-2-[5-fluoro-2-(methylsulfanyl)phenyl]pyrrolidin-1-yl]-N-[(3S)-pyrrolidin-3-yl]imidazo[1,2-b]pyridazine-3-carboxamide (Preparation 36, 0.20 g, 0.436 mmol) and methyl 3-formylbenzoate (0.0787 g, 0.479 mmol) in dry DCM (2.73 ml) were stirred for 30 min at rt under N₂. Sodium triacetoxyborohydride (0.277 g, 1.31 mmol) was added in one portion followed by acetic acid (0.0249 ml, 0.436 mmol) and the mixture was left to stir for 3 h at rt. The reaction was quenched by dropwise addition of saturated aqueous NaHCOs (5 ml). A further 15 ml of DCM was added. The layers were separated, the aqueous layer was extracted with DCM (2 × 15 ml). The combined organic layers were dried and concentrated *in vacuo* to afford a colourless oil. The crude residue was purified by column chromatography (Biotage^{®} Zip KP Sil 120 g cartridge 1-10 % MeOH in DCM for 10 CV) to give the title compound as a colourless solid (0.219 g, 83 %).
LCMS *m*/*z =* 607.1 [M+H]⁺
¹H NMR (MeOD-d₄, 396 MHz): δ: 8.07 (s, 1H), 8.03 (s, 1H), 7.89 (d, 1H), 7.73-7.70 (m, 1H), 7.60 (m, 1H), 7.45-7.38 (m, 2H), 7.04 (m, 1H), 6.96 (m, 1H), 6.50 (m, 1H), 5.58-5.29 (m, 2H), 4.62-4.61 (m, 1H), 4.27-4.17 (m, 2H), 3.78-3.76 (m, 5H), 3.06-2.83 (m, 3H), 2.72 (m, 1H), 2.55 (s, 3H), 2.51-2.44 (m, 2H), 2.27-2.11 (m, 1H), 1.91-1.87 (m, 1H).

### Example 11

### 6-[(2R,4S)-4-fluoro-2-[5-fluoro-2-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide

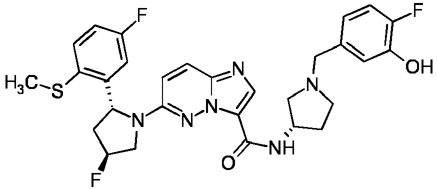

The following compound was prepared in 57% yield from 6-[(2R,4S)-4-fluoro-2-[5-fluoro-2-(methylsulfanyl)phenyl]pyrrolidin-1-yl]-N-[(3S)-pyrrolidin-3-yl]imidazo[1,2-b]pyridazine-3-carboxamide (Preparation 36) and 4-fluoro-3-hydroxybenzaldehyde according to the procedure described in Example 10.
LCMS *m*/*z* = 583.2 [M+H]⁺
¹H NMR (MeOD-d₄, 396 MHz): δ: 8.05 (s, 1H), 7.74 (m, 1H), 7.42 (m, 1H), 7.08-6.97 (m, 4H), 6.82 (m, 1H), 6.56 (m, 1H), 5.57 (m, 1H), 5.40 (d, 1H), 4.60 (m, 1H), 4.22 (s, 1H), 4.14 (s, 1H), 3.77-3.66 (m, 2H), 3.10-2.89 (m, 4H), 2.57 (s, 3H), 2.50 (m, 2H), 2.29-2.13 (m, 1H), 1.95 (m, 1H).

### Example 12

### Butyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate

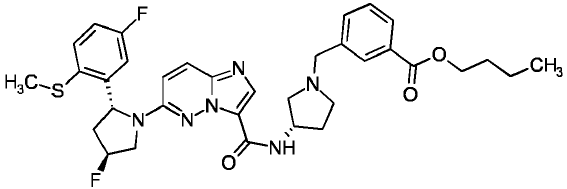

A solution of 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoic acid (Preparation 44, 78 mg, 0.132 mmol), DMAP (4.8 mg, 0.039 mmol) and EDC.HCl (50 mg, 0.264 mmol) in n-butanol (0.66 ml) was stirred for 16 h at rt. The reaction mixture was partitioned between water (5 ml) and EtOAc (15 ml), the organic layer washed with brine (3 × 10 ml), dried with Na2SO4, filtered and the solvent removed under reduced pressure. The crude material was purified by using NP chromatography (1- 5 % MeOH in DCM) to afford the title compound as a colourless solid (404 mg, 47%).
LCMS *m*/*z* = 649 [M+H]⁺
¹H NMR (MeOD-d₄, 396 MHz): δ: 8.08 (s, 1H), 8.03 (s, 1H), 7.89 (m, 1H), 7.71 (m, 1H), 7.59 (m, 1H), 7.45-7.38 (m, 2H), 7.06-6.94 (m, 2H), 6.50 (m, 1H), 5.57-5.27 (m, 2H), 4.62 (s, 1H), 4.24-4.13 (m, 4H), 3.80 (s, 2H), 3.09-3.06 (m, 1H), 2.99-2.86 (m, 2H), 2.72 (m, 1H), 2.54 (s, 3H), 2.51-2.44 (m, 2H), 2.26-2.11 (m, 1H), 1.92-1.90 (m, 1H), 1.66-1.58 (m, 2H), 1.42-1.33 (m, 2H), 0.94-0.86 (m, 3H).

### Example 13

### Ethyl 3-{[(3S)-3-{6-[(2R,4S)-4-fluoro-2-{5-fluoro-2-(methylsulfanyl)phenyl]pyrrolidin-1-yl]imidazo[1,2-b]pyridazine-3-amido}pyrrolidin-1-yl]methyl}benzoate

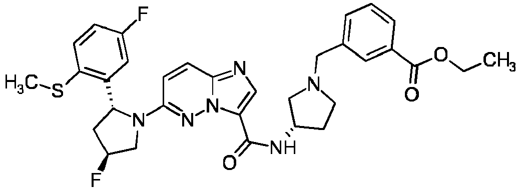

The following compound was prepared in 61% yield from 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoic acid (Preparation 44) and EtOH according to the procedure described in Example 12.
LCMS *m*/*z* = 621.1 [M+H]⁺
¹H NMR (MeOD-d₄, 396 MHz): δ: 8.07 (s, 1H), 8.03 (s, 1H), 7.89 (m, 1H), 7.71 (m, 1H), 7.59 (m, 1H), 7.38-7.44 (m, 2H), 6.94-7.06 (m, 2H), 6.50 (m, 1H), 5.29-5.57 (m, 2H), 4.62 (s, 1H), 4.17-4.28 (m, 4H), 3.75-3.82 (m, 2H), 2.69-3.10 (m, 4H), 2.55 (s, 3H), 2.42-2.50 (m, 2H), 2.12-2.27 (m, 1H), 1.87-1.91 (m, 1H), 1.24-1.28 (m, 3H).

### Example 14

### 6-[(2R,4S)-4-fluoro-2-[5-fluoro-2-(methylsulfanyl)phenyl]pyrrolidin-1-yl]-N-[(4-fluoro-3-hydroxyphenyl)methyl]imidazo[1,2-b]pyridazine-3-carboxamide

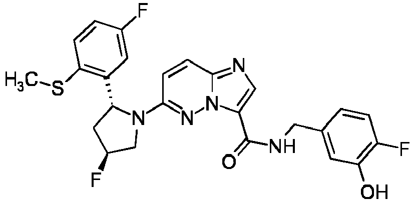

TPTU (18.26 g, 61.5 mmol) was added to a stirred suspension of 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 28, 20 g, 51.2 mmol), 5-(aminomethyl)-2-fluorophenol (14.46 g, 102.5 mmol), and DIEA (33.1 g, 256 mmol) in DMSO (200 ml). The reaction mixture was stirred at RT for 1.5 h. The reaction mixture was poured into EtOAc (600 ml) and water (600 ml) and the layers were separated. The organic layer was washed with distilled water (4 × 400 ml) and brine (400 ml). The organic layer was dried (MgSO₄), filtered and concentrated *in vacuo* to afford an oil. The crude material was purified by column chromatography (20 % MeOH in DCM) to give an oil. This was purified further on the Biotage^{®} by reverse phase chromatography (0.1 % NH3 in H2O/0.1 % NH3 in MeCN, 5 to 95) to give the bis-coupled product (7 g) and the title compound as an off white solid (5.60 g, 21 %).
LCMS *m*/*z* = 514 [M+H]⁺ and 512 [M-H]⁻

The bis-coupled product (7 g) was dissolved in EtOH (15 ml) at rt and sodium hydroxide (1.58 g, 7.9 mmol) was added which was previously dissolved in water (10 ml). The mixture was stirred for 1 h. The EtOH was removed *in vacuo,* diluted with water (50 ml) and then pH was adjusted with 2M HCl to ca. pH 4 to 5. The mixture was then extracted with DCM (2 × 15 ml). The combined organic layers were dried (Na₂SO₄), filtered and concentrated *in vacuo* to afford an off-white solid (3.99 g). This was purified further by silica column chromatography (5 % MeOH in EtOAc) to give the title compound as an off-white solid (1.84 g, 3.58 mmol, 45 %).
LCMS *m*/*z* = 514 [M+H]⁺ and 512 [M-H]⁻
¹H NMR (MeOD-d₄, 396 MHz): δ: 8.03 (s, 1H), 7.82 (m, 1H), 7.13 (m, 1H), 6.99 (m, 1H), 6.92 (m, 3H), 6.89 (m, 1H), 6.79 (m, 1H), 5.51-5.25 (m, 2H), 4.47 (s, 2H), 4.01-3.87 (m, 2H), 3.12-2.94 (m, 1H), 2.41 (s, 3H), 2.07 (m, 1H).

### Example 15

### N-[(3S)-1-[(4-fluoro-3-hydroxyphenyl)methyl]pyrrolidin-3-yl]-6-[(2R)-2-[3-fluoro-5-(methylsulfanyl)phenyl]pyrrolidin-1-yl]imidazo[1,2-b]pyridazine-3-carboxamide

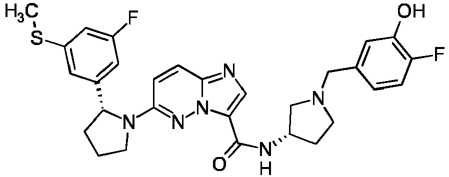

To a solution of 6-[(2R)-2-[3-fluoro-5-(methylsulfanyl)phenyl]pyrrolidin-1-yl]-N-[(3S)-pyrrolidin-3-yl]imidazo[1,2-b]pyridazine-3-carboxamide (Preparation 19, 70 mg, 0.16 mmol) in DCM (2 ml) were added 4-fluoro-3-hydroxybenzaldehyde (25 mg, 0.18 mmol) and sodium triacetoxyborohydride (144 mg, 0.66 mmol). The reaction mixture was stirred at rt for 1h and quenched with water and extracted with DCM. The combined organic layers were dried over anhydrous Na₂SO₄ and reduced to dryness. The obtained residue was purified by normal phase chromatography (eluting with 2 to 10% MeOH in DCM) and reverse phase chromatography (eluting with 0.1 NH₃ in H2O/0.1 NH₃ in MeCN 2 to 80% over 10 CVs) to afford the title compound as a white solid (20 mg, 22%).
LCMS *m*/*z* = 565.1 [M+H]⁺
¹H NMR (DMSO-d₆, 396 MHz): δ: 9.74 (s, 1H), 9.11 (s, 1H), 7.93 (m, 2H), 7.06-6.92 (m, 4H), 6.81-6.54 (m, 2H), 5.16 (m, 1H), 4.45 (m, 1H), 3.95-3.90 (m, 1H), 3.75-3.43 (m, 4H), 2.73-2.40 (m, 4H), 2.45 (s, 3H), 2.32-2.19 (m, 2H), 1.91 (m, 4H).

### Example 16

### Methyl 3-{[(3S)-3-{6-[(2R,4S)-4-fluoro-2-[3-fluoro-5-(methylsulfanyl)phenyl]pyrrolidin-1-yl]imidazor1,2-b]pyridazine-3-amido}pyrrolidin-1-yl]methyl}benzoate

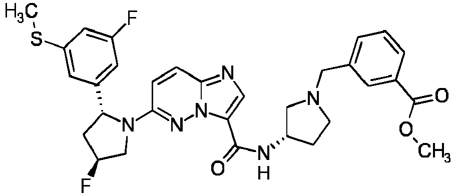

To a solution of 6-[(2R,4S)-4-fluoro-2-[3-fluoro-5-(methylsulfanyl)phenyl]pyrrolidin-1-yl]-N-[(3S)-pyrrolidin-3-yl]imidazo[1,2-b]pyridazine-3-carboxamide (Preparation 37, 386 mg, 0.842 mmol) in DCM (15 ml) was added methyl 3-formylbenzoate (276 mg, 1.68 mmol) followed by acetic acid (0.096 ml, 1.68 mmol) and the reaction stirred at rt under N₂ for 30 min. Sodium triacetoxyborohydride (357 mg, 1.68 mmol) was added and the mixture left to stir at rt for 18 h. After this time the reaction was quenched by the addition of saturated aqueous NaHCO₃ (20 ml) and extracted with EtOAc (15 ml × 3). The combined organic layers were washed with brine (30 ml), dried (Na₂SO₄), filtered, and evaporated to dryness to give a light brown residue. The residue was loaded onto a Biotage^{®} SNAP KP-Sil 100 g cartridge eluting with a gradient of MeOH in EtOAc (1 to 4%, 3CV; 4 to 6%, 1C; 20%, 1CV) followed by 5% MeOH in DCM. The correct fractions were combined to give the title compound as colourless solid (304 mg, 66%).
LCMS *m*/*z* = 607.1 [M+H]⁺
¹H NMR (DMSO-d₆, 396 MHz): δ: 8.90 (s, 1H), 7.98-7.93 (m, 3H), 7.86 (d, 1H), 7.63 (d, 1H), 7.49 (m, 1H), 7.12 (s, 1H), 6.96 (m, 2H), 6.74 (d, 1H), 5.44 (d, 1H), 5.27 (m, 1H), 4.49 (s, 1H), 4.27-4.06 (m, 2H), 3.85-3.65 (m, 2H), 3.78 (s, 3H), 3.4-3.3 (m, 2H), 2.93-2.63 (m, 3H), 2.50 (s, 3H), 2.33-2.09 (m, 2H), 1.66 (m, 1H),

The following compounds were prepared from the corresponding amine and aldehydes according to the procedure described in Example 16.

| Example No | Structure and Name | Starting materials, Yield and Data |
|---|---|---|
| 17 | 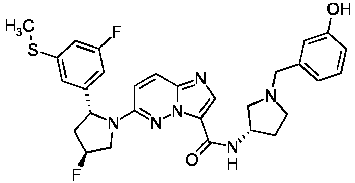 6-[(2R,4S)-4-fluoro-2-[3-fluoro-5-(methylsulfanyl)phenyl]pyrrolidin-1-yl]-N-[(3S-1[(3hydroxyphenyl) methyl]pyrrolidin-3-yl]imidazo[1,2-b]pyridazine-3-carboxamide | Amine: Preparation 37 62%. |
| | | LCMS *m*/*z* = 565 [M+H]⁺ |
| | | ¹H NMR (MeOD-d₄, 396 MHz): δ: 8.04 (s, 1H), 7.76 (m, 1H), 7.15 (m, 1H), 7.05 (s, 1H), 6.90 (m 1H), 6.88-6.76 (m, 4H), 6.70 (m, 1H), 5.45-5.21 (m, 2H), 4.67-4.54 (m, 1H), 4.26-4.00 (m, 2H), 3.73 (m, 1H), 3.65 (m, 1H), 3.15-3.01 (m, 1H), 3.00-2.77 (m, 3H), 2.59-2.37 (m, 5H), 2.20 (m, 1H), 1.88-1.69 (m, 1H). |
| 18 | 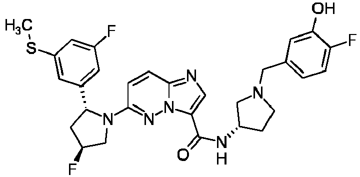 6-[(2R,4S)-4-fluoro-2-[3-fluoro-5-(methylsulfanyl)phenyl]pyrrolidin-1-yl]-N-[(3S)-1-[(4-fluoro-3-hydroxy phenyl)methyl]pyrrolidin-3-yl]imidazo[1,2-b]pyridazine-3-carboxamide | Amine: Preparation 37 33%. |
| | | LCMS *m*/*z* = 583.2 [M+H]⁺ |
| | | ¹H NMR (MeOD-d₄, 396 MHz): δ: 8.03 (s, 1H) 7.75 (d, 1H) 7.00 (s, 1H) 7.01-6.95 (m, 2H) 6.91-6.88 (m, 1H) 6.82-6.80 (m, 3H) 5.43-5.23 (m, 2H) 4.64-4.56 (m, 1H) 4.18-4.11(m, 2H) 3.67 (d, 1H) 3.58 (d, 1H) 3.00-2.91 (m, 2H) 2.81-2.79 (m, 2H) 2.45-2.41 (m, 5H) 2.26-2.16 (m, 1H) 1.83-1.71 (m, 1H). |
| 19 | 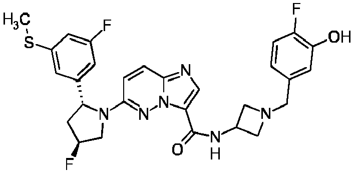 6-[(2R,4S)-4-fluoro-2-[3-fluoro-5-(methylsulfanyl)phenyl]pyrrolidin-1-yl]-N-{1-[(4-fluoro-3-hydroxyphenyl) methyl]azetidin-3-yl}imidazo[1,2-b]pyridazine-3-carboxamide | Amine: Preparation 38 33.1 mg, 31%. |
| | | LCMS *m*/*z* = 569.1 [M+H]⁺ |
| | | ¹H NMR (MeOD-d₄, 396 MHz): δ: 8.04 (s, 1H) 7.79 (d, 1H) 7.06 (s, 1H) 7.05-7.00 (m, 1H) 6.95-6.79 (m, 5H) 5.53-5.27 (m, 2H) 4.58-4.56 (m, 1H) 4.26-4.19 (m, 2H) 3.81-3.74 (m, 4H) 3.51-3.39 (m, 2H) 3.01-2.91 (m, 1H) 2.45 (s, 3H) 2.30-2.15 (m, 1H). |
| 20 | 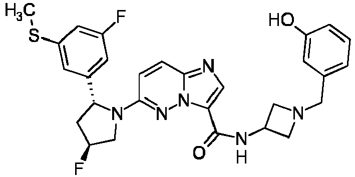 6-[(2R,4S)-4-fluoro-2-[3-fluoro-5-(methylsulfanyl)phenyl]pyrrolidin-1-yl]-N-{1-[(3-hydroxyphenyl)methyl]azetidin-3-yl}imidazo[1,2-b]pyridazine-3-carboxamide | Amine: Preparation 38 13%. |
| | | LCMS *m*/*z* = 551.1 [M+H]⁺ |
| | | ¹H NMR (MeOD-d₄, 396 MHz): δ: 8.04 (s, 1H) 7.77 (d, 1H) 7.16 (m, 1H) 7.06 (s, 1H) 6.91-6.79 (m, 5H) 6.72-6.70 (m, 1H) 5.53-5.26 (m, 2H) 4.60-4.57 (m, 1H) 4.26-4.29 (m, 2H) 3.76-3.69 (m, 4H) 3.36-3.31 (m, 2H) 3.01-2.91 (m, 1H) 2.45 (s, 3H) 2.31-2.14 (m, 1H). |
| 21 | 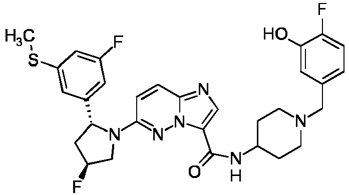 6-[(2R,4S)-4-fluoro-2-[3-fluoro-5-(methylsulfanyl)phenyl]pyrrolidin-1-yl]-N-{1-[(4-fluoro-3-hydroxyphenyl)methyl]piperidin-4-yl]imidazo[1,2-b]pyridazine-3-carboxamide | Amine: Preparation 39 19 mg, 38%. |
| | | LCMS *m*/*z* = 597.2 [M+H]⁺ |
| | | ¹H NMR (MeOD-d₄, 396 MHz): δ: 8.10-7.91 (m, 1H), 7.79-7.58 (m, 1H), 7.11-6.61 (m, 7H), 5.58-5.14 (m, 2H), 4.28-4.00 (m, 2H), 4.00-3.83 (m, 1H), 3.69-3.47 (m, 2H), 3.11-2.80 (m, 3H), 2.50-1.85 (m, 8H), 1.78-1.57 (m, 2H). |
| 22 | 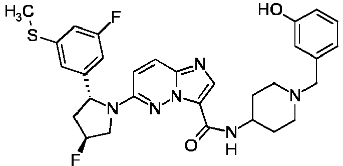 6-[(2R,4S)-4-fluoro-2-[3-fluoro-5-(methylsulfanyl)phenyl]pyrrolidin-1-yl]-N-{1-[(3-hydroxyphenyl)methyl]piperidin-4-yl}imidazo[1,2-b]pyridazine-3-carboxamide | Amine: Preparation 39 70%. |
| | | LCMS *m*/*z* = 579.2 [M+H]⁺ |
| | | ¹H NMR (MeOD-d₄, 396 MHz): δ: 8.05 (s, 1H), 7.77 (d, 1H), 7.16 (m, 1H), 7.07 (m, 1H), 6.91 (m, 1H), 6.84-6.81 (m, 4H), 6.72 (d, 1H), 5.46 (d, 1H), 5.27 (m, 1H), 4.21-4.20-4.14 (m, 2H), 3.97-3.90 (m, 1H), 3.52 (m, 2H), 3.01-2.91 (m, 3H), 2.43 (s, 3H), 2.32-2.15 (m, 3H), 2.15-2.00 (m, 2H) 1.76-1.64 (m, 2H). |
| 23 | 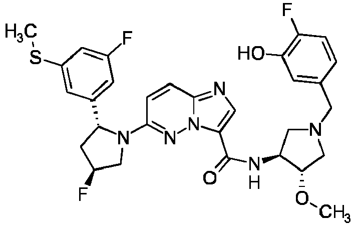 6-[(2R,4S)-4-fluoro-2-[3-fluoro-5-(methylsulfanyl)phenyl]pyrrolidin-1-yl]-N-[(3S,4S)-1-[(4-fluoro-3-hydroxyphenyl)methyl]-4-methoxypyrrolidin-3-yl]imidazo[1,2-b]pyridazine-3-carboxamide | Amine: Preparation 40 42.5 mg, 48%. |
| | | LCMS *m*/*z* = 613.3 [M+H]⁺ |
| | | ¹H NMR (DMSO-d₆, 396 MHz): δ: 8.05 (s, 1H) 7.79-7.37 (m, 1H) 7.05-6.93 (m, 4H) 6.85-6.76 (m, 3H) 5.41-5.27 (m, 2H) 4.49-4.42 (m, 1H) 4.25-3.99 (m, 2H) 3.91-3.55 (m, 3H) 3.44-3.38 (s, 3H) 3.27-3.21 (m, 1H) 2.92-2.86 (m, 2H) 2.80-2.72 (m, 1H) 2.46-2.37 (m, 4H), 2.28-2.10 (m, 1H). |
| 24 | 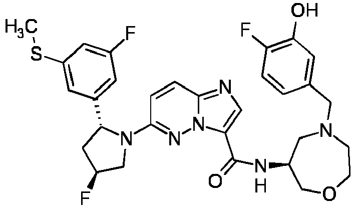 6-[(2R,4S)-4-fluoro-2-[3-fluoro-5-(methylsulfanyl)phenyl]pyrrolidin-1-yl]-N-[(6S)-4-[(4-fluoro-3-hydroxyphenyl)methyl]-1,4-oxazepan-6-yl]imidazo[1,2-b]pyridazine-3-carboxamide* | Amine: Preparation 41 26 mg, 46%. |
| | | LCMS *m*/*z* = 613.2 [M+H]⁺ |
| | | ¹H NMR (DMSO-d₆, 396 MHz): δ: 9.57 (d, 1H), 9.20 (s, 1H), 8.08-7.93 (m, 2H), 7.16-7.11 (m, 1H), 7.03-6.83 (m, 2H), 6.78-6.64 (m, 3H), 5.53 (m, 1H), 5.30 (m, 1H), 4.44-4.12 (m, 3H), 3.96 (m, 1H), 3.84-3.63 (m, 4H), 3.55-3.47 (m, 1H), 2.94-2.67 (m, 5H), 2.49 (m, 4H), 2.34-2.14 (m, 1H). |
| | | *Stereochemistry arbitrarily assigned. |
| 25 | 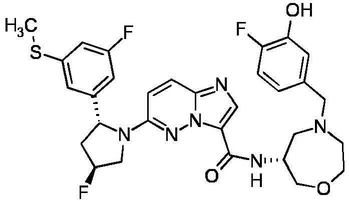 6-[(2R,4S)-4-fluoro-2-[3-fluoro-5-(methylsulfanyl)phenyl]pyrrolidin-1-yl]-N-[(6R)-4-[(4-fluoro-3-hydroxyphenyl)methyl]-1,4-oxazepan-6-yl]imidazo[1,2-b]pyridazine-3-carboxamide* | Amine: Preparation 42 27%. |
| | | LCMS *m*/*z* = 613.2 [M+H]⁺ |
| | | ¹H NMR (MeOD-d₄, 396 MHz): δ: 8.05 (s, 1H), 7.84 (m, 1H), 7.13 (m, 1H), 6.98-6.84 (m, 5H), 6.74 (m, 1H), 5.52-5.39 (m, 1H), 5.28 (m, 1H), 4.24-4.16 (m, 2H), 4.00-3.97 (m, 1H), 3.78-3.73 (m, 2H), 3.62-3.43 (m, 4H), 2.90-2.70 (m, 3H), 2.47-2.45 (m, 3H), 2.28-2.15 (m, 2H), 1.98 (m, 1H). |
| | | *Stereochemistry arbitrarily assigned. |

### Example 26

### 6-(2R,4S)-4-fluoro-2-[3-fluoro-5-(methylsulfanyl)phenyl]pyrrolidin-1-yl]-N-[(4-fluoro-3-hydroxyphenyl)methyl]imidazo[1,2-b]pyridazine-3-carboxamide

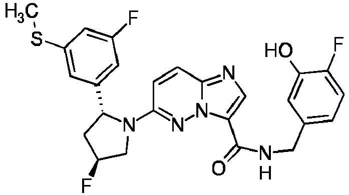

6-[(2R,4S)-4-fluoro-2-[3-fluoro-5-(methylsulfanyl)phenyl]pyrrolidin-1-yl]imidazo[1,2-b]pyridazine-3-carboxylic acid (Preparation 29, 109 mg, 0.28 mmol), 1-{3-[(tertbutyldimethylsilyl)oxy]-4-fluorophenyl}methanamine (Preparation 57, 79 mg, 0.31 mmol) and HATU (130 mg, 0.34 mmol) were dissolved in DMF (4.5 ml) under N₂ and DIPEA (108 mg, 0.84 mmol) was added. The reaction was stirred overnight at rt. Water (10 ml) and EtOAc (10 ml) were added and the layers were partitioned. The organic layer was washed with water (3 × 10 ml) and with brine (10 ml). After drying over MgSO4 the solvent was removed under vacuum. The crude was used in the next step without further purification.
LCMS *m*/*z* = 628.4 [M+H]⁺

The crude was dissolved in MeCN (2 ml) and TEAF.H₂O (117 mg, 0.7 mmol) was added. The reaction was heated at 50 °C. After 2 h the reaction mixture was cooled down to rt and the solvent was removed under vacuum. The crude was purified by reverse phase chromatography eluting with H₂O (0.1% NH₃):MeCN (0.1% NH₃) - 2 to 70% over 15 CV. The product was obtained as white solid after freeze-drying (73 mg, 51%).
LCMS *m*/*z* = 514.3 [M+H]⁺
¹H NMR (DMSO-d₆, 396 MHz): δ: 9.73 (s, 1H); 8.53 (br s, 1H); 7.98 (d, 1H); 7.93 (s, 1H); 7.06-7.00 (m, 2H); 6.84-6.81 (m, 4H); 6.65 (br s, 1H); 5.38 (d, 1H); 5.13 (m, 1H); 4.45 (m, 1H); 4.23-3.90 (m, 3H); 2.83-2.73 (m, 1H); 2.35 (s, 3H); 2.11-1.94 (m, 1H).

### Example 27

### 6-[(2R,4S)-4-fluoro-2-[3-fluoro-5-(methylsulfanyl)phenyl]pyrrolidin-1-yl]-N-[(3S)-1-(3-hydroxybenzoyl)pyrrolidin-3-yl]imidazo[1,2-b]pyridazine-3-carboxamide

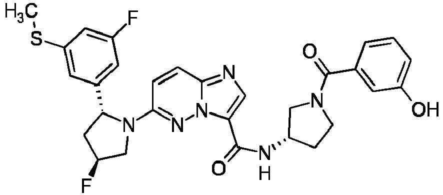

To 6-[(2R,4S)-4-fluoro-2-[3-fluoro-5-(methylsulfanyl)phenyl]pyrrolidin-1-yl]-N-[(3S)-pyrrolidin-3-yl]imidazo[1,2-b]pyridazine-3-carboxamide (Preparation 37, 70 mg, 0.152 mmol) in DMF (2 ml) were added 3-hydroxybenzoic acid (23 mg, 0.167 mmol) and HATU (64 mg, 0.167 mmol, 1.1 equiv.). The mixture was stirred at rt for 5 min, N-ethyl- N-isopropylpropan-2-amine (0.053 ml, 0.30 mmol) added and the reaction mixture stirred at rt for a further 16 h before diluting with EtOAc (15 ml). This was then washed with water (15 ml), saturated brine solution (15 ml), dried over Na₂SO₄, concentrated *in vacuo* and purified by Biotage^{®} ZIP KP-SIL 45g cartridge (EtOAc:MeOH, 100:0 to 90:10) to afford a solid which was loaded onto a Biotage^{®} C18 cartridge and eluted with a gradient (2to95 in 10CV) of ACN in water (both phases containing 0.1% ammonium hydroxide). The combined fractions were evaporated, redissolved in EtOAc, washed with water, dried (Na₂SO₄), filtered and evaporated to give the title compound as a colourless solid (36 mg, 40%).
LCMS *m*/*z* = 579.1 [M+H]⁺
¹H NMR (DMSO-d₆, 396 MHz): δ: 9.61 (d, 1H), 8.73-8.62 (br m, 1H), 7.90-7.97 (m, 2H), 7.19 (m, 1H), 7.11 (s, 1H), 6.69-6.96 (m, 6H), 5.25-5.54 (m, 2H), 4.83-4.39 (m, 1H), 3.95-4.22 (m, 2H), 3.37-3.84 (m, 3H), 2.77-2.91 (m, 1H), 2.46-2.45 (m, 4H), 1.86-2.29 (m, 3H).

### Example 28

### 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}phenyl acetate

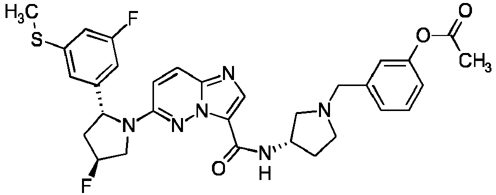

6-[(2R,4S)-4-fluoro-2-[3-fluoro-5-(methylsulfanyl)phenyl]pyrrolidin-1-yl]-N-[(3S-1[(3hydroxyphenyl)methyl]pyrrolidin-3-yl]imidazo[1,2-b]pyridazine-3-carboxamide (Example 17, 60.7 mg, 0.107 mmol) was dissolved in pyridine (1.07 ml), acetic anhydride (20 µL, 0.215 mmol) was added and the mixture was stirred at rt for 1 hour. The pyridine was removed under reduced pressure and the crude material was dissolved in water (5 ml) and extracted with EtOAc (3 × 5 ml). The combined organic layers were dried over anhydrous Na₂SO₄, filtered and the solvent removed under reduced pressure. The crude material was purified by Biotage^{®} chromatography (gradient 1-10 % MeOH in DCM) to give the title compound as a colourless solid (54.6 mg, 84%).
LCMS *m*/*z* = 607.2 [M+H]⁺
¹H NMR (MeOD-d₄, 396 MHz): δ: 8.04 (s, 1H) 7.76 (d, 1H) 7.35 (m, 1H) 7.25 (d, 1H) 7.16 (s, 1H) 7.04 (s, 1H) 7.00-6.98 (m, 1H) 6.92-6.89 (m, 1H) 6.82-6.79 (m, 2H), 5.38-5.25 (m, 2H) 4.66-4.57 (m, 1H) 4.16-4.07 (m, 2H) 3.77 (d, 1H) 3.69 (d, 1H) 3.02-2.75 (m, 4H) 2.46-2.39 (m, 5H) 2.25-2.16 (m, 4H) 1.82-1.72 (m, 1H).

### Example 29

### 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid

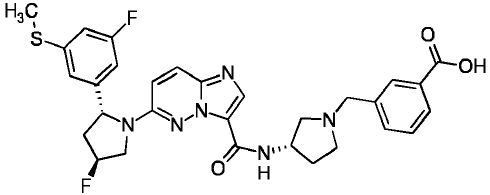

To a solution of methyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl} benzoate (Example 16, 234 mg, 0.386 mmol) in MeOH (2.0 ml) was added dropwise a solution of NaOH (77 mg, 1.93 mmol, 5.0 eq), dissolved in water (0.5 ml). The reaction mixture was stirred at rt for 30 min. After this time the reaction was adjusted to pH 4 by addition of 2.0 M HCl, the volatiles removed under reduced pressure and the aqueous extracted with DCM (10 ml). The organic phase was dried (Na₂SO₄), filtered and reduced to dryness to give the title compound as brown solid (100 mg) which was used without further purification. The aqueous layer was reduced to dryness, the residue was loaded onto a SNAP KP-SIL Biotage^{®} cartridge and purified eluting with a gradient of MeOH in EtOAc (1 to 4% in 3CV, 4 to 6% in 1CV, 20% for 1CV) then 20% MeOH in DCM. The correct fractions were collected and reduced to dryness to give a second batch of the title compound as colourless solid (101 mg, 87% overall).
LCMS *m*/*z =* 593.1 [M+H]⁺
¹H NMR (DMSO-d₆, 396 MHz): δ: 8.84 (br s, 1H), 7.91-7.72 (m, 4H), 7.34-7.22 (m, 2H), 7.09 (s, 1H), 6.94 (m, 2H), 6.67 (d, 1H), 5.38 (d, 1H), 5.20 (m, 1H), 4.52-4.36 (m, 1H), 4.19-3.98 (m, 2H), 3.73-3.55 (m, 2H), 2.87-2.58 (m, 4H), 2.44-2.41 (m, 3H), 2.36-2.09 (m, 2H), 1.84 (s, 1H), 1.65-1.49 (m, 1H).

### Example 30

### Butyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate

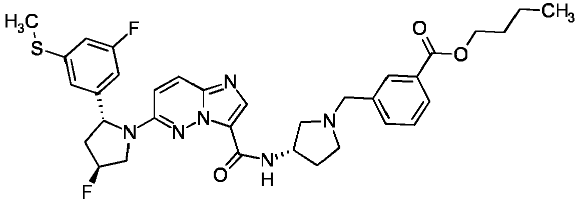

3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid (Example 29, 100 mg, 0.168 mmol), 4-dimethylamino pyridine (4.1 mg, 0.032 mmol), N-(3-dimethylaminopropyl)-*N*'-ethylcarbodiimide hydrochloride (64 mg, 0.337 mmol) and n-butanol (0.077 ml, 0.843 mmol) were dissolved in DMF (0.84 ml) under N₂. The reaction was stirred at rt for 16 h. After this time 4-dimethylamino pyridine (4,1 mg, 0.032 mmol), *N*-(3-dimethylaminopropyl)-*N*'-ethylcarbodiimide hydrochloride (64 mg, 0.337 mmol) and n-butanol (0.077 ml, 0.843 mmol) were added. The reaction was heated to 40°C and stirred for 18 h. The reaction was quenched by addition of water and extracted with EtOAc (15 ml). The organic layer was washed with brine (3 × 10 ml), dried with Na₂SO₄, filtered and the solvent removed under reduced pressure. The crude material was purified by chromatography (5 % MeOH in DCM for 10 CV). The resulting solid was further purified by reverse phase column chromatography (acetonitrile/water) to give the title compound as colourless solid (104 mg, 10%).
LCMS *m*/*z* = 649.4 [M+H]⁺
¹H NMR (MeOD-d₄, 396 MHz): δ: 8.02-8.07 (m, 2H), 7.89 (d, 1H), 7.72-7.76 (m, 1H), 7.59 (d, 1H), 7.43 (m, 1H), 7.02 (s, 1H), 6.88 (m, 1H), 6.75-6.80 (m, 2H), 5.21-5.38 (m, 2H), 4.61-4.62 (m, 1H), 4.13-4.25 (m, 4H), 3.78 (s, 2H), 2.70-3.10 (m, 4H), 2.41-2.48 (m, 5H), 2.11-2.28 (m, 1H), 1.73-1.78 (m, 1H), 1.60-1.68 (m, 2H), 1.36-1.43 (m, 2H), 0.86-0.92 (m, 3H).

### Example 31

### 5-[(2R,4S)-4-fluoro-2-[3-fluoro-5-(methylsulfanyl)phenyllpyrrolidin-1-yl]-N-{1-f(3-hydroxyphenyl)methyl]piperidin-4-yl}pyrazolo[1,5-a]pyrimidine-3-carboxamide

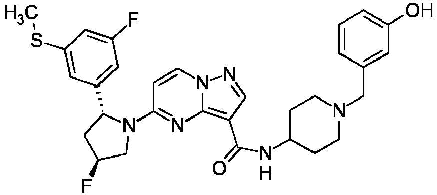

To a solution of 6-[(2R,4S)-4-fluoro-2-[3-fluoro-5-(methylsulfanyl)phenyl]pyrrolidin-1-yl]imidazo[1,2-b]pyridazine-3-carboxylic acid (Preparation 29, 100 mg, 0.256 mmol) in DCM (5.0 ml) at rt, TPTU (91 mg, 0.307 mmol) was added and the reaction was stirred for 10 min. Then 3-((4-aminopiperidin-1-yl)methyl)phenol hydrochloride (Preparation 50, 68 mg, 0.282 mmol) was added, followed by DIPEA (148 mg, 1.28 mmol, 5.0 equiv) and the reaction was allowed to stir for 2 h. The reaction was quenched with a saturated solution of NH₄Cl (30 ml) and extracted with DCM (3 × 50 ml). The combined organic layers were washed with brine, dried and evaporated. The crude was then loaded on NP Biotage^{®} (75-100%EtOAc), but some of the title compound remained on silica. The rest was flushed through (50%THF/DCM) and the cleaner crude purified by RP Biotage^{®}. The crude was loaded on RP biotage and columned under basic conditions to yield 972-102-1 as a white solid (10.4 mg, 7%).
LCMS *m*/*z* = 579 [M+H]⁺
¹H NMR (DMSO-d₆, 396 MHz, 80°C): δ: 8.96 (s, 1H), 8.62 (m, 1H), 8.09 (s, 1H), 7.57-7.30 (m, 1H), 7.07 (m, 2H), 6.94 (m, 1H), 6.86 (d, 1H), 6.75-6.69 (m, 2H), 6.62 (m, 2H), 6.42-6.20 (m, 1H), 5.47 (d, 1H), 5.37-5.28 (m, 1H), 4.30-4.09 (m, 2H), 3.80-3.67 (b, 1H), 3.38 (s, 2H), 2.97-5.69 (m, 3H), 2.42 (s, 3H), 2.18-2.07 (m, 3H), 1.91-1.82 (m, 1H), 1.55-1.38 (m, 2H).

### Preparation 1

### 5-Fluoro-2-(methylsulfanyl)benzaldehyde

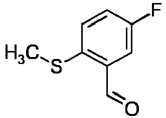

n-BuLi in hexane (2.5 M, 0.4 ml, 1 mmol) was added dropwise to a solution of 2-bromo-4-fluoro-1-(methylsulfanyl)benzene (221.0 mg, 1 mmol) in dry THF (10 ml) at -78°C under N₂ atmosphere, so the temperature was maintained below -70°C. DMF (80.0 mg, 1.1 mmol) was added and the reaction stirred at -78°C for a further 30 mins. The resulting mixture was quenched by the addition of ice-cold sat. aq. NH₄Cl solution (10 ml), warmed to rt and extracted with EtOAc (10 ml). The organic extracts were washed with saturated brine (10 ml), dried (MgSO₄), concentrated *in vacuo* and purified by column chromatography on silica gel eluting with heptanes:EtOAc (95:5) to afford the title compound as colourless oil (88 mg, 52%).

¹H NMR (CDCl₃, 400MHz): δ: 10.35 (s, 1H); 7.52-7.56 (m, 1H); 7.35-7.39 (m, 1H), 7.25-7.30 (m, 1H); 2.51 (s, 3H).

### Preparation 2

### 1-Bromo-3-fluoro-5-(methylsulfanyl)benzene

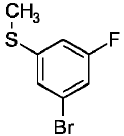

To 1-bromo-3,5-difluorobenzene (50.0 g, 259.08 mmol) in DMF (120 ml) was added sodium methanethiolate (18.16 g, 259.08 mmol). The mixture was heated to 150°C for 30 min, then cooled to rt, poured into a saturated solution of NH₄Cl (250 ml) and extracted with MTBE (2 × 150 ml). The combined organic layers were washed with water (150 ml) and brine (150 ml), dried (MgSO₄), filtered and evaporated to afford the desired compound as a yellow oil (25g, containing solvent residues, quantitative).

¹H NMR (CDCl₃, 400MHz): δ: 7.13 (s, 1H); 6.98-7.01 (m, 1H), 6.85-6.88 (m, 1H); 2.43 (s, 3H).

### Preparation 3

### 3-Fluoro-5-(methylthio)benzaldehyde

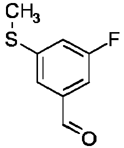

n-BuLi (1.6 M solution in hexanes, 2.82 ml, 4.52 mmol) was added dropwise *via* a syringe to a solution of 1-bromo-3-fluoro-5-(methylsulfanyl)benzene (Preparation 2, 1.0 g, 4.52 mmol) in anhydrous THF (20 ml) under N₂ at -78°C. The mixture was stirred for 15 min before anhydrous DMF (0.42 ml, 5.43 mmol) was added dropwise and the mixture stirred at -78°C for a further 30 min. The mixture was quenched by addition of 20 ml of saturated aqueous solution of NH₄Cl. The two layers were partitioned and the water phase was extracted with EtOAc (10 ml). The organic layers were combined, dried (Na₂SO₄), filtered and evaporated to afford the product as brown oil (0.73 g, 95%).

¹H NMR (CDCl₃, 400MHz): δ: 9.95 (s, 1H); 7.50-7.55 (m, 1H); 7.32-7.35 (m, 1H), 7.15-7.20 (m, 1H); 2.48 (s, 3H).

### Preparation 4

### (R)-N-[(1Z)-[5-fluoro-2-(methylsulfanyl)phenyl]methylidene]-2-methylpropane-2-sulfinamide

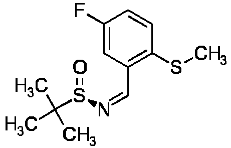

Cs₂CO₃ (300.0 mg, 0.92 mmol) was added to a solution of 5-fluoro-2-(methylsulfanyl)benzaldehyde (Preparation 1, 130.0 mg, 0.76 mmol) and (R)-2-methylpropane-2-sulfinamide (93.0 mg, 0.76 mmol) in DCM (15 ml) and the reaction stirred at rt for 18 h. Water (15 ml) was carefully added and the layers separated. The organic layer was dried (MgSO₄) and evaporated under reduced pressure. The residue was purified by column chromatography on silica gel eluting with heptanes:EtOAc (95:5 to 85:15) to afford the title compound as a yellow oil (130 mg, 62%).
LCMS *m*/*z* = 274.1 [M+H]⁺

### Preparation 5

### (R)-N-[(1Z)-[3-fluoro-5-(methylsulfanyl)phenyl]methylidene]-2-methylpropane-2-sulfinamide

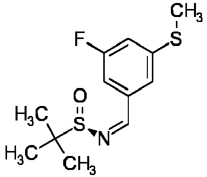

To 3-fluoro-5-(methylthio)benzaldehyde (Preparation 3, 7.85 g, 46.12 mmol), (R)-2-methylpropane-2-sulfinamide (6.71 g, 55.3 mmol) in anhydrous DCM (150 ml) was added cesium carbonate (15.0 g, 46.1 mmol) under N₂ and the mixture was stirred at rt for 16h. The mixture was diluted with water (200 ml) and the organic layer separated, dried (Na₂SO₄), filtered and evaporated. The crude oil was diluted with DCM (5 ml), loaded onto a Zip KP-SIL 120g biotage cartridge and eluted with 10 to 50% EtOAc in heptane to afford the title product as an orange oil (7.54g, 60%).
LCMS *m*/*z =* 274.0 [M+H]⁺

### Preparation 6

### (R)-N-[(1R)-3-(1,3-dioxan-2-yl)-1-[5-fluoro-2-(methylsulfanyl)phenyl]propyl]-2-methylpropane-2-sulfinamide

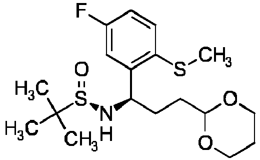

A solution (0.5 ml) of 2-(2-bromoethyl)-1,3-dioxolane (1.81 g, 10 mmol) in dry THF (5 ml) was added to a suspension of activated Mg turnings (729.0 mg, 30.0 mmol) under N₂ (g) in dry THF (10 ml) and the reaction warmed until Grignard formation had initiated. The remaining 2-(2-bromoethyl)-1,3-dioxolane solution (4.5 ml) was slowly added maintaining the temperature below 50°C. After complete addition, the reaction mixture was allowed to cool to rt, stirred for a further 1 h, then re-cooled to -50°C. A solution of (R)-N-[(1Z)-[5-fluoro-2-(methylsulfanyl)phenyl]methylidene]-2-methylpropane-2-sulfinamide

(Preparation 4, 270.0 mg, 1 mmol) in dry THF (5 ml) was added dropwise, the reaction stirred at -50°C for 1 h and then allowed to warm to rt. Saturated aq. NH₄Cl solution (20 ml) was added to quench the reaction and the mixture partitioned between EtOAc (30 ml) and water (30 ml). The aqueous layer was further extracted with EtOAc (30 ml) and the combined organic layers washed with brine (60 ml), dried (MgSO₄) and concentrated *in vacuo.* The crude product was purified by column chromatography on silica gel eluting with heptanes: EtOAc, (50:50 to 0:100) to afford the title compound as a colourless oil (420 mg, 100%).
LCMS *m*/*z* = 390.0 [M+H]⁺

### Preparation 7

### (R)-N-((R)-3-(1,3-dioxan-2-yl)-1-(3-fluoro-5-(methylthio)phenyl)propyl)-2-methylpropane-2-sulfinamide

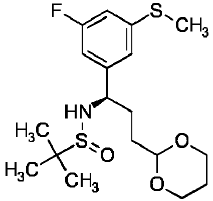

Mg turnings (2.67 g, 109.82 mmol) were stirred under N₂ at rt for 16 h. Anhydrous THF was added (20 ml) followed by iodine (15 mg, 59 µmοl), and the mixture heated up until iodine started to sublime. The mixture was allowed to cool to rt before 2ml of a solution (2 ml) of 2-(2-bromoethyl)-1,3-dioxolane (7.14 g, 36.6 mmol) in dry THF (25 ml) was added to the magnesium turning suspension. The mixture was gently warmed-up until the Grignard formation initiated and the remaining solution of 2-(2-bromoethyl)-1,3-dioxolane were added slowly while maintaining the temperature below 50°C. The Grignard solution was allowed to cool down to rt over 30 min and stirred for a further 1 h at rt. The mixture was cooled to -70°C and a solution of (R)-N-[(1Z)-[3-fluoro-5-(methylsulfanyl)phenyl]methylidene]-2-methylpropane-2-sulfinamide (Preparation 5, 1.0 g, 3.66 mmol) in dry THF (18 ml) was added, stirred at -50°C for 1 h and warmed up to rt for a further 1 h. The reaction mixture was quenched at 0°C with an aqueous solution of NH₄Cl (40 ml). The reaction mixture was partitioned between EtOAc and water (1:1, 200 ml). The layers were separated and the aqueous was extracted with EtOAc (100 ml). The combined organic layers were washed with brine (100 ml), dried over Na₂SO₄ and reduced to dryness. The crude yellow oil was dissolved in EtOAc (5 ml), loaded onto a Biotage^{®} ZIP KP-SIL 120 g column eluting with 50 to 100% EtOAc in heptane. The desired fractions were evaporated yielding the title compound as light yellow oil (1.41g, 99%).
LCMS *m*/*z* = 388.1 [M-H]⁺

### Preparation 8

### (2R)-2-[5-fluoro-2-(methylsulfanyl)phenyllpyrrolidine

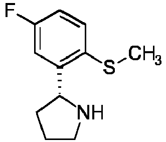

A solution of (R)-N-[(1R)-3-(1,3-dioxan-2-yl)-1-[5-fluoro-2-(methylsulfanyl)phenyl]propyl]-2-methylpropane-2-sulfinamide (Preparation 6, 390.0 mg, 1 mmol) in TFA:water (10 ml, 20:1) was stirred at rt for 30 min. Et₃SiH (1.16 g, 10 mmol) was added and the reaction stirred vigorously at rt for 16 h. The mixture was diluted with toluene (30 ml), concentrated *in vacuo* then azeotroped with toluene (2 × 30 ml). The residual oil was purified by column chromatography on silica gel eluting with (DCM:MeOH:NH₄OH, 98:2:0.2 to 95:5:0.5) to afford the title compound product as an oil (125 mg, 59%).
LCMS *m*/*z =* 212.0 [M+H]⁺

### Preparation 9

### (2R)-2-(3-Fluoro-5-(methylthio)phenyl)pyrrolidine

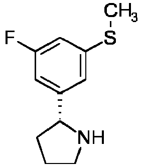

To a solution of (R)-N-((R)-3-(1,3-dioxan-2-yl)-1-(3-fluoro-5-(methylthio)phenyl)propyl)-2-methylpropane-2-sulfinamide (Preparation 7, 1.41 g, 3.62 mmol) in water (2 ml) was added TFA (36 ml). The resulting solution was stirred at rt for 30 min. Et₃SiH (4.21 g, 36.2 mmol) was then added and the biphasic solution was stirred vigorously at rt over 48h. The reaction mixture was concentrated *in vacuo.* The resulting crude was loaded onto a SCX cartridge in MeOH, the cartridge rinsed with 40 ml of MeOH and eluted with 7N NH₄OH in MeOH (50 ml).The collected fractions were evaporated affording the title compound as brown oil (0.65 g, 85%).
LCMS *m*/*z* = 212.0 [M+H]⁺

### Preparation 10

### Ethyl 6-[(2R)-2-[5-fluoro-2-(methylsulfanyl)phenyl]pyrrolidin-1-yl]imidazo[1,2-b]pyridazine-3-carboxylate

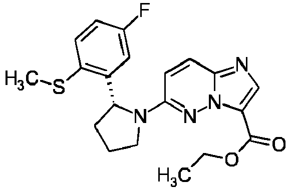

A solution of (2R)-2-[5-fluoro-2-(methylsulfanyl)phenyl]pyrrolidine (Preparation 8, 640 mg, 3.03 mmol) in 4M HCl in dioxane (20 ml) was stirred at rt for 30 min, then concentrated *in vacuo.* Ethyl 6-chloroimidazo[1,2-b]pyridazine-3-carboxylate (0.59 g, 2.52 mmol) in DMSO (20 ml) was added and the reaction heated at 130°C for 16 h. The cooled mixture was partitioned between water (20 ml) and EtOAc (20 ml), and the layers separated. The organic phase was washed with brine (3 × 20ml), dried (MgSO₄) and evaporated under reduced pressure to afford the title compound as a brown oil (1.13 g, 99%).
LCMS *m*/*z* 401.2 [M+H]⁺

### Preparation 11

### Ethyl 6-[(2R)-2-[3-fluoro-5-(methylsulfanyl)phenyl]pyrrolidin-1-yl]imidazo[1,2-b]pyridazine-3-carboxylate

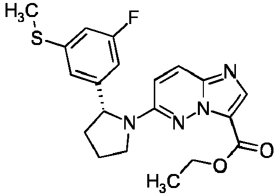

To (2R)-2-(3-fluoro-5-(methylthio)phenyl)pyrrolidine_(Preparation 9, 0.30mmol, 1.2 equiv.), ethyl 6-chloroimidazo[1,2-b]pyridazine-3-carboxylate (0.25mmol) and KF (2.8mmol, 11 equiv.) was added DMSO (2ml) and the reaction was heated at 130°C. After overnight reaction the reaction mixture was cooled to rt. Water (10ml) and EtOAc (10 ml) were added and the layers partitioned. The organic layer was washed three times with brine (10ml), dried over Na₂SO₄ and the solvent was removed under vacuum. The crude material containing the title compound was used without further purification.
LCMS *m*/*z* = 401.1 [M+H]⁺

### Preparation 12

### 6-{(2R)-2-[5-fluoro-2-(methylsulfanyl)phenyl]pyrrolidin-1-yl]imidazo[1,2-b]pyridazine-3-carboxylic acid

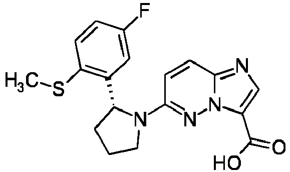

KOH (0.71 g, 12.6 mmol) was added portionwise to a solution of ethyl 6-[(2R)-2-[5-fluoro-2-(methylsulfanyl)phenyl]pyrrolidin-1-yl]imidazo[1,2-b]pyridazine-3-carboxylate (Preparation 10, 1.0 g, 2.52 mmol) in EtOH:water (12 ml, 6:1) and the reaction stirred at rt for 1.5 h. The mixture was concentrated *in vacuo,* the residue partitioned between water (20 ml) and DCM (20 ml) and the layers separated. The aqueous phase was adjusted to pH 4 with 2 M HCI solution, then extracted with DCM (3 × 20 ml). These combined organic layers were dried (MgSO₄) and concentrated *in vacuo* to give the title compound as a beige solid (999 mg, 99%).
LCMS *m*/*z* = 373.2 [M+H]⁺

### Preparation 13

### 6-[(2R)-2-[3-fluoro-5-(methylsulfanyl)phenyl]pyrrolidin-1-yl]imidazo[1,2-b]pyridazine-3-carboxylic acid

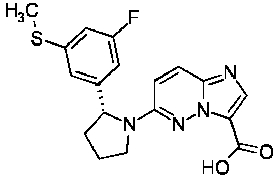

Water (0.15ml) and KOH (1.25mmol) were added to ethyl 6-[(2R)-2-[3-fluoro-5-(methylsulfanyl)phenyl]pyrrolidin-1-yl]imidazo[1,2-b]pyridazine-3-carboxylate (Preparation 11, 0.25mmol) in EtOH (1ml) under N₂. The reaction was stirred at rt. After 1 hour water (10ml) and DCM (10ml) were added to the reaction mixture and the pH was adjusted to 4. The layers were partitioned and the aqueous layer was extracted three times with 2-MeTHF (10ml). The combined organic layers were dried over Na₂SO₄ and the solvent was removed under vacuum to afford the title compound as a brown solid (98%, 91mg).
LCMS *m*/*z* = 373.1 [M+H]⁺

### Preparation 14

### tert-Butyl 3-{6-[(2R)-2-[5-fluoro-2-(methylsulfanyl)phenyl]pyrrolidin-1-yl]imidazo[1,2-b]pyridazine-3-amido}azetidine-1-carboxylate

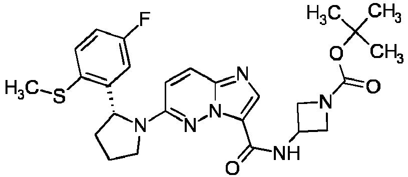

To a solution of 6-[(2R)-2-[5-fluoro-2-(methylsulfanyl)phenyl]pyrrolidin-1-yl]imidazo[1,2-b]pyridazine-3-carboxylic acid (Preparation 12, 150 mg, 0.4 mmol), HATU (183 mg, 0.48 mmol) and *tert*-butyl 3-aminoazetidine-1-carboxylate (76 mg, 0.44 mmol) in DMF (5 ml) was added DIPEA (0.14 ml, 0.81 mmol) and the reaction was stirred at rt for 72 h. The reaction was quenched with H₂O and diluted with MTBE the phases were separated and the aqueous extracted with MTBE, the combined organic layers were washed with H₂O (3 × 10 ml) and brine, dried (MgSO₄) and concentrated *in vacuo* to afford the title compound as a brown oil used without further purification (216 mg).

¹H NMR (CDCl₃, 396 MHz): δ: 8.22 (s, 1H), 7.70 (d, 1H), 7.22-7.31 (m, 1H *under CDCl3), 6.99-7.07 (m, 1H), 6.77 (m, 1H), 6.36 (d, 1H), 5.29 (d, 1H), 4.88 (m, 1H), 4.36 (m, 2H), 3.88-3.98 (m, 2H), 3.70-3.79 (m, 2H), 2.58 (s, 3H), 2.46-2.54 (m, 1H), 2.04-2.20 (m, 3H), 1.46 (d, 9H).

### Preparation 15

### tert-Butyl (3S)-3-{6-[(2R)-2-[5-fluoro-2-(methylsulfanyl)phenyl]pyrrolidin-1-yl]imidazo[1,2-blpyridazine-3-amido}pyrrolidine-1-carboxylate

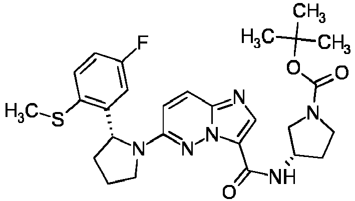

The title compound was prepared (216 mg, crude) from 6-[(2R)-2-[5-fluoro-2-(methylsulfanyl)phenyl]pyrrolidin-1-yl]imidazo[1,2-b]pyridazine-3-carboxylic acid (Preparation 12) and tert-butyl (S)-3-aminopyrrolidine-1-carboxylate following the procedure described in Preparation 14.
LCMS *m*/*z* = 541.2 [M+H]⁺

### Preparation 16

### tert-Butyl (3S)-3-{6-[(2R)-2-[3-fluoro-5-(methylsulfanyl)phenyl]pyrrolidin-1-yl]imidazo[1,2-b]pyridazine-3-amido}pyrrolidine-1-carboxylate

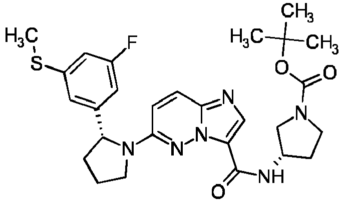

6-[(2R)-2-[3-fluoro-5-(methylsulfanyl)phenyl]pyrrolidin-1-yl]imidazo[1,2-b]pyridazine-3-carboxylic acid (Preparation 13, 91 mg, 0.24 mmol) and HATU (111 mg, 0.29 mmol) were dissolved in DMF (3 ml). *tert*-butyl (S)-3-aminopyrrolidine-1-carboxylate (51 mg, 0.27 mmol) was added followed by DIPEA (85 µL, 0.49 mmol). The reaction mixture was stirred for 3.5 h under N₂ and partitioned between water and MTBE. The aqueous phase was extracted with MTBE. The combined organic layers were washed with water, brine, dried over Na₂SO₄, and reduced to dryness under vacuum to afford the title compound which was used without further purification (117 mg, 90%).
LCMS *m*/*z* = 541.1[M+H]⁺

### Preparation 17

### N-(azetidin-3-yl)-6-[(2R)-2-[5-fluoro-2-(methylsulfanyl)phenyl]pyrrolidin-1-yl]imidazo[1,2-blpyridazine-3-carboxamide

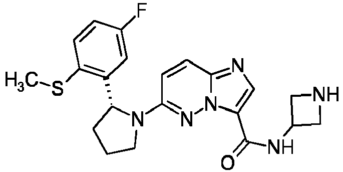

To a solution of *tert*-butyl 3-{6-[(2R)-2-[5-fluoro-2-(methylsulfanyl)phenyl]pyrrolidin-1-yl]imidazo[1,2-b]pyridazine-3-amido}azetidine-1-carboxylate (Preparation 14, 216 mg, 0.4 mmol) in DCM (2 ml) was added TFA (1 ml) and the reaction was stirred at rt for 30 min. The reaction was concentrated and azeotroped with DCM. The crude material was purified by SCX-ion exchange resin followed by normal phase chromatography 2-30 DCM/MeOH to afford the title compound (169 mg, 59%).
LCMS *m*/*z* = 427 [M+H]⁺

### Preparation 18

### 6-[(2R)-2-[5-fluoro-2-(methylsulfanyl)phenyl]pyrrolidin-1-yl]-N-[(3S)-pyrrolidin-3-yllimidazof 1,2-blpyridazine-3-carboxamide

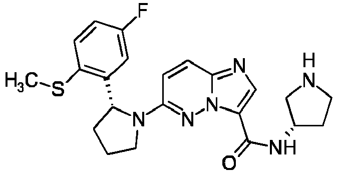

The following compound was prepared from *tert*-butyl (3S)-3-{6-[(2R)-2-[5-fluoro-2-(methylsulfanyl)phenyl]pyrrolidin-1-yl]imidazo[1,2-b]pyridazine-3-amido}pyrrolidine-1-carboxylate (Preparation 15) following the procedure described in Preparation 17, (140mg, 64%).
LCMS *m*/*z* = 441.3 [M+H]⁺

### Preparation 19

### 6-[(2R)-2-[3-fluoro-5-(methylsulfanyl)phenyl]pyrrolidin-1-yl]-N-(3S)-pyrrolidin-3-yl]imidazo[1,2-b]pyridazine-3-carboxamide

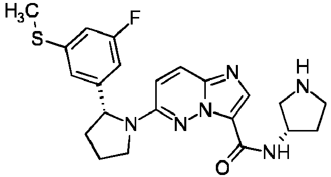

tert-Butyl (3S)-3-{6-[(2R)-2-[3-fluoro-5-(methylsulfanyl)phenyl]pyrrolidin-1-yl]imidazo[1,2-b]pyridazine-3-amido}pyrrolidine-1-carboxylate (Preparation 16, 117 mg, 0.22 mmol) was dissolved in DCM (1 ml) and TFA (2 ml) was added dropwise while stirring the mixture at rt. The reaction mixture was stirred at same temperature overnight. After removal of the solvent the residue was co-evaporated with DCM. The crude material was loaded on the SCX column and eluted first using MeOH and then ammonia 7.0 N in MeOH. The solvent was evaporated to give the title compound (70 mg, 72%).
LCMS *m*/*z* = 441.1[M+H]⁺

### Preparation 20

### 4-Fluoro-2-iodo-1-(methylsulfanyl)benzene

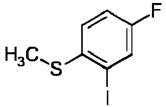

2-Bromo-4-fluoro-1-(methylsulfanyl)benzene (0.5 g, 2.26 mmol) was added dropwise to a suspension of activated Mg turnings (1.92 g, 79 mmol) under N₂ in dry THF (80 ml) and the reaction warmed until Grignard formation had initiated. The remaining 2-bromo-4-fluoro-1-(methylsulfanyl)benzene (17 g, 76.89 mmol) was added dropwise, so as to maintain the temperature below 50°C and after complete addition, the reaction was allowed to cool to rt and stirred for 16 h. The solution was added via cannula to an icecooled solution of iodine (24.11 g, 94.99 mmol) in dry THF (80 ml) maintaining the temperature below 10 °C. The reaction was stirred at 0°C for 1 h, at rt for 1 h, then poured into an ice-cold sat. NH₄Cl soln. (300 ml). The mixture was concentrated *in vacuo* to remove organic solvents then extracted with Et₂O (3 × 300 ml). The combined organic layers were washed with a sat. Na₂S₂O₃ solution, dried (Na₂SO₄), and concentrated *in vacuo* to afford the title compound as a brown oil (21.5 g, 83%).

¹H NMR (CDCl₃, 396 MHz): δ: 7.55 (m, 1H), 7.08-7.11 (m, 2H), 2.45 (s, 3H).

### Preparation 21

### 1-Fluoro-3-iodo-5-(methylsulfanyl)benzene

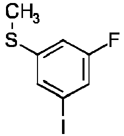

To a stirred solution of 1,3-difluoro-5-iodobenzene (50.0 g, 208.3 mmol) in DMF (250 ml) was added portionwise MeSNa (14.6 g, 208.3 mmol). The reaction was heated at 150°C for 1 h. Further MeSNa (1.5 g, 21 mmol) was added and the reaction was stirred at 150°C for a further 30 min. The reaction mixture was allowed to cool to rt before being diluted with distilled water (250 ml) and then extracted five times with MTBE (5 × 150 ml). The combined organic layers were then washed three times with brine (3 × 150 ml). The organic layer was dried (MgSO₄), filtered and concentrated *in vacuo* to afford a yellow oil (61 g). This oil was combined with 54 g batch from another synthesis to give a combined total of 115 g (429 mmol). This was purified by silica column chromatography (n-heptane) to afford a colourless oil (84.18 g, 76 %).

¹H NMR (CDCl₃; 400 MHz): δ: 7.31 (s, 1H), 7.18 (m, 1H), 6.88 (m, 1H), 2.45 (s, 3H).

### Preparation 22

### 1-tert-Butyl 2-(1,3-dioxo-2,3-dihydro-1H-isoindol-2-yl) (2S,4S)-4-fluoropyrrolidine-1,2-dicarboxylate

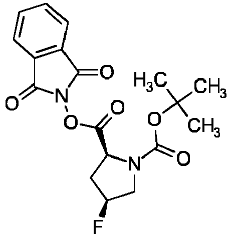

A solution of (2S,4S)-1-(tert-butoxycarbonyl)-4-fluoropyrrolidine-2-carboxylic acid (1.07 g, 4.6 mmol) in EtOAc (12.5 ml) was added to a stirred mixture of N-hydroxyphthalimide (0.75 g, 4.6 mmol) and N,N'-dicyclohexylcarbodiimide (0.95 g, 4.6 mmol) in EtOAc (12.5 ml) under N₂(g) and the reaction stirred at rt for 4 h. The mixture was filtered through a plug of silica, washed with EtOAc (50 ml) and the filtrate concentrated *in vacuo.* The resulting oil was re-dissolved in EtOAc (20 ml), washed with sat. aq. NaHCOs (4 × 30 ml) and the organic layer dried (MgSO₄), filtered and evaporated under reduced pressure to afford the title compound as a white solid (1.55 g, 89 %).
LCMC *m*/*z* = 278.9 [M-Boc]⁺

### Preparation 23

### tert-Butyl (2R,4S)-4-fluoro-2-f5-fluoro-2-(methylsulfanyl)phenyllpyrrolidine-1-carboxylate

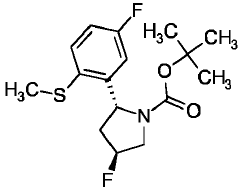

Dry N,N-dimethylacetamide (4 ml) was added to nickel dibromide glyme complex (0.09 g, 0.291 mmol) and 4,4'-di-tert-butyl-2,2'-bipyridine (0.08 g, 0.298 mmol) under N₂. The mixture was stirred for 15 min, then 4-fluoro-2-iodo-1-(methylsulfanyl)benzene (Preparation 20, 0.51 g, 1.49 mmol), 1-*tert*-butyl 2-(1,3-dioxo-2,3-dihydro-1H-isoindol-2-yl) (2S,4S)-4-fluoropyrrolidine-1,2-dicarboxylate (Preparation 22, 0.62 g, 1.64 mmol) and zinc dust (0.251 g, 3.84 mmol) were added and the reaction mixture was stirred at 28°C for 17 h. The mixture was filtered through a plug of silica and washed with diethyl ether (75 ml). The collected solution was extracted with brine (4 × 75 ml), the organic layers were combined, dried (MgSO₄), filtered and concentrated *in vacuo.* The residue was purified by column chromatography on silica gel eluting with heptanes:EtOAc, 100:0 to 90:10 to afford the title compound as a yellow oil (0.24 g, 36%).
LCMS *m*/*z* = 230.1 [M-Boc]⁺

### Preparation 24

### (2R,4S)-4-fluoro-2-f5-fluoro-2-(methylsulfanyl)phenyllpyrrolidine

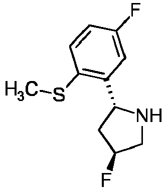

To a solution of *tert-butyl* (2R,4S)-4-fluoro-2-[5-fluoro-2-(methylsulfanyl)phenyl]pyrrolidine-1-carboxylate (Preparation 23, 1.21 g, 3.67 mmol) in MeOH (15 ml) was added HCl (4M solution in dioxane, 10 ml) and the mixture was stirred at rt for 2 h. The mixture was concentrated *in vacuo* to afford a dark brown oil which was taken up in MeOH (2 ml) and loaded onto an SCX cartridge and flushed with 7N ammonium hydroxyde in MeOH. The combined solution was concentrated *in vacuo* to afford the title compound as a dark orange oil (0.4 g, 53%).
LCMS *m*/*z* = 230.0 [M+H]⁺

### Preparation 25

### (2R,4S)-4-fluoro-2-[3-fluoro-5-(methylsulfanyl)phenyl]pyrrolidine

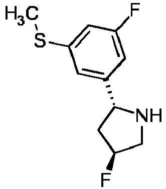

NiBr2(glyme) (14.33 g, 46.43 mmol, 15 mol %) was added to 4,4'-di-tert-butyl-2,2'-bipyridine (12.46 g, 46.43 mmol, 15 mol %). N₂ was flushed through the flask for 15 min before the addition of dry N,N-dimethylacetamide (325 ml). The mixture was stirred under N₂ for 15 min. 1-Fluoro-3-iodo-5-(methylsulfanyl)benzene (Preparation 21, 82.98 g, 309.5 mmol), 1-tert-butyl 2-(1,3-dioxo-2,3-dihydro-1H-isoindol-2-yl) (2S,4S)-4-fluoropyrrolidine-1,2-dicarboxylate (Preparation 22, 175.67 g, 464.3 mmol) and zinc dust (40.47 g, 619.1 mmol) were added. The temperature was controlled to maintain the internal temperature below 40 °C. The reaction mixture was stirred at 28°C for 17 h. The reaction mixture was filtered through a plug of silica and washed three times with MTBE (3 × 200 ml). The filtrate was washed with brine (2 × 500 ml), then with 2 M KOH (5 × 300 ml) and then finally with distilled water (500 ml). The organic layer was collected, dried (MgSO₄), filtered and concentrated *in vacuo* to afford a dark orange/brown residue (132 g). This residue was purified by silica column chromatography (on 900 g of silica) eluting from neat n-heptane to 30 % MTBE in n-heptane to afford tert-butyl (2R,4S)-4-fluoro-2-[3-fluoro-5-(methylsulfanyl)phenyl]pyrrolidine-1-carboxylate (42.40 g, 70 % purity by ¹H NMR) as a light yellow oil, which is contaminated with with tert-butyl (S)-3-fluoropyrrolidine-1-carboxylate (30 % by ¹H NMR).

The light yellow oil was dissolved in MeOH (64 ml) at rt under N₂. To this at 0°C was added portionwise 4 M HCl in 1,4-dioxane (130 ml, 510 mmol). After 1 h the reaction mixture was concentrated *in vacuo* and taken up in distilled water (400 ml). This aqueous mixture (pH 2) was extracted twice with MTBE (2 × 250 ml) before being adjusted to pH 10 with solid NaOH. The basic aqueous layer (pH 10) was extracted four times with DCM (4 × 250 ml). The combined organic layers were dried (Na₂SO₄), filtered and concentrated *in vacuo* to afford the title compound as a reddish/brown oil (20.49 g, 89.4 mmol).
LCMS *m*/*z* = 230 [M+H]⁺

The title compound (20.49 g, 89.4 mmol, 1 eq.) was dissolved in dry 1,4-dioxane (45 ml) under N₂ and cooled to 0 °C. To this stirred solution was added portionwise 4 M HCl in 1,4-dioxane (33.5 ml, 134 mmol, 1.5 eq.) and the mixture was stirred at this temperature for 15 min before being concentrated *in vacuo* to afford the title compound as hydrochloride salt which was used without further purification (23.75 g, 29 % overall).

### Preparation 26

### Ethyl 6-(2R,4S)-4-fluoro-2-[5-fluoro-2-(methylsulfanyl)phenyl]pyrrolidin-1-yl]imidazo[1,2-b]pyridazine-3-carboxylate

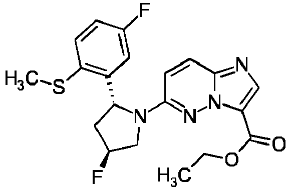

Ethyl 6-chloroimidazo[1,2-b]pyridazine-3-carboxylate (89 mg, 0.40 mmol), KF (253 mg, 4.36 mmol) and (2R,4S)-4-fluoro-2-[5-fluoro-2-(methylsulfanyl)phenyl]pyrrolidine (Preparation 24, 100 mg, 0.38 mmol) were suspended in dry degassed DMSO (5 ml). The reaction was heated at 130°C for 20 h. The mixture was diluted with EtOAc (20 ml), washed with water (15 ml) and brine (2 × 15 ml), dried (Na₂SO₄), filtered and evaporated to dryness to afford the title compound as a light yellow solid (174 mg, 85%).
LCMS *m*/*z* = 419.0 [M+H]⁺

### Preparation 27

### Ethyl 6-(2R,4S)-4-fluoro-2-[3-fluoro-5-(methylsulfanyl)phenyl]pyrrolidin-1-yl]imidazo[1,2-b]pyridazine-3-carboxylate

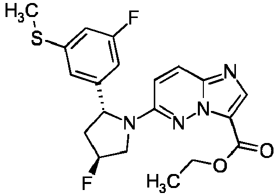

(2R,4S)-4-Fluoro-2-[3-fluoro-5-(methylsulfanyl)phenyl]pyrrolidine hydrochloride (Preparation 25, 23.75 g, 89.4 mmol), KF (46.73 g, 804.3 mmol), ethyl 6-chloroimidazo[1,2-b]pyridazine-3-carboxylate (19.76 g, 87.6 mmol) were suspended in DMSO (350 ml). The reaction was heated at 130°C for 82 h. The reaction mixture was allowed to cool to rt before being poured into distilled water (750 ml), which precipitated a beige solid. The liquid was decanted and the solid was washed with distilled water three times (3 × 250 ml). The beige solid was taken up in MTBE (500 ml) and partitioned between sat. aq. NH₄Cl (500 ml) and then finally brine (500 ml). The organic layer was dried (Na₂SO₄), filtered and concentrated *in vacuo* to afford a dark reddish residue which was used without further purification (30.52 g, 82 %).
LCMS *m*/*z* = 419 [M+H]⁺

### Preparation 28

### 6-[(2R,4S)-4-fluoro-2-[5-fluoro-2-(methylsulfanyl)phenyl]pyrrolidin-1-yl]imidazo[1,2-b]pyridazine-3-carboxylic acid

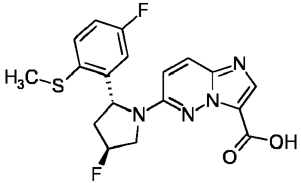

To a solution of ethyl 6-[(2R,4S)-4-fluoro-2-[5-fluoro-2-(methylsulfanyl)phenyl]pyrrolidin-1-yl]imidazo[1,2-b]pyridazine-3-carboxylate (Preparation 26, 174 mg, 0.42 mmol) in EtOH (897 µL) and water (143 µL) was added KOH (117 mg, 2.08 mmol). The reaction was stirred at rt for 15 min, then diluted with water (20 ml) and washed with EtOAc (2 × 20 ml). The water was acidified to pH 4, then extracted with EtOAc (3 × 20 ml) and concentrated *in vacuo* to afford the title compound as a light yellow vitreous solid (94 mg, 50%).
LCMS *m*/*z* = 391 [M+H]⁺

### Preparation 29

### 6-[(2R,4S)-4-fluoro-2-[3-fluoro-5-(methylsulfanyl)phenyl]pyrrolidin-1-yl]imidazo[1,2-blpyridazine-3-carboxylic acid

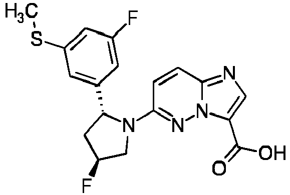

Ethyl 6-[(2R,4S)-4-fluoro-2-[3-fluoro-5-(methylsulfanyl)phenyl]pyrrolidin-1-yl]imidazo[1,2-b]pyridazine -3-carboxylate (Preparation 27, 30.52 g, 72.9 mmol) was dissolved in EtOH (159 ml) at rt. To this stirred mixture was added portionwise a solution of NaOH (14.59 g, 364.7 mmol) in water (26 ml) at rt. After 3 h, EtOH was removed *in vacuo* and the resulting residue was dissolved in distilled water (500 ml). This was extracted with MTBE (2 × 250 ml). The basic aqueous solution was acidified to pH 5 by the dropwise addition of conc. HCl and then was extracted with EtOAc (5 × 250 ml). The combined organic layers were dried (MgSO4), filtered and concentrated *in vacuo* to give a beige solid (24.65 g, 87%).
LCMS *m*/*z* = 391 [M+H]⁺

### Preparation 30

### tert-Butyl (3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidine-1-carboxylate

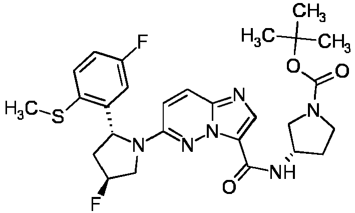

6-[(2R,4S)-4-Fluoro-2-[5-fluoro-2-(methylsulfanyl)phenyl]pyrrolidin-1-yl]imidazo[1,2-b]pyridazine-3-carboxylic acid (Preparation 28, 0.30 g, 0.768 mmol) was dissolved in anhydrous DCM (5.49 ml) followed by TPTU (0.273 g, 0.922 mmol) and left to stir under N₂ for 10 min. (S)-(-)-1-Boc-3-aminopyrrolidine (0.157 g, 0.845 mmol) was added and left to stir for a further 20 min at rt before DIPEA (0.267 ml, 1.54 mmol) was added. The resulting solution was stirred at rt under N2 for 1 hour. The reaction was diluted with DCM (20 ml) and water (15 ml), the organic layer was further washed with sat. NH₄Cl (3 × 15 ml) and with brine (15 ml). The organic layer was dried with MgSO₄ filtered and the solvent removed under reduced pressure to afford the title compound as a pale yellow solid which was used in the next step without further purification (0.463 g, >99 %).
LCMS *m*/*z =* 559.1 [M+H]⁺

### Preparation 31

### tert-Butyl (3S)-3-{6-r(2R,4S)-4-fluoro-2-[3-fluoro-5-(methylsulfanyl)phenyllpyrrolidin-1-yl]imidazo[1,2-b]pyridazine-3-amido}pyrrolidine-1-carboxylate

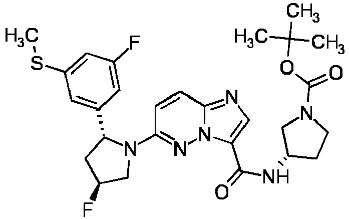

6-[(2R,4S)-4-Fluoro-2-[3-fluoro-5-(methylsulfanyl)phenyl]pyrrolidin-1-yl]imidazo[1,2-b]pyridazine-3-carboxylic acid (Preparation 29, 600 mg, 1.54 mmol) was dissolved in DMF (15.84 ml) and stirred at rt under N₂. HATU (0.699 g, 1.84 mmol) was added in one portion and the solution was stirred for 10 min at rt. *tert*-Butyl (S)-3-aminopyrrolidine-1-carboxylate (0.314 g, 1.69 mmol) was added and the solution stirred for 20 min before DIPEA (0.53 ml, 3.09 mmol) was added. The mixture was stirred at rt for 16 h. The reaction mixture was diluted with EtOAc (50 ml) and washed with brine (3 × 30 ml). The combined organic layers were dried (Na₂SO₄), filtered and concentrated *in vacuo* to give the title compound as a yellow oil which was used without further purification (1.42 g).

### LCMS m/z = 559.2 [M+H]⁺

The following compounds were prepared from 6-[(2R,4S)-4-fluoro-2-[3-fluoro-5-(methylsulfanyl)phenyl]pyrrolidin-1-yl]imidazo[1,2-b]pyridazine-3-carboxylic acid (Preparation 29) and appropriate amines according to the procedure described in Preparation 31.

| Preparation No | Name and Structure | Starting Material, Yield and Data |
|---|---|---|
| 32 | 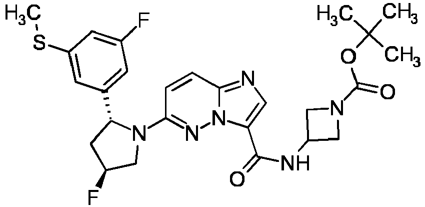 *tert*-butyl 3-{6-[(2R,4S)-4-fluoro-2-[3-fluoro-5-(methylsulfanyl)phenyl]pyrrolidin-1-yl]imidazo[1,2-b]pyridazine-3-amido}azetidine-1-carboxylate | Amine: *tert*-butyl 3-aminoazetidine-1-carboxylate 480 mg, 98%. |
| | | LCMS *m*/*z* = 545 [M+H]⁺ |
| 33 | 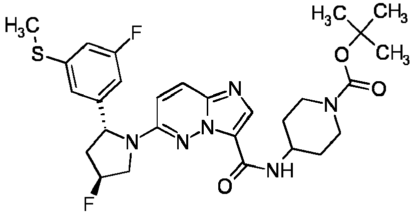 *tert*-butyl 4-{6-[(2R,4S)-4-fluoro-2-[3-fluoro-5-(methylsulfanyl)phenyl]pyrrolidin-1-yl]imidazo[1,2-b]pyridazine-3-amido}piperidine-1-carboxylate | Amine: *tert*-butyl 4-aminopiperidine-1-carboxylate 167 mg, 78%. |
| | | LCMS *m*/*z* = 571.2 [M-H]⁺ |
| 34 | 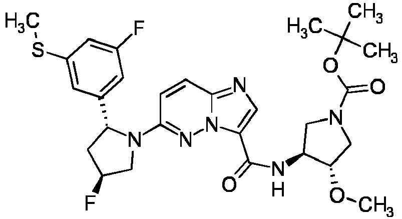 *tert-butyl* (3S,4S)-3-{6-[(2R,4S)-4-fluoro-2-[3-fluoro-5-(methylsulfanyl)phenyl]pyrrolidin-1-yl]imidazo[1,2-b]pyridazine-3-amido}-4-methoxypyrrolidine-1-carboxylate | Amine: *tert-butyl* (3S,4S)-3-amino-4-methoxypyrrolidine-1-carboxylate 94.6 mg, 78%. |
| | | LCMS *m*/*z* = 589.2 [M+H]⁺ |
| 35 | 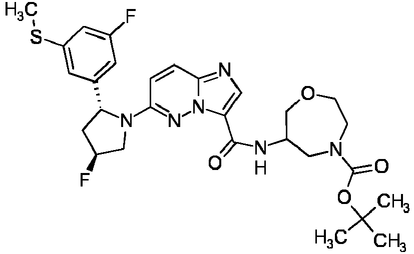 *tert*-butyl 6-{6-[(2R,4S)-4-fluoro-2-[3-fluoro-5-(methylsulfanyl)phenyl]pyrrolidin-1-yl]imidazo[1,2-b]pyridazine-3-amido}-1,4-oxazepane-4-carboxylate | Amine: *tert*-butyl 6-amino-1,4-oxazepane-4-carboxylate 230 mg, 73%. |
| | | LCMS *m*/*z* = 589.3 [M+H]⁺ |

### Preparation 36

### 6-[(2R,4S)-4-fluoro-2-[5-fluoro-2-(methylsulfanyl)phenyl]pyrrolidin-1-yl]-N-(3S)-pyrrolidin-3-yl]imidazo[1,2-b]pyridazine-3-carboxamide

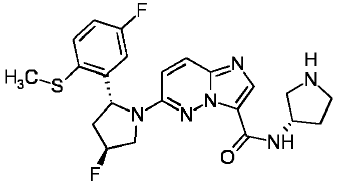

*tert*-Butyl (3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidine-1-carboxylate (Preparation 30, 0.429 g, 0.768 mmol) was dissolved in DCM (5.91 ml) at rt and TFA (1.69 ml, 14.86 mmol) was added dropwise. The mixture was allowed to stir at rt for 90 min. The mixture was concentrated *in vacuo,* then DCM (30 ml) was added before being concentrated *in vacuo* again. This was repeated (2 × 30 ml of DCM). The crude residue was loaded onto an SCX cartridge (10 g, pre-washed with 3 CVs MeOH), washed with 4 CVs MeOH and then finally eluted with 5 CVs of 2M NH₃ in MeOH. The relevant fractions were combined and concentrated *in vacuo* to afford the title compound as a yellow oil which was used without further purification (0.351 g, quantitative).
LCMS *m*/*z* = 459.0 [M+H]⁺

### Preparation 37

### 6-[(2R,4S)-4-fluoro-2-[3-fluoro-5-(methylsulfanyl)phenyl]pyrrolidin-1-yl]-N-(3S)-pyrrolidin-3-yl]imidazo[1,2-b]pyridazine-3-carboxamide

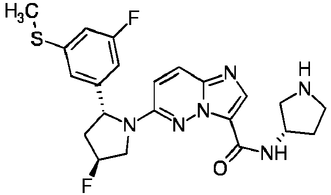

*tert*-Butyl (3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidine-1-carboxylate (Preparation 31, 0.858 g, 1.54 mmol) was dissolved in DCM (11.81 ml) at rt and TFA (2.27 ml, 29.7 mmol, 19.35 eq.) was added dropwise. The reaction was allowed to stir at rt for 90 min. The reaction was concentrated *in vacuo* and then co-evaporated with DCM (3 × 10 ml). The crude residue was loaded onto an SCX cartridge (5g, pre-washed with 3 CVs MeOH), washed with 4 CVs MeOH and then finally eluted with 5 CVs of 2M NH₃ in MeOH. The relevant fractions were combined and concentrated *in vacuo* to afford the title compound as a yellow oil (0.722 g, quantitative).
LCMS *m*/*z =* 459.2 [M+H]⁺

The following compounds were prepared from the appropriate protected amines according to the procedure described in Preparation 37.

| Preparation No | Structure and Name | Starting Material, Yield and Data |
|---|---|---|
| 38 | 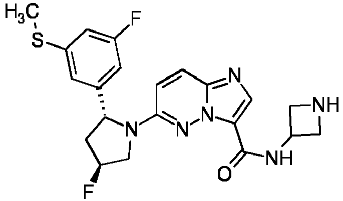 N-(azetidin-3-yl)-6-[(2R,4S)-4-fluoro-2-[3-fluoro-5-(methylsulfanyl)phenyl]pyrrolidin-1-yl]imidazo[1,2-b]pyridazine-3-carboxamide | Amine: Preparation 32 0.355g, 89 %. |
| | | LCMS *m*/*z* = 445.0 [M+H]⁺ |
| 39 | 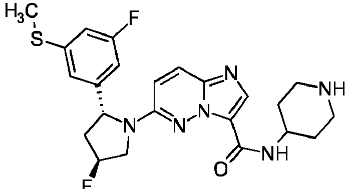 6-[(2R,4S)-4-fluoro-2-[3-fluoro-5-(methylsulfanyl)phenyl]pyrrolidin-1-yl]-N-(piperidin-4-yl)imidazo[1,2-b]pyridazine-3-carboxamide | Amine: Preparation 33 87 mg, 79%. |
| | | LCMS *m*/*z* = 473.2 [M+H]⁺ |
| 40 | 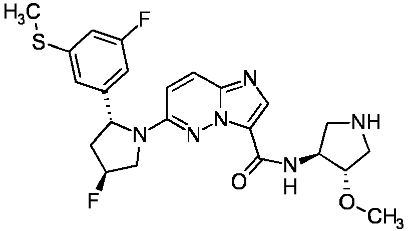 6-[(2R,4S)-4-fluoro-2-[3-fluoro-5-(methylsulfanyl)phenyl]pyrrolidin-1-yl]-N-[(3S,4S)-4-methoxypyrrolidin-3-yl]imidazo[1,2-b]pyridazine-3-carboxamide | Amine: Preparation 34 0.0712 g, 91%. |
| | | LCMS *m*/*z* = 489.2 [M+H]⁺ |
| 41* | 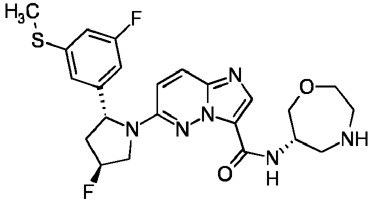 6-[(2R,4S)-4-fluoro-2-[3-fluoro-5-(methylsulfanyl)phenyl]pyrrolidin-1-yl]-N-[(6S)-1,4-oxazepan-6-yl]imidazo[1,2-b]pyridazine-3-carboxamide* | Amine: Preparation 34 |
| | | 44 mg (41) and 17 mg (42), 37% overall. |
| | | Isomer (41): LCMS *m*/*z* = 489.1 [M+H]⁺ |
| | | Isomer (42): LCMS *m*/*z* = 489.1 [M+H]⁺ |
| | | Boc deprotection was carried out using formic acid instead of trifluoroacetic acid in DCM. |
| 42* | 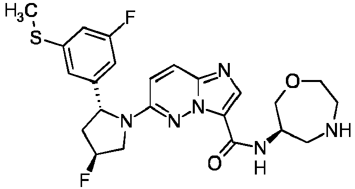 6-[(2R,4S)-4-fluoro-2-[3-fluoro-5-(methylsulfanyl)phenyl]pyrrolidin-1-yl]-N-[(6R)-1,4-oxazepan-6-yl]imidazo[1,2-b]pyridazine-3-carboxamide* | |
| | | *Stereochemistry arbitrarily assigned. |

### Preparation 43

### 6-[(2S,4S)-4-fluoro-2-[5-fluoro-2-(methylsulfanyl)phenyl]cyclopentyl]imidazo[1,2-b]pyridazine-3-carbonyl chloride

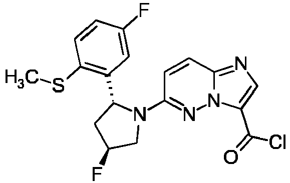

To a suspension of 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 28, 1.0 g, 2.57 mmol) in DCM (50 ml) was added oxalyl chloride (390 mg, 0.264 ml, 3.08 mmol) followed by 1 drop of DMF. Gas evolution was observed and internal temperature increased from 20°C to 21 °C. The reaction was left to stir at rt for 30 min and then reduced to dryness to yield the title compound as pale yellow foam which was used without further purification.

### Preparation 44

### 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoic acid

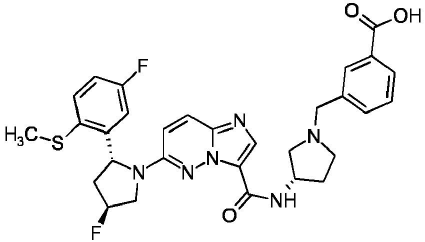

To a solution of methyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate (Example 10, 190 mg, 0.313 mmol, 1 equiv.) in MeOH (0.69 ml) was added a solution of NaOH (62 mg, 1.57 mmol, 5.0 equiv.) in water (0.46 ml) at rt. The mixture was stirred for 1 hour, then a further solution of NaOH (5 equiv.) in water (0.46 ml) added and the reaction mixture stirred at rt for 16 h. The MeOH was removed *in vacuo,* the reaction mixture diluted with water (15 ml) and the pH adjusted with 2M HCl to pH 4. The mixture was extracted with EtOAc (2 × 20 ml). The combined organic layers were dried (Na₂SO₄), filtered and concentrated *in vacuo* to afford the title compound as a white solid (166 mg, 89%).
LCMS *m*/*z* = 593 [M+H]⁺

### Preparation 45

### tert-Butyl N-{1-[(3-hydroxyphenyl)methyllpiperidin-4-yl}carbamate

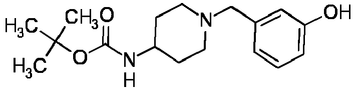

To *tert*-butyl N-(piperidin-4-yl)carbamate (8.19 g, 40.9 mmol) and 3-hydroxybenzaldehyde (5.00 g, 40.9 mmol) in DCM (100 ml) at rt was added acetic acid (3.79 ml, 49.1 mmol, 1.1 eq.) and the reaction mixture stirred for 15 min before adding sodium triacetoxyborohydride (17.35 g, 81.80 mmol) portionwise. The resulting mixture was stirred at rt for 18 h. The reaction mixture was diluted with DCM (250 ml) and the organic layer was washed with water (2 × 250 ml). The aqueous layer was basified to pH 5 by careful addition of sodium hydrogen carbonate. The aqueous was then extracted using MTBE/EtOAc (3 × 300 ml). The combined organic layers were dried (MgSO₄), filtered and concentrated *in vacuo* to give the title compound as a colourless solid (9.0 g, 72%).

¹H NMR (CDCl₃; 400 MHz): δ: 7.18 (m, 1H); 7.08 (bs, 1H); 6.94-6.75 (m, 2H); 4.60 (m, 1H); 3.71 (s, 2H); 3.61-3.33 (m, 1H); 3.20-2.90 (m, 2H); 2.48-2.18 (m, 2H); 2.18-1.87 (m, 2H); 1.87-1.57(m, 2H); 1.52-1.34 (m, 10H).

The following compounds were prepared from the appropriate amines and aldehydes according to the procedure described in Preparation 45.

| Preparation No | Structure and Name | Starting Materials, Yield and Data |
|---|---|---|
| 46 | 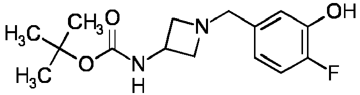 tert-butyl N-{1-[(4-fluoro-3-hydroxyphenyl)methyl]azetidin-3-yl)carbamate | *tert-butyl* N-(azetidine-4-yl)carbamate, 4-fluoro-3-hydroxybenzaldehyde. 329 mg, 97%, colourless solid. |
| | | LCMS *m*/*z* = 241.0 [M-tBu]⁺ |
| 47 | 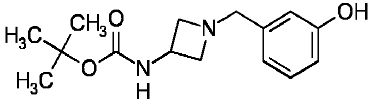 *tert-butyl* N-{1-[(3-hydroxyphenyl)methyl]azetidin-3-yl}carbamate | *tert-butyl* N-(azetidine-4-yl)carbamate; 3-hydroxybenzaldehyde. |
| | | 311 mg, 96%, white solid. |
| | | LCMS *m*/*z* = 223.1 [M-tBu]⁺ |
| 48 | 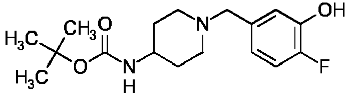 *tert*-butyl N-{1-[(4-fluoro-3-hydroxyphenyl)methyl]piperidin-4-yl}carbamate | *tert*-butyl N-(piperidin-4-yl)carbamate, 4-fluoro-3-hydroxybenzaldehyde. 6.6 g, 76%. |
| | | ¹H-NMR (MeOD-d₄; 396 MHz): δ: 6.95 (m, 1H), 6.88 (m, 1H), 6.75-6.67 (m, 1H), 3.39 (s, 2H), 2.82 (m, 2H), 2.07 (m, 2H), 1.81 (m, 2H), 1.51-1.36 (m, 12H). |

### Preparation 49

### tert-butyl N-[(3S)-1-[(3-hydroxyphenyl)methyl]pyrrolidin-3-yl]carbamate

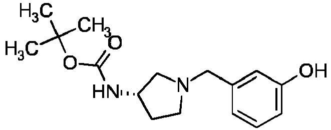

To *tert*-butyl N-[(3S)-pyrrolidin-3-yl]carbamate (20.0 g, 107 mmol, 1 eq.) and 3-hydroxybenzaldehyde (13.1 g, 107 mmol, 1 eq.) in DCM (250 ml) at rt was added acetic acid (6.76 ml, 118 mmol, 1.1 eq.) and the reaction mixture stirred for 1 hour before adding portionwise sodium triacetoxyborohydride (45.5 g, 215 mmol, 2 eq.). The resulting mixture was stirred at room for 4 h. The reaction mixture was carefully poured into a beaker of sat. aq. NaHCO₃ at 0°C and stirred overnight. The layers were separated and the aqueous was extracted with further DCM (250 ml). The combined organic layers were dried (MgSO₄), filtered and concentrated *in vacuo,* then azeotroped with TBME to give the title compound as a clear glass solid (33.60 g, quantitative).
LCMS m/z = 293 [M+H]⁺

### Preparation 50

### 3-((4-Aminopiperidin-1-yl)methyl)phenol hydrochloride

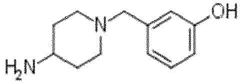

To a solution of *tert*-butyl N-{1-[(3-hydroxyphenyl)methyl]piperidin-4-yl}carbamate (Preparation 45, 9.00 g, 29.40 mmol, 1 eq.) in MeOH (75 ml) was added 4 M HCl in 1,4-dioxane (37 ml, 146.9 mmol, 9 eq.) at rt under N₂ and the resulting mixture was stirred for 18 h. The reaction mixture was concentrated *in vacuo* and triturated with TBME to give the title compound as a white hydrochloride salt (7.00 g, quantitative). *Note*: this material is hygroscopic and should be stored under N₂.

¹H NMR (MeOD-d₄; 400MHz): δ: 7.34-7.19 (m, 1H); 7.02-6.93 (m, 2H); 6.92-6.79 (m, 1H); 4.23 (s, 2H); 3.69-3.36 (m, 3H); 3.15 (bs, 2H); 2.21 (bs, 2H); 1.99 (bs, 2H).

The following compounds were prepared from the appropriate protected amines according to the procedure described in Preparation 50.

| **Preparation No** | **Structure and Name** | **Starting material, Yield & Data** |
|---|---|---|
| **51** | 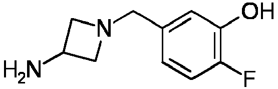 5-[(3-aminoazetidin-1-yl)methyl]-2-fluorophenol hydrochloride | Amine: Preparation 46 |
| | | 300 mg, 97%, yellow solid. |
| | | ¹H-NMR (MeOD-d₄; 396 MHz) δ: 7.18-7.10 (m, 2H), 6.99-6.91 (m, 1H), 4.47-4.32 (m, 5H), 3.66 (s, 2H). |
| **52** | 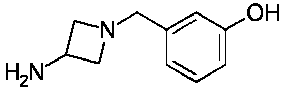 3-[(3-aminoazetidin-1-yl)methyl]phenol hydrochloride | Amine: Preparation 47 |
| | | 267 mg, 99%, white solid. |
| | | ¹H-NMR (MeOD-d₄; 396 MHz): δ: 7.29 (m, 1H), 6.96-6.88 (m, 3H), 4.45-4.35 (m, 7H). |
| **53** | 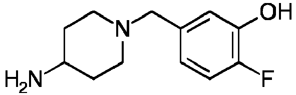 5-[(4-aminopiperidin-1-yl)methyl]-2-fluorophenol hydrochloride | Amine: Preparation 48 |
| | | 5.5 g, quantitative, off white solid. |
| | | ¹H-NMR (MeOD-d₄; 396 MHz): δ: 7.19-7.08 (m, 2H), 7.01-6.92 (m, 1H), 4.22 (s, 2H), 3.55 (d, 3H), 3.12 (m, 2H), 2.23 (m, 2H), 1.98 (m, 2H). |

### Preparation 54

### 3-{[(3S)-3-aminopyrrolidin-1-yi]methyl}phenol hydrochloride

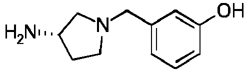

To the title compound of Preparation 49 (33.60 g, 115 mmol) at rt under N₂ was added 4 M HCl in 1,4-dioxane (259 ml, 1.03 mol) and the resulting mixture was stirred for 2 h. The reaction mixture was concentrated *in vacuo* and azeoptroped with TBME (2 × 100 ml) and DCM (100 ml) to give the title compound as a white solid (31.20 g, quantitative). *Note*: this material is hygroscopic and should be stored under N₂).
LCMS *m*/*z* = 193 [M+H]⁺

### Preparation 55

### tert- -butyl N-f(4-fluoro-3-hydroxyphenyl)methyllcarbamate

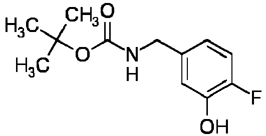

To 5-(aminomethyl)-2-fluorophenol (500 mg, 3.54 mmol) in THF (7 ml) at rt were added sodium hydrogen carbonate (893 mg, 10.63 mmol) in water (7 ml) and di-tert-butyl dicarbonate (850 mg, 3.90 mmol) and the resulting mixture was stirred at rt for 2 h. The reaction mixture was diluted with EtOAc (15 ml), washed with brine (2 × 15 ml), dried (Na₂SO₄), filtered and then concentrated *in vacuo.* The residue was purified on the Biotage^{®} with a ZIP KP-Sil 80 g column (10 % EtOAc in n-heptane for 1 CV and then 10-80 % EtOAc in n-heptane over 10 CVs) to afford the title compound as an amber oil (294 mg, 34 %).
LCMS *m*/*z* = 240.0 [M-H]⁺

### Preparation 56

### tert-Butyl N-({3-[(tert-butyldimethylsilyl)oxy]-4-fluorophenyl}methyl)carbamate

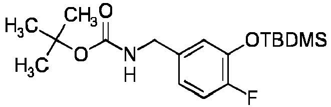

To a solution of *tert-* -butyl N-[(4-fluoro-3-hydroxyphenyl)methyl]carbamate (Preparation 55, 294 mg, 1.22 mmol) in DMF was added at rt TBDMSCI (276 mg, 1.83 mmol) and imidazole (125 mg, 1.83 mmol). The reaction mixture was stirred for 2 h, then partitioned between water (20 ml) and EtOAc (20 ml). The organic layer was washed with water (3 × 20 ml), saturated brine solution (20 ml), dried over Na₂SO₄ and concentrated *in vacuo* to afford the title compound as an amber oil (433 mg, quantitative).
LCMS *m*/*z* = 240 [M-TBDMS]⁺

### Preparation 57

### 1-{3-[(tert-butyldimethylsilyl)oxy]-4-fluorophenyl}methanamine

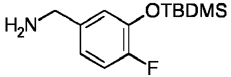

To a solution of *tert-butyl* N-({3-[(tert-butyldimethylsilyl)oxy]-4-fluorophenyl}methyl)carbamate (Preparation 56, 433 mg, 1.22 mmol) in DCM (2.4 ml), cooled to 0°C, was added TFA (2.4 ml) dropwise and the reaction mixture stirred at 0°C for 3 h before partitioning between DCM (20 ml) and water (20 ml). The pH was adjusted with NaOH to -pH 11, the layers separated and the aqueous layer extracted with 20 ml DCM. The combined organic layers were dried over Na₂SO₄ and concentrated *in vacuo* to afford the title compound as a brown oil (320 mg, quantitative).

¹H-NMR (CDCl₃; 396 MHz): 6.99 (m, 1H); 6.86 (m, 1H); 6.84-6.80 (m, 1H); 3.77 (s, 2H); 1.53 (br s, 2H); 1.00 (s, 9H); 0.19 (s, 3H); 0.18 (s, 3H).

## Claims

1. A compound of Formula (I): 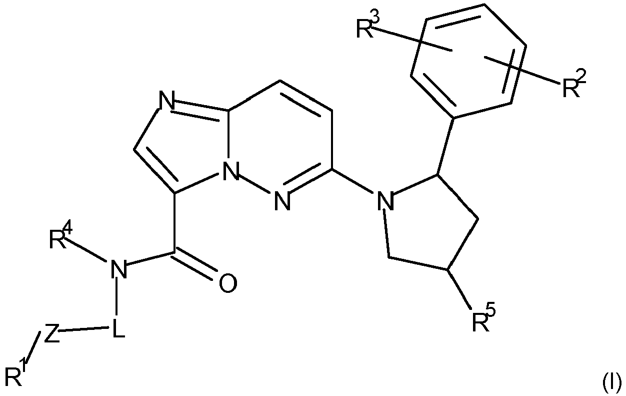 or a pharmaceutically acceptable salt or solvate thereof;
wherein:
L is (CR⁶R⁷)ᵣ;
Z is absent or selected from:
i) 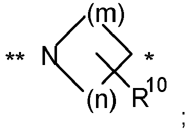 and
ii) 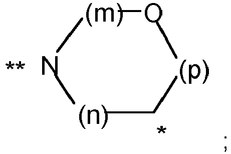
wherein * denotes the point of attachment to L and ** denotes the point of attachment to R1;
m is 1 or 2;
n is 1 or 2;
p is 0 or 1;
provided that the sum of m, n and p is in the range of 2 to 4;
r is 0 or 1;
R¹ is -XR⁹;
X is selected from -CH₂-, -C(O)-, and -S(O₂)-;
R² is -SR⁸;
R³ is selected from H and halo;
R⁴ is selected from H and (C₁-C₃)alkyl;
R⁵ is selected from H, hydroxyl and halo;
R⁶ and R⁷ are each independently selected from H and (C₁-C₃)alkyl;
R⁸ is methyl;
R⁹ is phenyl substituted by a group selected from hydroxy, -OC(O)(C₁-C₆)alkyl, C(O)OH and -C(O)O(C₁-C₆)alkyl, wherein the phenyl ring is optionally further substituted by halo;
R¹⁰ is selected from H and (C₁-C₃)alkoxy;
and wherein the compound is not 6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboxamide:

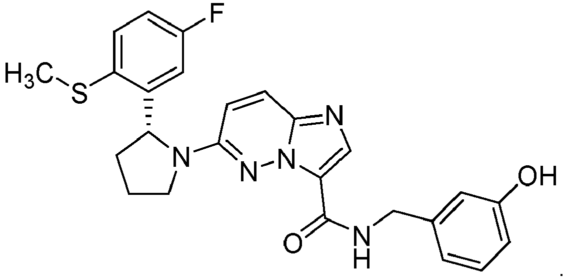

2. A compound according to claim 1 wherein
R¹ is -CH₂R⁹;
R³ is H or fluoro;
R⁴ is H;
R⁵ is H or fluoro;
R⁶ is H;
R⁷ is H;
R⁹ is phenyl substituted by hydroxy wherein the hydroxyphenyl is optionally further substituted by fluoro;
R¹⁰ is H;
r is 0; and/or
Z is absent.

3. The compound according to claim 1 or claim 2, wherein the compound is of Formula I' 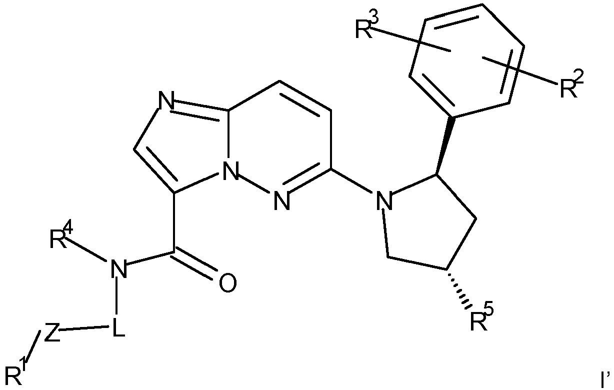 or a pharmaceutically acceptable salt or solvate thereof wherein R¹, R², R³, R⁴, R⁵, L and Z, are as defined in any preceding claim.

4. The compound according to any preceding claim, wherein the compound is of Formula la 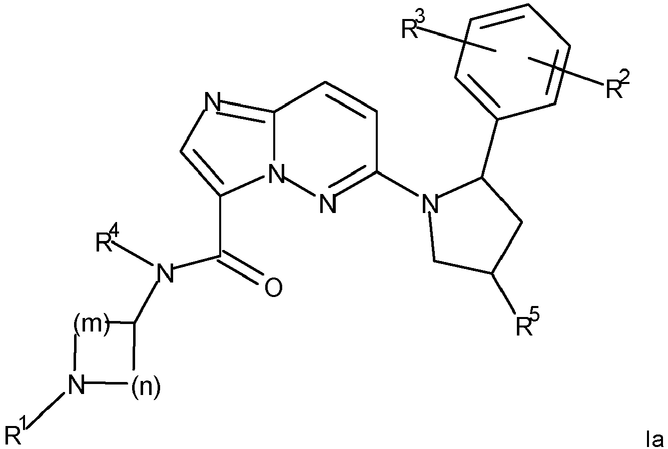 or a pharmaceutically acceptable salt or solvate thereof wherein R¹, R², R³, R⁴, R⁵, m and n, are as defined in any preceding claim.

5. The compound according to any preceding claim, wherein the compound is of Formula la' 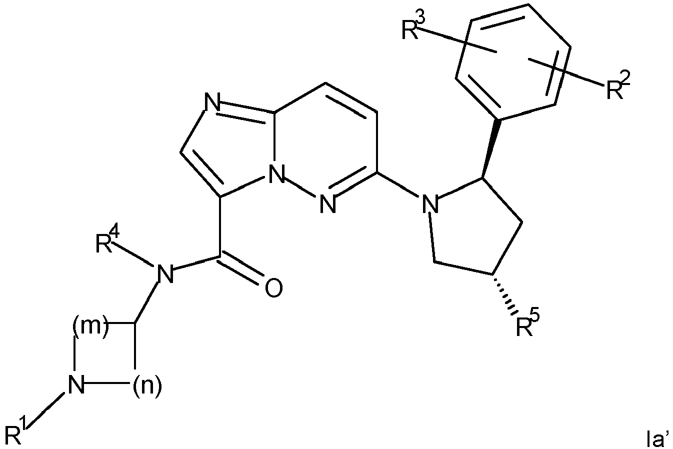 or a pharmaceutically acceptable salt or solvate thereof wherein R¹, R², R³, R⁴, R⁵, m and n, are as defined in any preceding claim.

6. The compound according to claim 1 or claim 2, wherein the compound is of Formula lb 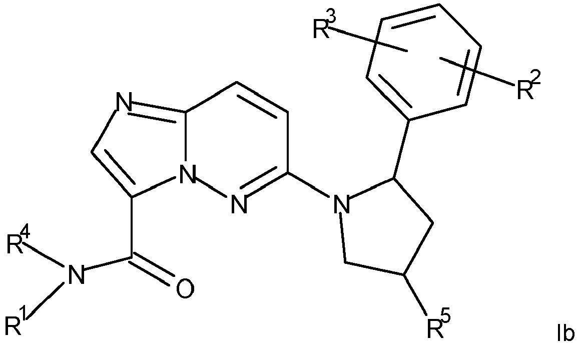 or a pharmaceutically acceptable salt or solvate thereof wherein R¹, R², R³, R⁴, R⁵, m and n, are as defined in claim 1 or claim 2.

7. The compound according to claim 1, 2, or 6, wherein the compound is of Formula Ib' 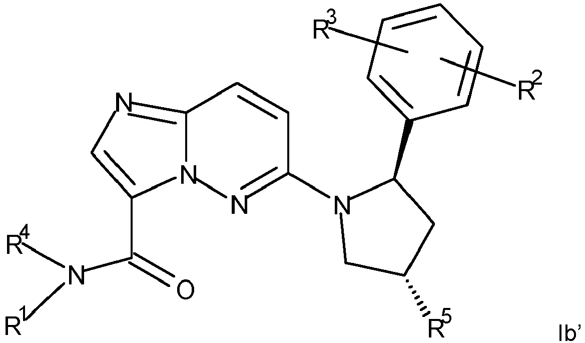 or a pharmaceutically acceptable salt or solvate thereof wherein R¹, R², R³, R⁴, R⁵, m and n, are as defined in claim 1, 2 or 6.

8. A compound according to claim 1, wherein said compound is selected from:
6-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[2-[5-fluoro-2-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
6-[4-fluoro-2-[5-fluoro-2-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[4-fluoro-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[4-fluoro-2-[5-fluoro-2-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[4-fluoro-2-[5-fluoro-2-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[4-fluoro-2-[5-fluoro-2-(methylsulfanyl)phenyl]pyrrolidin-1-yl]-N-[(3-hydroxyphenyl)methyl]imidazo[1,2-b]pyridazine-3-carboxamide;
6-[4-fluoro-2-[5-fluoro-2-(methylsulfanyl)phenyl]pyrrolidin-1-yl]-N-[1-[(3-hydroxyphenyl)methyl]pyrrolidin-3-yl]imidazo[1,2-b]pyridazine-3-carboxamide;
3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}phenyl pentanoate;
Methyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[4-fluoro-2-[5-fluoro-2-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
Butyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
Ethyl 3-{[3-{6-[4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[4-fluoro-2-[5-fluoro-2-(methylsulfanyl)phenyl]pyrrolidin-1-yl]-N-[(4-fluoro-3-hydroxyphenyl)methyl]imidazo[1,2-b]pyridazine-3-carboxamide;
N-[1-[(4-fluoro-3-hydroxyphenyl)methyl]pyrrolidin-3-yl]-6-[2-[3-fluoro-5-(methylsulfanyl)phenyl]pyrrolidin-1-yl]imidazo[1,2-b]pyridazine-3-carboxamide;
Methyl 3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
6-[4-fluoro-2-[3-fluoro-5-(methylsulfanyl)phenyl]pyrrolidin-1-yl]-N-[1-[(3-hydroxyphenyl)methyl]pyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-[1-[(4-fluoro-3-hydroxyphenyl)methyl]pyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-{1-[(4-fluoro-3-hydroxyphenyl) methyl]azetidin-3-yl}imidazo[1,2-b]pyridazine-3-carboxamide;
6-[4-fluoro-2-[3-fluoro-5-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[4-fluoro-2-[3-fluoro-5-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[4-fluoro-2-[3-fluoro-5-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[4-fluoro-2-[3-fluoro-5-(methylsulfanyl)phenyl]pyrrolidin-1-yl]-N-[1-[(4-fluoro-3-hydroxyphenyl)methyl]-4-methoxypyrrolidin-3-yl]imidazo[1,2-b]pyridazine-3-carboxamide;
6-[4-fluoro-2-[3-fluoro-5-(methylsulfanyl)phenyl]pyrrolidin-1-yl]-N-[4-[(4-fluoro-3-hydroxyphenyl)methyl]-1,4-oxazepan-6-yl]imidazo[1,2-b]pyridazine-3-carboxamide;
6-[4-fluoro-2-[3-fluoro-5-(methylsulfanyl)phenyl]pyrrolidin-1-yl]-N-[(4-fluoro-3-hydroxyphenyl)methyl]imidazo[1,2-b]pyridazine-3-carboxamide;
6-[4-fluoro-2-[3-fluoro-5-(methylsulfanyl)phenyl]pyrrolidin-1-yl]-N-[1-(3-hydroxybenzoyl)pyrrolidin-3-yl]imidazo[1,2-b]pyridazine-3-carboxamide;
3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}phenyl acetate;
3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid;
Butyl 3-{[3-{6-[4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
5- [4-fluoro-2-[3-fluoro-5-(methylsulfanyl)phenyl]pyrrolidin-1-yl]-N-{1-[(3-hydroxyphenyl)methyl]piperidin-4-yl}pyrazolo[1,5-a]pyrimidine-3-carboxamide;
or a pharmaceutically acceptable salt or solvate thereof,
preferably wherein said compound is selected from:
6-[(2 R)-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[(2R)-2-[5-fluoro-2-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(4-fluoro-3-hydroxyphenyl)methyl]azetidin-3-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[(2R,4S)-4-fluoro-2-[5-fluoro-2-(methylsulfanyl)phenyl]pyrrolidin-1-yl]-N-[(3-hydroxyphenyl)methyl]imidazo[1,2-b]pyridazine-3-carboxamide;
6-[(2R,4S)-4-fluoro-2-[5-fluoro-2-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide;
3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}phenyl pentanoate;
Methyl3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[5-fluoro-2-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide; Butyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
Ethyl 3-{[(3S)-3-{6-[(2R,4S)-4-fluoro-2-[5-fluoro-2-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[5-fluoro-2-(methylsulfanyl)phenyl]pyrrolidin-1 -yl]-N-[(4-fluoro-3-hydroxyphenyl)methyl]imidazo[1,2-b]pyridazine-3-carboxamide;
N-[(3S)-1-[(4-fluoro-3-hydroxyphenyl)methyl]pyrrolidin-3-yl]-6-[(2R)-2-[3-fluoro-5-(methylsulfanyl)phenyl]pyrrolidin-1-yl]imidazo[1,2-b]pyridazine-3-carboxamide;
Methyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
6-[(2R,4S)-4-fluoro-2-[3-fluoro-5-(methylsulfanyl)phenyl]pyrrolidin-1-yl]-N-[(3S)-1-[(3-hydroxyphenyl)methyl]pyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S)-1-[(4-fluoro-3-hydroxyphenyl)methyl]pyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(4-fluoro-3-hydroxyphenyl) methyl]azetidin-3-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(3-hydroxyphenyl)methyl]azetidin-3-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(4-fluoro-3-hydroxyphenyl)methyl]piperidin-4-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-{1-[(3-hydroxyphenyl)methyl]piperidin-4-yl}imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S,4S)-1-[(4-fluoro-3-hydroxyphenyl)methyl]-4-methoxypyrrolidin-3-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(6S)-4-[(4-fluoro-3-hydroxyphenyl)methyl]-1,4-oxazepan-6-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(6R)-4-[(4-fluoro-3-hydroxyphenyl)methyl]-1,4-oxazepan-6-yl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(4-fluoro-3-hydroxyphenyl)methyl]imidazo[1,2-b]pyridazine-3-carboxamide;
6-[(2R,4S)-4-fluoro-2-[3-fluoro-5-(methylsulfanyl)phenyl]pyrrolidin-1-yl]-N-[(3S)-1-(3-hydroxybenzoyl)pyrrolidin-3-yl]imidazo[1,2-b]pyridazine-3-carboxamide;
3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}phenyl acetate;
3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoic acid;
Butyl 3-{[(3S)-3-{6-[(2R,4S)-4-fluoro-2-[3-fluoro-5-(methylsulfanyl)phenyl]pyrrolidin-1-yl]imidazo[1,2-b]pyridazine-3-amido}pyrrolidin-1-yl]methyl}benzoate;
5-[(2R,4S)-4-fluoro-2-[3-fluoro-5-(methylsulfanyl)phenyl]pyrrolidin-1-yl]-N-{1-[(3-hydroxyphenyl)methyl]piperidin-4-yl}pyrazolo[1,5-a]pyrimidine-3-carboxamide;
or a pharmaceutically acceptable salt or solvate thereof.

9. A pharmaceutical composition comprising a compound of Formula (I): 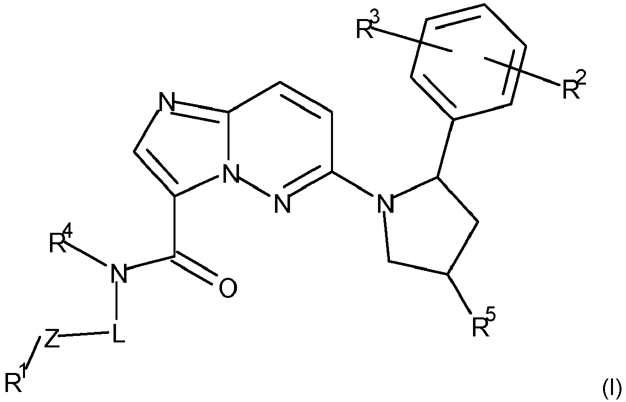 or a pharmaceutically acceptable salt or solvate thereof;
wherein:
L is (CR⁶R⁷)ᵣ;
Z is absent or selected from:
i) 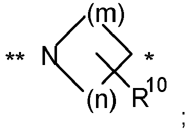 and
ii) 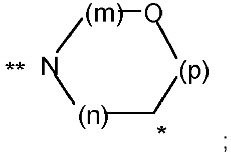
wherein * denotes the point of attachment to L and ** denotes the point of attachment to R1;
m is 1 or 2;
n is 1 or 2;
p is 0 or 1;
provided that the sum of m, n and p is in the range of 2 to 4;
r is 0 or 1;
R1 is -XR⁹;
X is selected from -CH₂-, -C(O)-, and -S(O₂)-;
R² is -SR⁸;
R³ is selected from H and halo;
R⁴ is selected from H and (C₁-C₃)alkyl;
R⁵ is selected from H, hydroxyl and halo;
R⁶ and R⁷ are each independently selected from H and (C₁-C₃)alkyl;
R⁸ is methyl;
R⁹ is phenyl substituted by a group selected from hydroxy , -OC(O)(C₁₋C₆)alkyl, C(O)OH and -C(O)O(C₁-C₆)alkyl, wherein the phenyl ring is optionally further substituted by halo;
R¹⁰ is selected from H and (C₁-C₃)alkoxy,
and one or more pharmaceutically acceptable excipients.

10. A pharmaceutical composition according to claim 9, wherein
R¹ is -CH₂R⁹;
R³ is H or fluoro;
R⁴ is H;
R⁵ is H or fluoro;
R⁶ is H;
R⁷ is H;
R⁹ is phenyl substituted by hydroxy wherein the hydroxyphenyl is optionally further substituted by fluoro;
R¹⁰ is H;
r is 0; and/or
Z is absent.

11. A pharmaceutical composition according to claim 9 or 10, wherein the compound is of Formula I' or Formula lb, and preferably Formula Ib', or a pharmaceutically acceptable salt or solvate thereof 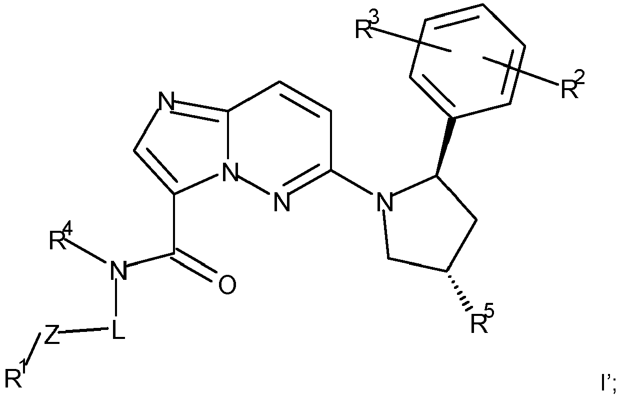 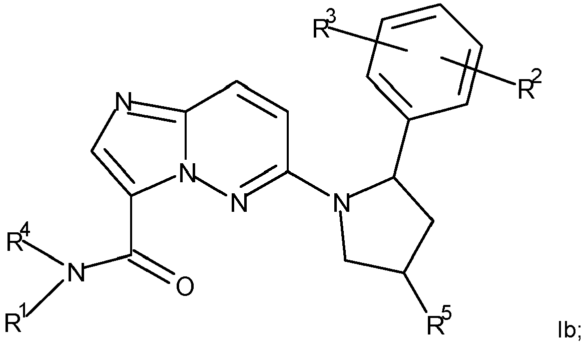 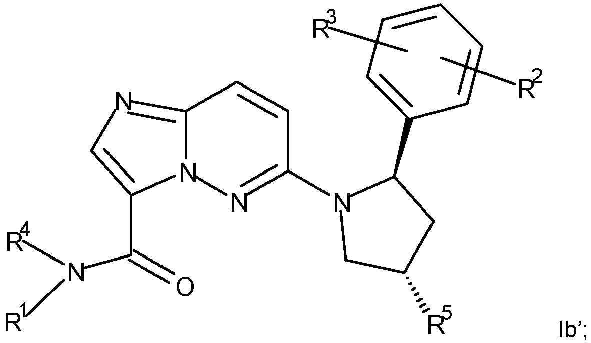 wherein R¹, R², R³, R⁴, R⁵, L, Z, m and n, are as defined in claim 9 or claim 10.

12. A pharmaceutical composition comprising a compound according to any of claims 4, 5 or 8 and one or more pharmaceutically acceptable excipients.

13. A pharmaceutical composition according to any of claims 9 to 12, in combination with one or more other therapeutic agents.

14. A compound according to any of claims 1 to 8, or a pharmaceutical composition according to any of claims 9 to 13, for use as a pharmaceutical.

15. A compound as defined in any of claims 1 to 8, or a pharmaceutical composition according to any of claims 9 to 13, for use in treating or preventing a condition or disorder which is mediated by Trk.

16. A compound or pharmaceutical composition for use according to claim 15 wherein the condition or disorder is mediated by TrkA, TrkB, and TrkC.

17. A compound or pharmaceutical composition for use according to claim 15 or 16 wherein the condition or disorder is atopic dermatitis.

18. A compound or pharmaceutical composition for use according to any one of claims 15 to 17 wherein the compound or pharmaceutical composition is administered topically.

## Patentansprüche

1. Verbindung der Formel (I): 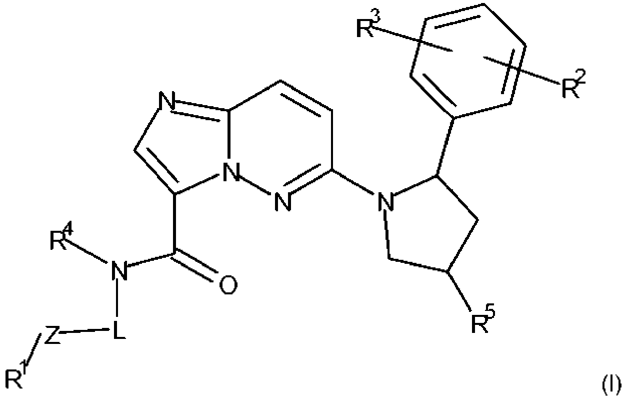 oder ein pharmazeutisch verträgliches Salz oder Solvat davon;
wobei:
L (CR⁶R⁷)ᵣ ist;
Z fehlt oder ausgewählt ist aus:
i) 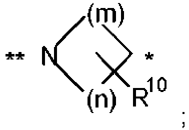 und
ii) 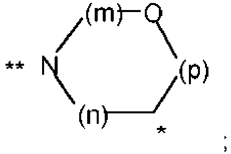
wobei * den Bindungspunkt an L bezeichnet und ** den Bindungspunkt an R¹ bezeichnet;
m 1 oder 2 ist;
n 1 oder 2 ist;
p 0 oder 1 ist;
unter der Voraussetzung, dass die Summe aus m, n und p im Bereich von 2 bis 4 liegt;
r 0 oder 1 ist;
R¹ -XR⁹ ist;
X ausgewählt ist aus -CH₂-, -C(O)-, und -S(O₂)-;
R² -SR⁸ ist;
R³ ausgewählt ist aus H und Halogen;
R⁴ ausgewählt ist aus H und (C₁-C₃)Alkyl;
R⁵ ausgewählt ist aus H, Hydroxyl und Halogen;
R⁶ und R⁷ jeweils unabhängig ausgewählt sind aus H und (C₁-C₃)Alkyl;
R⁸ Methyl ist;
R⁹ Phenyl ist, substituiert durch eine Gruppe, ausgewählt aus Hydroxy, -OC(O)(C₁-C₆)Alkyl, C(O)OH und -C(O)O(C₁-C₆)Alkyl, wobei der Phenylring gegebenenfalls weiter durch Halogen substituiert ist;
R¹⁰ ausgewählt ist aus H und (C₁-C₃)Alkoxy;
und wobei die Verbindung nicht 6-[(2R)-2-[5-Fluor-2-(methylsulfanyl)phenyl]pyrrolidin-1-yl]-N-[(3-hydroxyphenyl)methyl]imidazo[1,2-b]pyridazin-3-carboxamid ist:

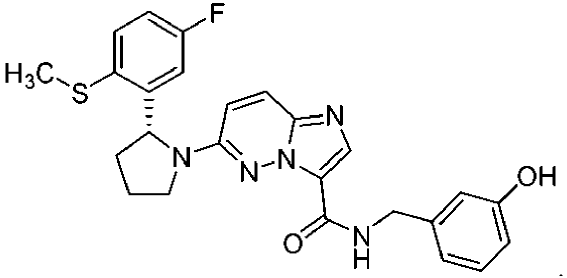

2. Verbindung nach Anspruch 1, wobei
R¹ -CH₂R⁹ ist;
R³ H oder Fluor ist;
R⁴ H ist;
R⁵ H oder Fluor ist;
R⁶ H ist;
R⁷ H ist;
R⁹ durch Hydroxy substituiertes Phenyl ist, wobei das Hydroxyphenyl gegebenenfalls weiter durch Fluor substituiert ist;
R¹⁰ H ist;
r 0 ist; und/oder
Z fehlt.

3. Verbindung nach Anspruch 1 oder Anspruch 2, wobei die Verbindung nach der Formel I' ist 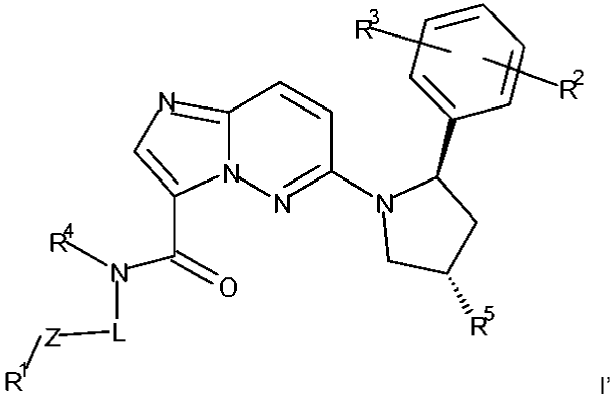 oder ein pharmazeutisch verträgliches Salz oder Solvat davon, wobei R¹, R², R³, R⁴, R⁵, L und Z wie in einem vorstehenden Anspruch definiert sind.

4. Verbindung nach einem vorstehenden Anspruch, wobei die Verbindung nach der Formel la ist 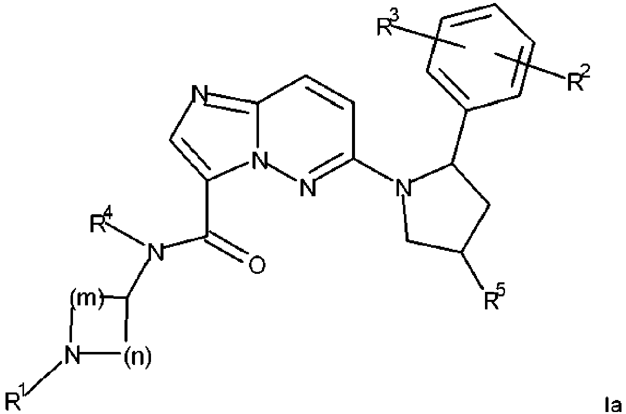 oder ein pharmazeutisch verträgliches Salz oder Solvat davon, wobei R¹, R², R³, R⁴, R⁵, m und n wie in einem vorstehenden Anspruch definiert sind.

5. Verbindung nach einem vorstehenden Anspruch, wobei die Verbindung nach der Formel la' ist 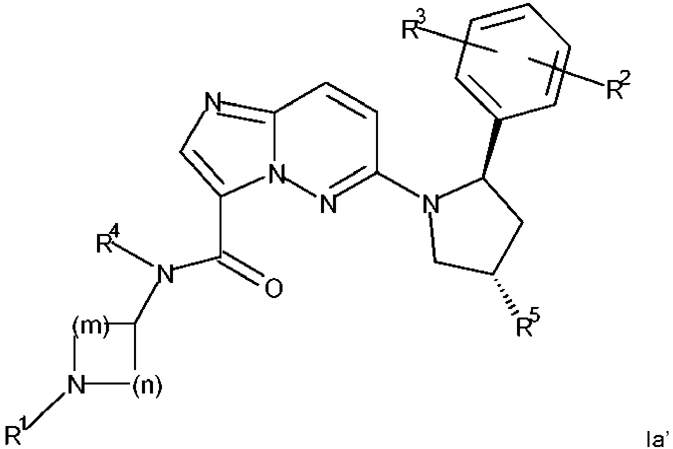 oder ein pharmazeutisch verträgliches Salz oder Solvat davon, wobei R¹, R², R³, R⁴, R⁵, m und n wie in einem vorstehenden Anspruch definiert sind.

6. Verbindung nach Anspruch 1 oder Anspruch 2, wobei die Verbindung nach der Formel Ib ist 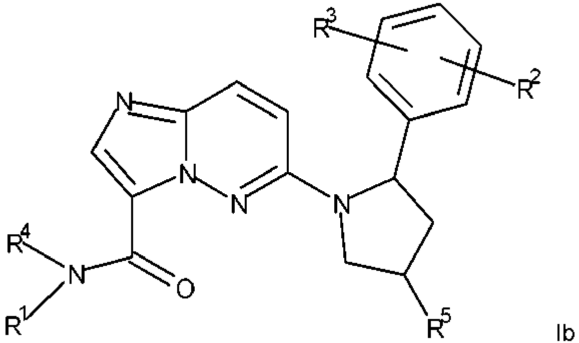 oder ein pharmazeutisch verträgliches Salz oder Solvat davon, wobei R¹, R², R³, R⁴, R⁵, m und n wie in Anspruch 1 oder Anspruch 2 definiert sind.

7. Verbindung nach Anspruch 1, 2, oder 6, wobei die Verbindung nach der Formel Ib' ist 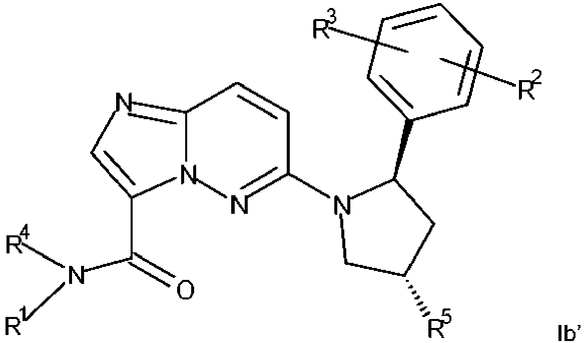 oder ein pharmazeutisch verträgliches Salz oder Solvat davon, wobei R¹, R², R³, R⁴, R^{5,} m und n wie in Anspruch 1, 2 oder 6 definiert sind.

8. Verbindung nach Anspruch 1, wobei die Verbindung ausgewählt ist aus:
6-2-[5-Fluor-2-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[2-[5-Fluor-2-(methylsulfanyl)phenyl]pyrrolidin-1-yl]-N-[1-[(4-fluor-3-hydroxyphenyl)methyl]pyrrolidin-3-yl]imidazo[1,2-b]pyridazin-3-carboxamid;
6-[4-Fluor-2-[5-fluor-2-(methylsulfanyl)phenyl]pyrrolidin-1-yl]-N-{1-[(3-hydroxyphenyl)methyl]piperidin-4-yl}imidazo[1,2-b]pyridazin-3-carboxamid;
6-[4-Fluor-2-[5-fluor-2-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[4-Fluor-2-[5-fluor-2-(methylsulfanyl)phenyl]pyrrolidin-1-yl]-N-{1-[(3-hydroxyphenyl)methyl]azetidin-3-yl}imidazo[1,2-b]pyridazin-3-carboxamid;
6-[4-Fluor-2-[5-fluor-2-(methylsulfanyl)phenyl]pyrrolidin-1-yl]-N-{1-[(4-fluor-3-hydroxyphenyl)methyl]piperidin-4-yl}imidazo[1,2-b]pyridazin-3-carboxamid;
6-[4-Fluor-2-[5-fluor-2-(methylsulfanyl)phenyl]pyrrolidin-1-yl]-N-[(3-hydroxyphenyl)methyl]imidazo[1,2-b]pyridazin-3-carboxamid;
6-[4-Fluor-2-[5-fluor-2-(methylsulfanyl)phenyl]pyrrolidin-1-yl]-N-[1-[(3-hydroxyphenyl)methyl]pyrrolidin-3-yl]imidazo[1,2-b]pyridazin-3-carboxamid;
3-{[3-{6-[4-Fluor-2-[5-fluor-2-(methylsulfanyl)phenyl]pyrrolidin-1-yl]imidazo[1,2-b]pyridazin-3-amido}pyrrolidin-1-yl]methyl}phenylpentanoat;
Methyl 3-{[3-{6-[4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
6-[4-Fluor-2-[5-fluor-2-(methylsulfanyl)phenyl]pyrrolidin-1-yl]-N-[1-[(4-fluor-3-hydroxyphenyl)methyl]pyrrolidin-3-yl]imidazo[1,2-b]pyridazin-3-carboxamid;
Butyl 3-{[3-{6-[4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
Ethyl 3-{[3-{6-[4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
6-[4-Fluor-2-[5-fluor-2-(methylsulfanyl)phenyl]pyrrolidin-1-yl]-N-[(4-fluor-3-hydroxyphenyl)methyl]imidazo[1,2-b]pyridazin-3-carboxamid;
N-[1-[(4-Fluor-3-hydroxyphenyl)methyl]pyrrolidin-3-yl]-6-[2-[3-fluor-5-(methylsulfanyl)phenyl]pyrrolidin-1-yl]imidazo[1,2-b]pyridazin-3-carboxamid;
Methyl 3-{[3-{6-[4-fluor-2-[3-fluor-5-(methylsulfanyl)phenyl]pyrrolidin-1-yl]imidazo[1,2-b]pyridazin-3-amido}pyrrolidin-1-yl]methyl}benzoat;
6-[4-Fluor-2-[3-fluor-5-(methylsulfanyl)phenyl]pyrrolidin-1-yl]-N-[1-[(3-hydroxyphenyl)methyl]pyrrolidin-3-yl]imidazo[1,2-b]pyridazin-3-carboxamid;
6-[4-Fluor-2-[3-fluor-5-(methylsulfanyl)phenyl]pyrrolidin-1-yl]-N-[1-[(4-fluor-3-hydroxyphenyl)methyl]pyrrolidin-3-yl]imidazo[1,2-b]pyridazin-3-carboxamid;
6-[4-Fluor-2-[3-fluor-5-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[4-Fluor-2-[3-fluor-5-(methylsulfanyl)phenyl]pyrrolidin-1-yl]-N-{1-[(3-hydroxyphenyl)methyl]azetidin-3-yl}imidazo[1,2-b]pyridazin-3-carboxamid;
6-[4-Fluor-2-[3-fluor-5-(methylsulfanyl)phenyl]pyrrolidin-1-yl]-N-{1-[(4-fluor-3-hydroxyphenyl)methyl]piperidin-4-yl}imidazo[1,2-b]pyridazin-3-carboxamid;
6-[4-Fluor-2-[3-fluor-5-(methylsulfanyl)phenyl]pyrrolidin-1-yl]-N-{1-[(3-hydroxyphenyl)methyl]piperidin-4-yl}imidazo[1,2-b]pyridazin-3-carboxamid;
6-[4-Fluor-2-[3-fluor-5-(methylsulfanyl)phenyl]pyrrolidin-1-yl]-N-[1-[(4-fluor-3-hydroxyphenyl)methyl]-4-methoxypyrrolidin-3-yl]imidazo[1,2-b]pyridazin-3-carboxamid;
6-[4-Fluor-2-[3-fluor-5-(methylsulfanyl)phenyl]pyrrolidin-1-yl]-N-[4-[(4-fluor-3-hydroxyphenyl)methyl]-1,4-oxazepan-6-yl]imidazo[1,2-b]pyridazin-3-carboxamid;
6-[4-Fluor-2-[3-fluor-5-(methylsulfanyl)phenyl]pyrrolidin-1-yl]-N-[(4-fluor-3-hydroxyphenyl)methyl]imidazo[1,2-b]pyridazin-3-carboxamid;
6-[4-Fluor-2-[3-fluor-5-(methylsulfanyl)phenyl]pyrrolidin-1-yl]-N-[1-(3-hydroxybenzoyl)pyrrolidin-3-yl]imidazo[1,2-b]pyridazin-3-carboxamid;
3-{[3-{6-[4-Fluor-2-[3-fluor-5-(methylsulfanyl)phenyl]pyrrolidin-1-yl]imidazo[1,2-b]pyridazin-3-amido}pyrrolidin-1-yl]methyl}phenylacetat;
3-{[3-{6-[4-Fluor-2-[3-fluor-5-(methylsulfanyl)phenyl]pyrrolidin-1-yl]imidazo[1,2-b]pyridazin-3-amido}pyrrolidin-1-yl]methyl}benzoesäure;
Butyl 3-{[3-{6-[4-fluor-2-[3-fluor-5-(methylsulfanyl)phenyl]pyrrolidin-1-yl]imidazo[1,2-b]pyridazin-3-amido}pyrrolidin-1-yl]methyl}benzoat;
5-[4-Fluor-2-[3-fluor-5-(methylsulfanyl)phenyl]pyrrolidin-1-yl]-N-{1-[(3-hydroxyphenyl)methyl]piperidin-4-yl}pyrazol[1,5-a]pyrimidin-3-carboxamid;
oder ein pharmazeutisch verträgliches Salz oder Solvat davon,
vorzugsweise wobei die Verbindung ausgewählt ist aus:
6-[(2R)-2-[5-Fluor-2-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[(2R)-2-[5-Fluor-2-(methylsulfanyl)phenyl]pyrrolidin-1-yl]-N-[(3S)-1-[(4-fluor-3-hydroxyphenyl)methyl]pyrrolidin-3-yl]imidazo[1,2-b]pyridazin-3-carboxamid;
6-[(2R,4S)-4-Fluor-2-[5-fluor-2-(methylsulfanyl)phenyl]pyrrolidin-1-yl]-N-{1-[(3-hydroxyphenyl)methyl]piperidin-4-yl}imidazo[1,2-b]pyridazin-3-carboxamid;
6-[(2R,4S)-4-Fluor-2-[5-fluor-2-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[(2R,4S)-4-Fluor-2-[5-fluor-2-(methylsulfanyl)phenyl]pyrrolidin-1-yl]-N-{1-[(3-hydroxyphenyl)methyl]azetidin-3-yl}imidazo[1,2-b]pyridazin-3-carboxamid;
6-[(2R,4S)-4-Fluor-2-[5-fluor-2-(methylsulfanyl)phenyl]pyrrolidin-1-yl]-N-{1-[(4-fluor-3-hydroxyphenyl)methyl]piperidin-4-yl}imidazo[1,2-b]pyridazin-3-carboxamid;
6-[(2R,4S)-4-Fluor-2-[5-fluor-2-(methylsulfanyl)phenyl]pyrrolidin-1-yl]-N-[(3-hydroxyphenyl)methyl]imidazo[1,2-b]pyridazin-3-carboxamid;
6-[(2R,4S)-4-Fluor-2-[5-fluor-2-(methylsulfanyl)phenyl]pyrrolidin-1-yl]-N-[(3S)-1-[(3-hydroxyphenyl)methyl]pyrrolidin-3-yl]imidazo[1,2-b]pyridazin-3-carboxamid;
3-{[(3S)-3-{6-[(2R,4S)-4-Fluor-2-[5-fluor-2-(methylsulfanyl)phenyl]pyrrolidin-1-yl]imidazo[1,2-b]pyridazin-3-amido}pyrrolidin-1-yl]methyl}phenylpentanoat;
Methyl 3-{[(3S)-3-{6-[(2R,4S)-4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
6-[(2R,4S)-4-Fluor-2-[5-fluor-2-(methylsulfanyl)phenyl]pyrrolidin-1-yl]-N-[(3S)-1-[(4-fluor-3-hydroxyphenyl)methyl]pyrrolidin-3-yl]imidazo[1,2-b]pyridazin-3-carboxamid;
Butyl 3-{[(3S)-3-{6-[(2R,4S)-4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
Ethyl 3-{[(3S)-3-{6-[(2R,4S)-4-fluor-2-[5-fluor-2-(methylsulfanyl)phenyl]pyrrolidin-1-yl]imidazo[1,2-b]pyridazin-3-amido}pyrrolidin-1-yl]methyl}benzoat;
6-[(2R,4S)-4-Fluor-2-[5-fluor-2-(methylsulfanyl)phenyl]pyrrolidin-1-yl]-N-[(4-fluor-3-hydroxyphenyl)methyl]imidazo[1,2-b]pyridazin-3-carboxamid;
N-[(3S)-1-[(4-Fluor-3-hydroxyphenyl)methyl]pyrrolidin-3-yl]-6-[(2R)-2-[3-fluor-5-(methylsulfanyl)phenyl]pyrrolidin-1-yl]imidazo[1,2-b]pyridazin-3-carboxamid;
Methyl 3-{[(3S)-3-{6-[(2R,4S)-4-fluor-2-[3-fluor-5-(methylsulfanyl)phenyl]pyrrolidin-1-yl]imidazo[1,2-b]pyridazin-3-amido}pyrrolidin-1-yl]methyl}benzoat;
6-[(2R,4S)-4-Fluor-2-[3-fluor-5-(methylsulfanyl)phenyl]pyrrolidin-1-yl]-N-[(3S)-1-[(3-hydroxyphenyl)methyl]pyrrolidin-3-yl]imidazo[1,2-b]pyridazin-3-carboxamid;
6-[(2R,4S)-4-Fluor-2-[3-fluor-5-(methylsulfanyl)phenyl]pyrrolidin-1-yl]-N-[(3S)-1-[(4-fluor-3-hydroxyphenyl)methyl]pyrrolidin-3-yl]imidazo[1,2-b]pyridazin-3-carboxamid;
6-[(2R,4S)-4-Fluor-2-[3-fluor-5-(methylsulfanyl)phenyl]pyrrolidin-1-yl]-N-{1-[(4-fluor-3-hydroxyphenyl)methyl]azetidin-3-yl}imidazo[1,2-b]pyridazin-3-carboxamid;
6-[(2R,4S)-4-Fluor-2-[3-fluor-5-(methylsulfanyl)phenyl]pyrrolidin-1-yl]-N-{1-[(3-hydroxyphenyl)methyl]azetidin-3-yl}imidazo[1,2-b]pyridazin-3-carboxamid;
6-[(2R,4S)-4-Fluor-2-[3-fluor-5-(methylsulfanyl)phenyl]pyrrolidin-1-yl]-N-{1-[(4-fluor-3-hydroxyphenyl)methyl]piperidin-4-yl}imidazo[1,2-b]pyridazin-3-carboxamid;
6-[(2R,4S)-4-Fluor-2-[3-fluor-5-(methylsulfanyl)phenyl]pyrrolidin-1-yl]-N-{1-[(3-hydroxyphenyl)methyl]piperidin-4-yl}imidazo[1,2-b]pyridazin-3-carboxamid;
6-[(2R,4S)-4-Fluor-2-[3-fluor-5-(methylsulfanyl)phenyl]pyrrolidin-1-yl]-N-[(3S,4S)-1-[(4-fluor-3-hydroxyphenyl)methyl]-4-methoxypyrrolidin-3-yl]imidazo[1,2-b]pyridazin-3-carboxamid;
6-[(2R,4S)-4-Fluor-2-[3-fluor-5-(methylsulfanyl)phenyl]pyrrolidin-1-yl]-N-[(6S)-4-[(4-fluor-3-hydroxyphenyl)methyl]-1,4-oxazepan-6-yl]imidazo[1,2-b]pyridazin-3-carboxamid;
6-[(2R,4S)-4-Fluor-2-[3-fluor-5-(methylsulfanyl)phenyl]pyrrolidin-1-yl]-N-[(6R)-4-[(4-fluor-3-hydroxyphenyl)methyl]-1,4-oxazepan-6-yl]imidazo[1,2-b]pyridazin-3-carboxamid;
6-[(2R,4S)-4-Fluor-2-[3-fluor-5-(methylsulfanyl)phenyl]pyrrolidin-1-yl]-N-[(4-fluor-3-hydroxyphenyl)methyl]imidazo[1,2-b]pyridazin-3-carboxamid;
6-[(2R,4S)-4-Fluor-2-[3-fluor-5-(methylsulfanyl)phenyl]pyrrolidin-1-yl]-N-[(3S)-1-(3-hydroxybenzoyl)pyrrolidin-3-yl]imidazo[1,2-b]pyridazin-3-carboxamid;
3-{[(3S)-3-{6-[(2R,4S)-4-Fluor-2-[3-fluor-5-(methylsulfanyl)phenyl]pyrrolidin-1-yl]imidazo[1,2-b]pyridazin-3-amido}pyrrolidin-1-yl]methyl}phenylacetat;
3-{[(3S)-3-{6-[(2R,4S)-4-Fluor-2-[3-fluor-5-(methylsulfanyl)phenyl]pyrrolidin-1-yl]imidazo[1,2-b]pyridazin-3-amido}pyrrolidin-1-yl]methyl}benzoesäure;
Butyl 3-{[(3S)-3-{6-[(2R,4S)-4-fluor-2-[3-fluor-5-(methylsulfanyl)phenyl]pyrrolidin-1-yl]imidazo[1,2-b]pyridazin-3-amido}pyrrolidin-1-yl]methyl}benzoat;
5-[(2R,4S)-4-Fluor-2-[3-fluor-5-(methylsulfanyl)phenyl]pyrrolidin-1-yl]-N-{1-[(3-hydroxyphenyl)methyl]piperidin-4-yl}pyrazol[1,5-a]pyrimidin-3-carboxamid;
oder ein pharmazeutisch verträgliches Salz oder Solvat davon.

9. Pharmazeutische Zusammensetzung, die eine Verbindung der Formel (I) umfasst: 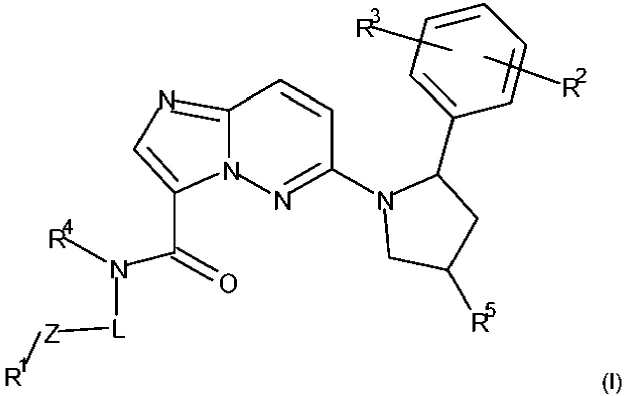 oder ein pharmazeutisch verträgliches Salz oder Solvat davon;
wobei:
L (CR⁶R⁷)ᵣ ist;
Z fehlt oder ausgewählt ist aus:
i) 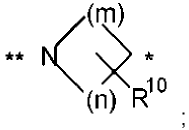 und
ii) 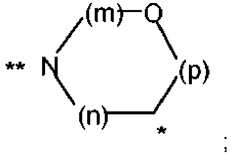
wobei * den Bindungspunkt an L bezeichnet und ** den Bindungspunkt an R¹ bezeichnet;
m 1 oder 2 ist;
n 1 oder 2 ist;
p 0 oder 1 ist;
unter der Voraussetzung, dass die Summe aus m, n und p im Bereich von 2 bis 4 liegt;
r 0 oder 1 ist;
R¹ -XR⁹ ist;
X ausgewählt ist aus -CH₂-, -C(O)-, und -S(O₂)-;
R² -SR⁸ ist;
R³ ausgewählt ist aus H und Halogen;
R⁴ ausgewählt ist aus H und (C₁-C₃)Alkyl;
R⁵ ausgewählt ist aus H, Hydroxyl und Halogen;
R⁶ und R⁷ jeweils unabhängig ausgewählt sind aus H und (C₁-C₃)Alkyl;
R⁸ Methyl ist;
R⁹ Phenyl ist, substituiert durch eine Gruppe, ausgewählt aus Hydroxy, -OC(O)(C₁-C₆)Alkyl, C(O)OH und -C(O)O(C₁-C₆)Alkyl, wobei der Phenylring gegebenenfalls weiter durch Halogen substituiert ist;
R¹⁰ ausgewählt ist aus H und (C₁-C₃)Alkoxy,
und ein oder mehrere pharmazeutisch verträgliche Hilfsstoffe.

10. Pharmazeutische Zusammensetzung nach Anspruch 9, wobei
R¹ -CH₂R⁹ ist;
R³ H oder Fluor ist;
R⁴ H ist;
R⁵ H oder Fluor ist;
R⁶ H ist;
R⁷ H ist;
R⁹ durch Hydroxy substituiertes Phenyl ist, wobei das Hydroxyphenyl gegebenenfalls weiter durch Fluor substituiert ist;
R¹⁰ H ist;
r 0 ist; und/oder
Z fehlt.

11. Pharmazeutische Zusammensetzung nach Anspruch 9 oder 10, wobei die Verbindung nach der Formel I' oder Formel Ib, und vorzugsweise Formel Ib' ist, oder ein pharmazeutisch verträgliches Salz oder Solvat davon, 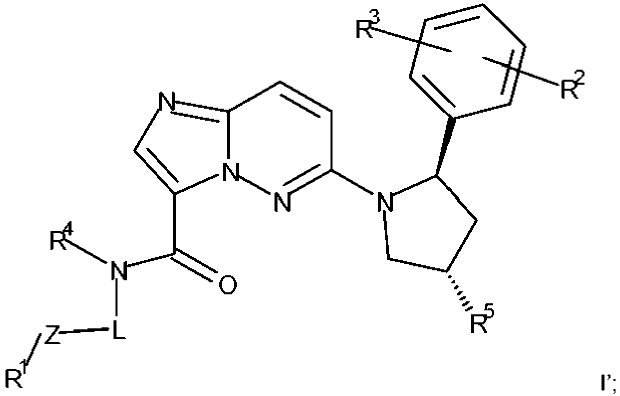 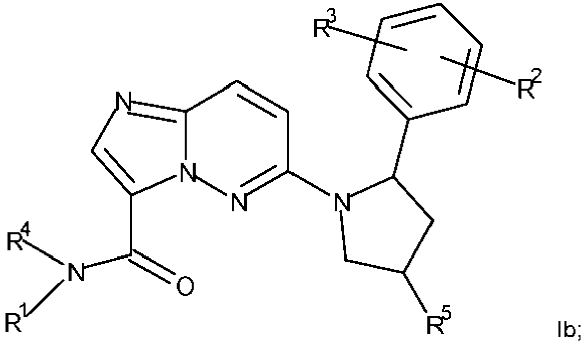 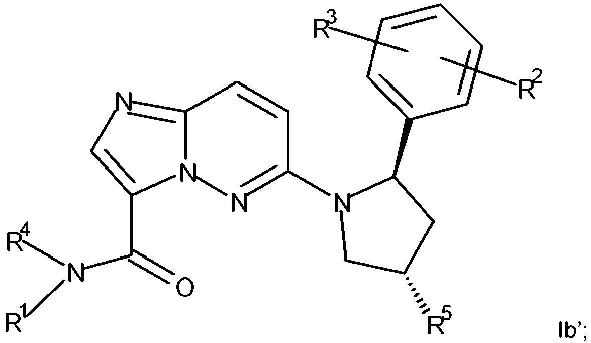 wobei R¹, R², R³, R⁴, R⁵, L, Z, m und n wie in Anspruch 9 oder Anspruch 10 definiert sind.

12. Pharmazeutische Zusammensetzung, die eine Verbindung nach einem der Ansprüche 4, 5 oder 8 und einen oder mehrere pharmazeutisch verträgliche Hilfsstoffe umfasst.

13. Pharmazeutische Zusammensetzung nach einem der Ansprüche 9 bis 12 in Kombination mit einem oder mehreren anderen therapeutischen Mitteln.

14. Verbindung nach einem der Ansprüche 1 bis 8 oder pharmazeutische Zusammensetzung nach einem der Ansprüche 9 bis 13 zur Verwendung als Arzneimittel.

15. Verbindung wie in einem der Ansprüche 1 bis 8 definiert oder pharmazeutische Zusammensetzung nach einem der Ansprüche 9 bis 13 zur Verwendung in der Behandlung oder Vorbeugung einer Erkrankung oder Störung, die durch Trk vermittelt wird.

16. Verbindung oder pharmazeutische Zusammensetzung zur Verwendung nach Anspruch 15, wobei die Erkrankung oder Störung durch TrkA, TrkB, und TrkC vermittelt wird.

17. Verbindung oder pharmazeutische Zusammensetzung zur Verwendung nach Anspruch 15 oder 16, wobei die Erkrankung oder Störung atopische Dermatitis ist.

18. Verbindung oder pharmazeutische Zusammensetzung zur Verwendung nach einem der Ansprüche 15 bis 17, wobei die Verbindung oder pharmazeutische Zusammensetzung topisch verabreicht wird.

## Revendications

1. Composé de Formule (I) : 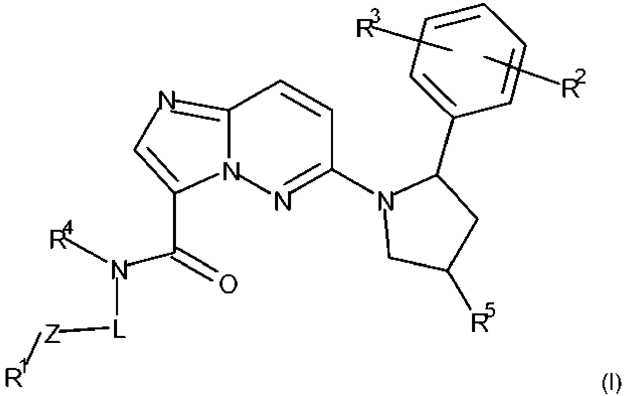 ou un sel ou solvate pharmaceutiquement acceptable de celui-ci ;
dans lequel :
Lest (CR⁶R⁷)ᵣ;
Z est absent ou sélectionné parmi :
i) 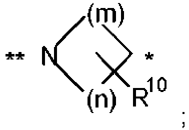 et
ii) 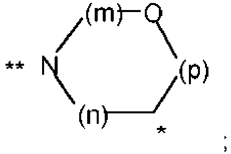
dans laquelle * désigne le point d'attache à L et ** désigne le point d'attache à R¹ ;
m est 1 ou 2 ;
n est 1 ou 2 ;
p est 0 ou 1 ;
à condition que la somme de m, n et p soit dans la plage de 2 à 4 ;
r est 0 ou 1 ;
R¹ est -XR⁹ ;
X est sélectionné parmi -CH₂-, -C(O)-, et -S(O₂)- ;
R² est -SR⁸ ;
R³ est sélectionné parmi H et un halo ;
R⁴ est sélectionné parmi H et un alkyle en C₁-C₃ ;
R⁵ est sélectionné parmi H, un hydroxyle et un halo ;
R⁶ et R⁷ sont chacun indépendamment sélectionnés parmi H et un alkyle en C₁-C₃ ;
R⁸ est un méthyle ;
R⁹ est un phényle substitué par un groupe sélectionné parmi un hydroxy, - OC(O)alkyle en C₁-C₆, C(O)OH et -C(O)Oalkyle en C₁-C₆, dans lequel le cycle phényle est facultativement substitué en outre par un halo ;
R¹⁰ est sélectionné parmi H et un alcoxy en C₁-C₃ ;
et dans lequel le composé n'est pas le 6-[(2R)-2-[5-fluoro-2-(méthylsulfanyl)phényl]pyrrolidin-1-yl]-N-[(3-hydroxyphényl)méthyl]imidazo[1,2-b]pyridazine-3-carboxamide:

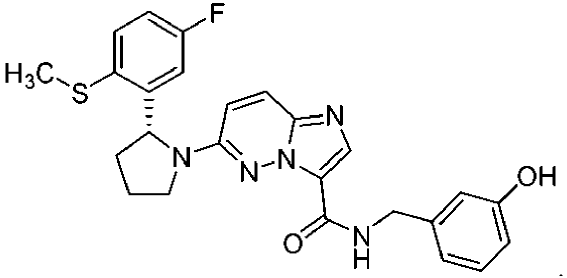

2. Composé selon la revendication 1, dans lequel
R¹ est -CH₂R⁹ ;
R³ est H ou un fluoro ;
R⁴ est H ;
R⁵ est H ou un fluoro ;
R⁶ est H ;
R⁷ est H ;
R⁹ est un phényle substitué par un hydroxy, dans lequel l'hydroxyphényle est facultativement substitué en outre par un fluoro ;
R¹⁰ est H ;
r est 0 ; et/ou
Z est absent.

3. Composé selon la revendication 1 ou la revendication 2, dans lequel le composé est de
Formule I' 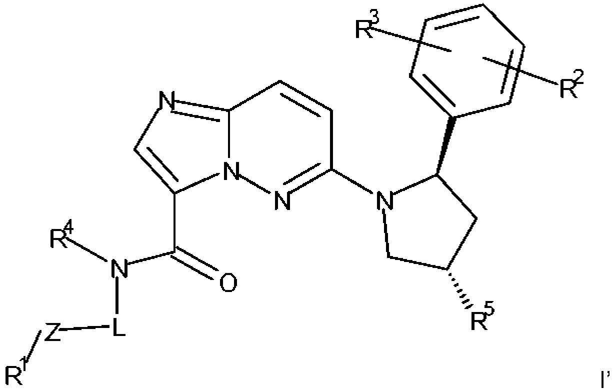 ou un sel ou solvate pharmaceutiquement acceptable de celui-ci, dans lequel R¹, R², R³, R⁴, R⁵, L et Z sont tels que définis dans une quelconque revendication précédente.

4. Composé selon une quelconque revendication précédente, dans lequel le composé est de Formule la 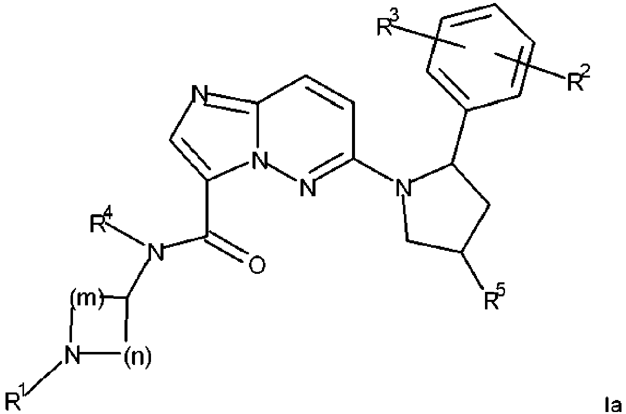 ou un sel ou solvate pharmaceutiquement acceptable de celui-ci, dans lequel R¹, R², R³, R⁴, R⁵, m et n sont tels que définis dans une quelconque revendication précédente.

5. Composé selon une quelconque revendication précédente, dans lequel le composé est de Formule la' 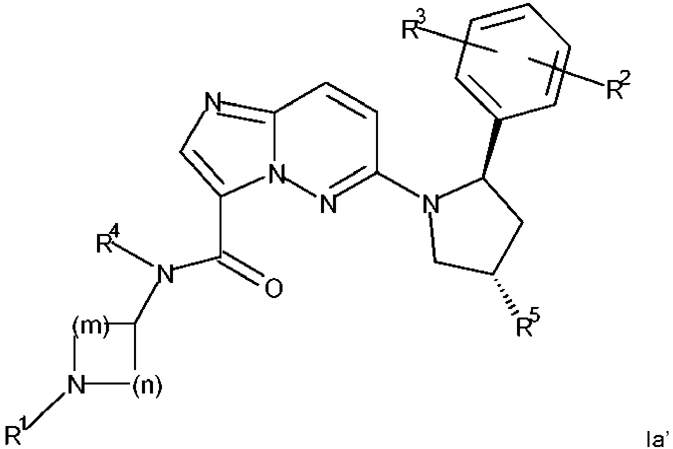 ou un sel ou solvate pharmaceutiquement acceptable de celui-ci, dans lequel R¹, R², R³, R⁴, R⁵, m et n sont tels que définis dans une quelconque revendication précédente.

6. Composé selon la revendication 1 ou la revendication 2, dans lequel le composé est de Formule Ib 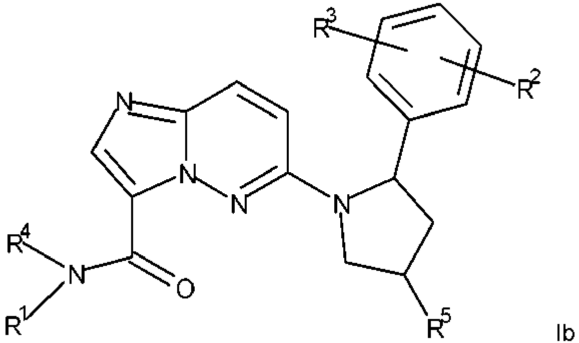 ou un sel ou solvate pharmaceutiquement acceptable de celui-ci, dans lequel R¹, R², R³, R⁴, R⁵, m et n sont tels que définis dans la revendication 1 ou la revendication 2.

7. Composé selon la revendication 1, 2 ou 6, dans lequel le composé est de Formule Ib' 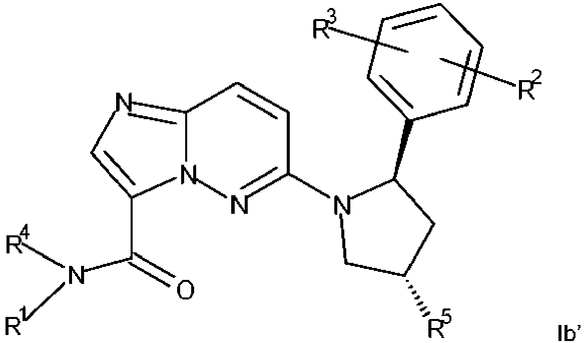 ou un sel ou solvate pharmaceutiquement acceptable de celui-ci, dans lequel R¹, R², R³, R⁴, R⁵, m et n sont tels que définis dans la revendication 1, 2 ou 6.

8. Composé selon la revendication 1, dans lequel ledit composé est sélectionné parmi :
le 6-2-[5-fluoro-2-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide;
le 6-[2-[5-fluoro-2-(méthylsulfanyl)phényl]pyrrolidin-1-yl]-N-[1-[(4-fluoro-3-hydroxyphényl)méthyl]pyrrolidin-3-yl]imidazo[1,2-b]pyridazine-3-carboxamide;
le 6-[4-fluoro-2-[5-fluoro-2-(méthylsulfanyl)phényl]pyrrolidin-1-yl]-N-{1-[(3-hydroxyphényl)méthyl]pipéridin-4-yl}imidazo[1,2-b]pyridazine-3-carboxamide;
le 6-[4-fluoro-2-[5-fluoro-2-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide ;
le 6-[4-fluoro-2-[5-fluoro-2-(méthylsulfanyl)phényl]pyrrolidin-1-yl]-N-{1-[(3-hydroxyphényl)méthyl]azétidin-3-yl}imidazo[1,2-b]pyridazine-3-carboxamide;
le 6-[4-fluoro-2-[5-fluoro-2-(méthylsulfanyl)phényl]pyrrolidin-1-yl]-N-{1-[(4-fluoro-3-hydroxyphényl)méthyl]pipéridin-4-yl}imidazo[1,2-b]pyridazine-3-carboxamide ;
le 6-[4-fluoro-2-[5-fluoro-2-(méthylsulfanyl)phényl]pyrrolidin-1-yl]-N-[(3-hydroxyphényl)méthyl]imidazo[1,2-b]pyridazine-3-carboxamide;
le 6-[4-fluoro-2-[5-fluoro-2-(méthylsulfanyl)phényl]pyrrolidin-1-yl]-N-[1-[(3-hydroxyphényl)méthyl]pyrrolidin-3-yl]imidazo[1,2-b] pyridazine-3-carboxamide;
le 3-{[3-{6-[4-fluoro-2-[5-fluoro-2-(méthylsulfanyl)phényl]pyrrolidin-1-yl]imidazo[1,2- b]pyridazine-3-amido}pyrrolidin-1-yl]méthyl}phényl pentanoate ;
le méthyl 3-{[3-{6-[4-fluoro-2-[5-fluoro-2-(méthylsulfanyl)phényl]pyrrolidin-1-yl]imidazo[1,2-b]pyridazine-3-amido}pyrrolidin-1-yl]méthyl}benzoate;
le 6-[4-fluoro-2-[5-fluoro-2-(méthylsulfanyl)phényl]pyrrolidin-1-yl]-N-[1-[(4-fluoro-3-hydroxyphényl)méthyl]pyrrolidin-3-yl]imidazo[1,2-b]pyridazine-3-carboxamide;
le butyl 3-{[3-{6-[4-fluoro-2-[5-fluoro-2-(méthylsulfanyl)phényl]pyrrolidin-1-yl]imidazo[1,2-b]pyridazine-3-amido}pyrrolidin-1-yl]méthyl}benzoate;
l'éthyl 3-{[3-{6-[4-fluoro-2-[5-fluoro-2-(méthylsulfanyl)phényl]pyrrolidin-1-yl]imidazo[1,2- b]pyridazine-3-amido}pyrrolidin-1-yl]méthyl}benzoate ;
le 6-[4-fluoro-2-[5-fluoro-2-(méthylsulfanyl)phényl]pyrrolidin-1-yl]-N-[(4-fluoro-3-hydroxyphényl)méthyl]imidazo[1,2-b]pyridazine-3-carboxamide;
le N-[1-[(4-fluoro-3-hydroxyphényl)méthyl]pyrrolidin-3-yl]-6-[2-[3-fluoro-5-(méthylsulfanyl)phényl]pyrrolidin-1-yl]imidazo[1,2-b]pyridazine-3-carboxamide;
le méthyl 3-{[3-{6-[4-fluoro-2-[3-fluoro-5-(méthylsulfanyl)phényl]pyrrolidin-1-yl]imidazo[1,2-b]pyridazine-3-amido}pyrrolidin-1-yl]méthyl}benzoate;
le 6-[4-fluoro-2-[3-fluoro-5-(méthylsulfanyl)phényl]pyrrolidin-1-yl]-N-[1-[(3-hydroxyphényl)méthyl]pyrrolidin-3-yl]imidazo[1,2-b]pyridazine-3-carboxamide;
le 6-[4-fluoro-2-[3-fluoro-5-(méthylsulfanyl)phényl]pyrrolidin-1-yl]-N-[1-[(4-fluoro-3-hydroxyphényl)méthyl]pyrrolidin-3-yl]imidazo[1,2-b]pyridazine-3-carboxamide;
le 6-[4-fluoro-2-[3-fluoro-5-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide ;
le 6-[4-fluoro-2-[3-fluoro-5-(méthylsulfanyl)phényl]pyrrolidin-1-yl]-N-{1-[(3-hydroxyphényl)méthyl]azétidin-3-yl}imidazo[1,2-b]pyridazine-3-carboxamide;
le 6-[4-fluoro-2-[3-fluoro-5-(méthylsulfanyl)phényl]pyrrolidin-1-yl]-N-{1-[(4-fluoro-3-hydroxyphényl)méthyl]pipéridin-4-yl}imidazo[1,2-b]pyridazine-3-carboxamide ;
le 6-[4-fluoro-2-[3-fluoro-5-(méthylsulfanyl)phényl]pyrrolidin-1-yl]-N-{1-[(3-hydroxyphényl)méthyl]pipéridin-4-yl}imidazo[1,2-b]pyridazine-3-carboxamide;
le 6-[4-fluoro-2-[3-fluoro-5-(méthylsulfanyl)phényl]pyrrolidin-1-yl]-N-[1-[(4-fluoro-3-hydroxyphényl)méthyl]4-méthoxypyrrolidin-3-yl]imidazo[1,2-b]pyridazine-3-carboxamide ;
le 6-[4-fluoro-2-[3-fluoro-5-(méthylsulfanyl)phényl]pyrrolidin-1-yl]-N-[4-[(4-fluoro-3-hydroxyphényl)méthyl]-1,4-oxazépan-6-yl]imidazo[1,2-b]pyridazine-3-carboxamide;
le 6-[4-fluoro-2-[3-fluoro-5-(méthylsulfanyl)phényl]pyrrolidin-1-yl]-N-[(4-fluoro-3-hydroxyphényl)méthyl]imidazo[1,2-b]pyridazine-3-carboxamide;
le 6-[4-fluoro-2-[3-fluoro-5-(méthylsulfanyl)phényl]pyrrolidin-1-yl]-N-[1-(3-hydroxybenzoyl)pyrrolidin-3-yl]imidazo[1,2-b]pyridazine-3-carboxamide;
l'acétate de 3-{[3-{6-[4-fluoro-2-[3-fluoro-5-(méthylsulfanyl)phényl]pyrrolidin-1-yl]imidazo[1,2-b]pyridazine-3-amido}pyrrolidin-1-yl]méthyl}phényl ;
l'acide 3-{[3-{6-[4-fluoro-2-[3-fluoro-5-(méthylsulfanyl)phényl]pyrrolidin-1-yl]imidazo[1,2-b]pyridazine-3-amido}pyrrolidin-1-yl]méthyl}benzoïque ;
le butyl 3-{[3-{6-[4-fluoro-2-[3-fluoro-5-(méthylsulfanyl)phényl]pyrrolidin-1-yl]imidazo[1,2- b]pyridazine-3-amido}pyrrolidin-1-yl]méthyl}benzoate ;
le 5-[4-fluoro-2-[3-fluoro-5-(méthylsulfanyl)phényl]pyrrolidin-1-yl]-N-{1-[(3-hydroxyphényl)méthyl]pipéridin-4-yl}pyrazolo[1,5-a]pyrimidine-3-carboxamide ;
ou un sel ou solvate pharmaceutiquement acceptable de celui-ci,
de préférence dans lequel ledit composé est sélectionné parmi :
le 6-[(2R)-2-[5-fluoro-2-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide;
le 6-[(2R)-2-[5-fluoro-2-(méthylsulfanyl)phényl]pyrrolidin-1-yl]-N-[(3S)-1-[(4-fluoro-3-hydroxyphényl)méthyl]pyrrolidin-3-yl]imidazo[1,2-b]pyridazine-3-carboxamide ;
le 6-[(2R,4S)-4-fluoro-2-[5-fluoro-2-(méthylsulfanyl)phényl]pyrrolidin-1-yl]-N-{1-[(3-hydroxyphényl)méthyl]pipéridin-4-yl}imidazo[1,2-b]pyridazine-3-carboxamide ;
le 6-[(2R,4S)-4-fluoro-2-[5-fluoro-2-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide ;
le 6-[(2R,4S)-4-fluoro-2-[5-fluoro-2-(méthylsulfanyl)phényl]pyrrolidin-1-yl]-N-{1-[(3-hydroxyphényl)méthyl]azétidin-3-yl}imidazo[1,2-b]pyridazine-3-carboxamide ;
le 6-[(2R,4S)-4-fluoro-2-[5-fluoro-2-(méthylsulfanyl)phényl]pyrrolidin-1-yl]-N-{1-[(4-fluoro-3-hydroxyphényl)méthyl]pipéridin-4-yl}imidazo[1,2-b]pyridazine-3-carboxamide ;
le 6-[(2R,4S)-4-fluoro-2-[5-fluoro-2-(méthylsulfanyl)phényl]pyrrolidin-1-yl]-N-[(3-hydroxyphényl)méthyl]imidazo[1,2-b]pyridazine-3-carboxamide;
le 6-[(2R,4S)-4-fluoro-2-[5-fluoro-2-(méthylsulfanyl)phényl]pyrrolidin-1-yl]-N-[(3S)-1-[(3-hydroxyphényl)méthyl]pyrrolidin-3-yl]imidazo[1,2-b]pyridazine-3-carboxamide ;
le 3-{[(3S)-3-{6-[(2R,4S)-4-fluoro-2-[5-fluoro-2-(méthylsulfanyl)phényl]pyrrolidin-1-yl]imidazo[1,2- b]pyridazine-3-amido}pyrrolidin-1-yl]méthyl}phényl pentanoate ;
le méthyl 3-{[(3S)-3-{6-[(2R,4S)-4-fluoro-2-[5-fluoro-2-(méthylsulfanyl)phényl]pyrrolidin-1-yl]imidazo[1,2- b]pyridazine-3-amido}pyrrolidin-1-yl]méthyl}benzoate ;
le 6-[(2R,4S)-4-fluoro-2-[5-fluoro-2-(méthylsulfanyl)phényl]pyrrolidin-1-yl]-N-[(3S)-1-[(4-fluoro-3-hydroxyphényl)méthyl]pyrrolidin-3-yl]imidazo[1,2-b]pyridazine-3-carboxamide ;
le butyl 3-{[(3S)-3-{6-[(2R,4S)-4-fluoro-2-[5-fluoro-2-(méthylsulfanyl)phényl]pyrrolidin-1-yl]imidazo[1,2- b]pyridazine-3-amido}pyrrolidin-1-yl]méthyl}benzoate ;
l'éthyl 3-{[(3S)-3-{6-[(2R,4S)-4-fluoro-2-[5-fluoro-2-(méthylsulfanyl)phényl]pyrrolidin-1-yl]imidazo[1,2- b]pyridazine-3-amido}pyrrolidin-1-yl]méthyl}benzoate ;
le 6-[(2R,4S)-4-fluoro-2-[5-fluoro-2-(méthylsulfanyl)phényl]pyrrolidin-1-yl]-N-[(4-fluoro-3-hydroxyphényl)méthyl]imidazo[1,2-b]pyridazine-3-carboxamide;
le N-[(3S)-1-[(4-fluoro-3-hydroxyphényl)méthyl]pyrrolidin-3-yl]-6-[(2R)-2-[3-fluoro-5-(méthylsulfanyl)phényl]pyrrolidin-1-yl]imidazo[1,2-b]pyridazine-3-carboxamide;
le méthyl 3-{[(3S)-3-{6-[(2R,4S)-4-fluoro-2-[3-fluoro-5-(méthylsulfanyl)phényl]pyrrolidin-1-yl]imidazo[1,2- b]pyridazine-3-amido}pyrrolidin-1-yl]méthyl}benzoate ;
le 6-[(2R,4S)-4-fluoro-2-[3-fluoro-5-(méthylsulfanyl)phényl]pyrrolidin-1-yl]-N-[(3S)-1-[(3-hydroxyphényl)méthyl]pyrrolidin-3-yl]imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-[(3S)-1-[(4-fluoro-3-hydroxyphényl)méthyl]pyrrolidin-3-yl]imidazo[1,2-b]pyridazine-3-carboxamide;
le 6-[(2R,4S)-4-fluoro-2-[3-fluoro-5-(méthylsulfanyl)phényl]pyrrolidin-1-yl]-N-{1-[(4-fluoro-3-hydroxyphényl)méthyl]azétidin-3-yl}imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-{1-[(3-hydroxyphényl)méthyl]azétidin-3-yl}imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-{1-[(4-fluoro-3-hydroxyphényl)méthyl]pipéridin-4-yl}imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-{1-[(3-hydroxyphényl)méthyl]pipéridin-4-yl}imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-[(3S,4S)-1-[(4-fluoro-3-hydroxyphényl)méthyl]-4-méthoxypyrrolidin-3-yl]imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-[(6S)-4-[(4-fluoro-3-hydroxyphényl)méthyl]-1,4-oxazépan-6-yl]imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-[(6R)-4-[(4-fluoro-3-hydroxyphényl)méthyl]-1,4-oxazépan-6-yl]imidazo[1,2-b]pyridazine-3-carboxamide ;
le 6-[(2R,4S)-4-fluoro-2-[3-fluoro-5-(méthylsulfanyl)phényl]pyrrolidin-1-yl]-N-[(4-fluoro-3-hydroxyphényl)méthyl]imidazo[1,2-b]pyridazine-3-carboxamide;
le 6-[(2R,4S)-4-fluoro-2-[3-fluoro-5-(méthylsulfanyl)phényl]pyrrolidin-1-yl]-N-[(3S)-1-(3-hydroxybenzoyl)pyrrolidin-3-yl]imidazo[1,2-b]pyridazine-3-carboxamide ;
l'acétate 3-{[(3S)-3-{6-[(2R,4S)-4-fluoro-2-[3-fluoro-5-(méthylsulfanyl)phényl]pyrrolidin-1-yl]imidazo[1,2- b]pyridazine-3-amido}pyrrolidin-1-yl]méthyl}phényl ;
l'acide 3-{[(3S)-3-{6-[(2R,4S)-4-fluoro-2-[3-fluoro-5-(méthylsulfanyl)phényl]pyrrolidin-1-yl]imidazo[1,2- b]pyridazine-3-amido}pyrrolidin-1-yl]méthyl}benzoïque ;
le butyl 3-{[(3S)-3-{6-[(2R,4S)-4-fluoro-2-[3-fluoro-5-(méthylsulfanyl)phényl]pyrrolidin-1-yl]imidazo[1,2- b]pyridazine-3-amido}pyrrolidin-1-yl]méthyl}benzoate ;
le 5-[(2R,4S)-4-fluoro-2-[3-fluoro-5-(méthylsulfanyl)phényl]pyrrolidin-1-yl]-N-{1-[(3-hydroxyphényl)méthyl]pipéridin-4-yl}pyrazolo[1,5-a]pyrimidine-3-carboxamide;
ou un sel ou solvate pharmaceutiquement acceptable de celui-ci.

9. Composition pharmaceutique comprenant un composé de Formule (I) : 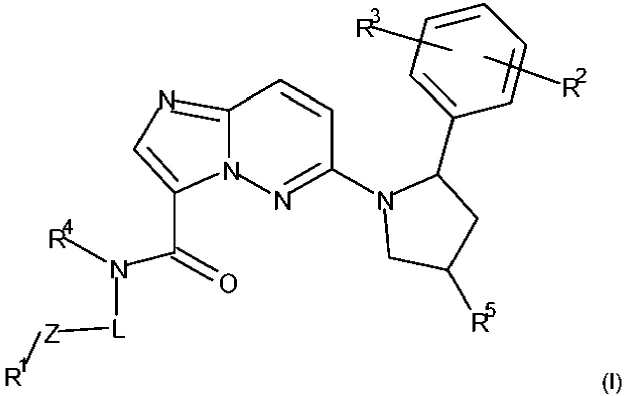 ou un sel ou solvate pharmaceutiquement acceptable de celui-ci ;
dans lequel :
Lest (CR⁶R⁷)ᵣ;
Z est absent ou sélectionné parmi :
i) 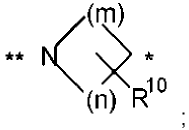 et
ii) 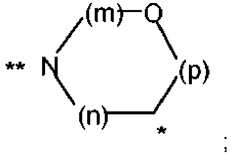
dans laquelle * désigne le point d'attache à L et ** désigne le point d'attache à R¹ ;
m est 1 ou 2 ;
n est 1 ou 2 ;
p est 0 ou 1 ;
à condition que la somme de m, n et p soit dans la plage de 2 à 4 ;
r est 0 ou 1 ;
R¹ est -XR⁹ ;
X est sélectionné parmi -CH₂-, -C(O)-, et -S(O₂)- ;
R² est -SR⁸ ;
R³ est sélectionné parmi H et un halo ;
R⁴ est sélectionné parmi H et un alkyle en C₁-C₃ ;
R⁵ est sélectionné parmi H, un hydroxyle et un halo ;
R⁶ et R⁷ sont chacun indépendamment sélectionnés parmi H et un alkyle en C₁-C₃ ;
R⁸ est un méthyle ;
R⁹ est un phényle substitué par un groupe sélectionné parmi un hydroxy, - OC(O)alkyle en C₁-C₆, C(O)OH et -C(O)Oalkyle en C₁-C₆, dans lequel le cycle phényle est facultativement substitué en outre par un halo ;
R¹⁰ est sélectionné parmi H et un alcoxy en C₁-C₃), et un ou plusieurs excipients pharmaceutiquement acceptables.

10. Composition pharmaceutique selon la revendication 9, dans laquelle
R¹ est -CH₂R⁹ ;
R³ est H ou un fluoro ;
R⁴ est H ;
R⁵ est H ou un fluoro ;
R⁶ est H ;
R⁷ est H ;
R⁹ est un phényle substitué par un hydroxy, dans lequel l'hydroxyphényle est facultativement substitué en outre par un fluoro ;
R¹⁰ est H ;
r est 0 ; et/ou
Z est absent.

11. Composition pharmaceutique selon la revendication 9 ou 10, dans laquelle le composé est de Formule I' ou de Formule Ib, et de préférence de Formule Ib', ou un sel ou solvate pharmaceutiquement acceptable de celui-ci 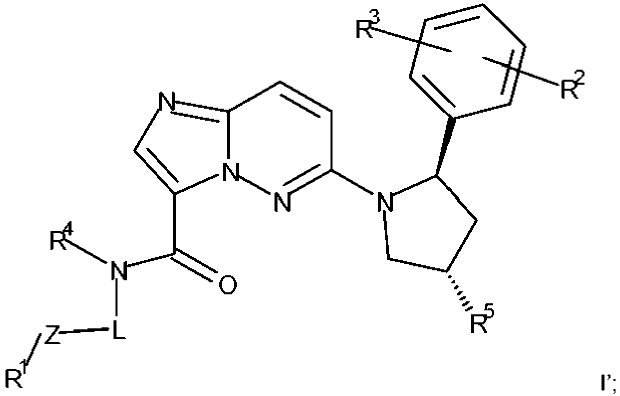 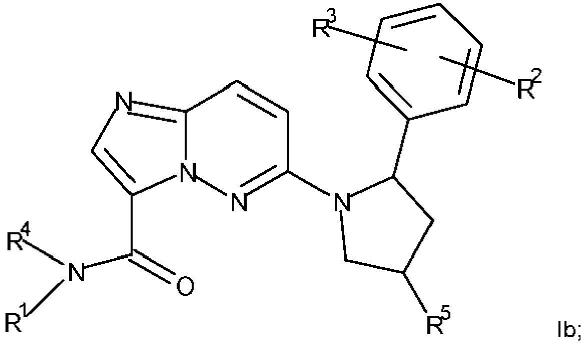 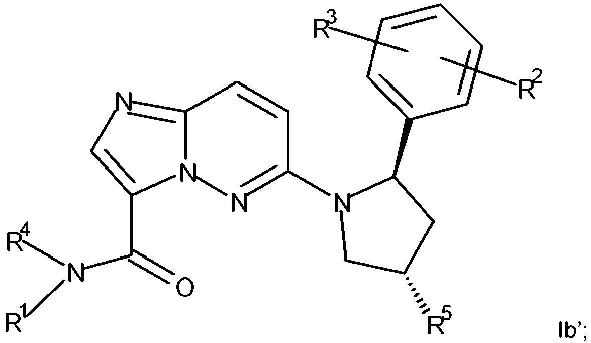 dans laquelle R¹, R², R³, R⁴, R⁵, L, Z, m et n sont tels que définis dans la revendication 9 ou la revendication 10.

12. Composition pharmaceutique comprenant un composé selon l'une quelconque des revendications 4, 5 ou 8 et un ou plusieurs excipients pharmaceutiquement acceptables.

13. Composition pharmaceutique selon l'une quelconque des revendications 9 à 12, en combinaison avec un ou plusieurs autres agents thérapeutiques.

14. Composé selon l'une quelconque des revendications 1 à 8, ou composition pharmaceutique selon l'une quelconque des revendications 9 à 13, pour une utilisation comme un produit pharmaceutique.

15. Composé selon l'une quelconque des revendications 1 à 8, ou composition pharmaceutique selon l'une quelconque des revendications 9 à 13, pour une utilisation dans le traitement ou la prévention d'une maladie ou d'un trouble qui est médié par Trk.

16. Composé ou composition pharmaceutique pour une utilisation selon la revendication 15, dans lequel/laquelle la maladie ou le trouble est médié par TrkA, TrkB et TrkC.

17. Composé ou composition pharmaceutique pour une utilisation selon la revendication 15 ou 16, dans lequel/laquelle la maladie ou le trouble est la dermatite atopique.

18. Composé ou composition pharmaceutique pour une utilisation selon l'une quelconque des revendications 15 à 17, dans lequel/laquelle le composé ou la composition pharmaceutique est administré(e) par voie topique.
